# EUROPEAN PATENT APPLICATION

(11) **EP 4 141 119 A2**
(43) Date of publication of application: **01.03.2023**
(21) Application number: 22188491.9
(22) Date of filing: 03.08.2022
(51) Int. Cl.: C12N 15/64, C12N 15/70

(54) **HAIRPIN LOOP ENDED SELF-COMPLEMENTARY DOUBLE-STRANDED COVALENTLY CLOSED LINEAR DNA VECTOR, MANUFACTURING SYSTEM AND PROCESS, AND USES OF SAID RESULTING DNA VECTOR**

(30) Priority: 06.08.2021 US 202163230603 P
(71) Applicant: Williams, Martin, 1008 Buenos Aires (AR); SYTE.bio Inc, Chestnut Hill, MA 02467 (US)
(72) Inventor: Williams, Martin, 1008 Buenos Aires (AR)
(74) Representative: Fernández-Pacheco, Aurelio Fernández

(57) **Abstract**

The present invention relates to a system and process for the production of a hairpin loop ended self-complementary double-stranded covalently closed linear deoxyribonucleic acid (DNA) vector, and the use of said vector as part of a therapeutic formulation enabling modulation of gene expression for medical applications. This system comprises a recombinant cell and at least two engineered parental circular covalently closed synthetic plasmids DNA, housed in the recombinant cell, wherein the synthetic transcriptional units of the at least two engineered parental circular covalently closed synthetic plasmids DNA are cloned in opposite directions. This DNA vector manufacturing system provides an efficient and high yield inducible process for producing hairpin loop ended linear covalently closed DNA vectors that incorporate a nucleic acid sequence of interest.

## Description

### FIELD OF INVENTION

The present invention relates to an engineered DNA manufacturing technology, and, in particular, to a system and process for the production of hairpin loop ended self-complementary double-stranded covalently closed linear DNA vectors, the resulting molecules and their uses.

### BACKGROUND OF THE INVENTION

Gene therapy is defined as the introduction of a foreign genetic material into a host cell *in vivo* (e.g., inside the host organism) or *ex vivo* (e.g., a cell that was transiently removed from the host organism) to prevent, treat, or ultimately cure, diseases by altering the expression of a gene. Some naturally occurring gene mutations result in proteins not being expressed correctly or not being expressed at all which can lead to genetically borne disorders. Gene therapy works by repairing, turning-off, or replacing dysfunctional genes that cause disease with the aim of (re)establishing normal function. Also, other pathological conditions (which may or may not include genetic disorders), such as cancer or heart disease, among others, can be treated by supplying genetic sequences that express biologically active factors (RNA and/or proteins) that promote cell death, or that promote or inhibit certain metabolic pathways, in order to overcome or collaborate in the treatment of that disease. Nowadays protein-coding nucleic acids are also used for the *in vivo* production of vaccine antigens, expanding the productive front for the development of gene therapies aimed at infectious diseases.

During the past years, there has been great interest in the field of gene and cell therapy and the first approved therapies have seen light following decades of efforts. Approved gene therapies include the first oligonucleotide-based therapies such as Spinraza, Exondys 51, Vyondys 53, three *ex vivo* cell therapies such as Kymriah, Yescarta and Tescartus, and two *in vivo* gene therapies such as Luxturna and Zolgensma, with more following close behind. These therapies have been designed to treat diverse diseases, including cancer, neuro-muscular disease, and inherited blindness. These approved therapies are life-changing for the affected patients and they harbor an enormous potential for extrapolation to many other pathological conditions. The success of *in vivo* delivered Adeno-associated virus (AAV)-based gene transfer into the human retina by Luxturna, and into the central nervous system by Zolgensma has facilitated the development of other AAV-based gene therapies for gene delivery into the liver to treat Hemophilia, and into skeletal muscle cells to treat Duchenne muscular dystrophy. As well, there have been developments using *ex vivo* lentiviral and retroviral gene transfer to T cells that led to adoptive cell immunotherapy that expanded to modification of hematopoietic stem cells, enabling therapies for inherited disorders, such as sickle cell disease and beta thalassemia recently approved in the European Union and currently under review in the United States.

In addition, it is important to mention that gene therapies with viral vectors can present immune-related problems in patients, and there are also obstacles in the manufacturing processes, since expensive and not entirely efficient eukaryotic cell systems are required to produce multiple doses. Usually, it is necessary to administer a high quantity of recombinant virions for each patient to achieve an adequate level of therapeutic success.

While of course there is an exciting potential for these early gene therapy achievements to be applied to other medical disorders, the use of viral-based technologies is generating increasing concern in treating human diseases. The major primary obstacle to a broader application continues to be the immune response to viral vectors and potentially harmful side-effects derived from foreign transgenes and foreign gene-controlling promoters. Consequently, the most important near-future work must be focused on the control of the immune system. For example, despite the remarkable success of several AAV-based gene therapies, 50% of patients are currently excluded from treatment due to pre-existing natural immunity to the adenoviral capsids. Recent advances and efforts in clinical trials, such as engineering the AAV capsids, have led to technological advances that avoid this immune obstacle, but there are still concerns when redosing is mandatory to generate a significant therapeutic effect.

Thus, while viral vectors offer great potential to deliver a genetic payload with high efficiency into target cells, they inevitably pose several safety issues, including genotoxic events such as the upregulation of proto-oncogenes, or the undesired activation of nearby genes. All of these vector-derived toxicities depend both on the virus type used, as well as on other non-viral factors, including patient age, overall health status, dose, route of administration and other variables that can also influence the genotoxic potential. Also, viral vectors can cause a regeneration of virulent particles adding yet another layer of safety concerns to take into consideration.

In contrast to viral vectors, non-viral strategies based on naked, lipoplexed, or polyplexed genetic material can offer safer and effective alternatives. DNA vectors are easy to generate, purify and store, while offering extraordinary engineering capacity. However, several barriers must be overcome first in order to deliver safe and effective genetic therapies *in vivo.*

The most used and researched genetic vectors are the circular covalently closed double-stranded plasmid DNA molecules, which have been used for several decades for gene transfer into eukaryotic cells. Despite their great ability to express coding sequences in host cells, these circular supercoiled molecules have a number of disadvantages that make them unsafe for use in medical applications. Such disadvantages include the presence of bacteria-derived sequences and genes, such as prokaryotic origins of replication and antibiotic resistance genes that could be transferred to the patient's microbiota leading to yet unknown disorders. To overcome said limitations, several vectors have been designed, such as DNA 'minicircles', lacking an origin of replication, antibiotic resistance, or other bacteria-derived sequences. Unfortunately, these molecules are still not effective enough due to their supercoiling structure that decreases the effectiveness with which they gain access to the nucleus.

There are other barriers that the DNA molecules have to overcome to get into the cell with their genetic cargo intact so as to be expressed. it is well known in the state of the art that nucleases present in patients' sera are able to digest unprotected DNA. This is why nucleic acid-based vectors must rapidly overpass the cell plasma membrane. This DNA vector transmembrane transport is complicated because cell membranes represent a lipoprotein barrier that hampers efficient nucleic acid translocation *per se.* To overcome this limitation, several strategies have been and are being developed, such as nucleic acid complexing with synthetic lipid nanoparticles to form a structure similar to the plasma membrane, or receptor-targeting nanoparticles for receptor-mediated endocytosis, among others. Although some of these strategies allow successful transmembrane transport of nucleic acids, once inside the cell these still show low efficiency in gaining access to the nuclear compartment, a site where they must enter to achieve gene expression.

The successful expression of a genetic sequence inside a host target cell also depends on a number of intracellular events. Immediately after the entry into the cytoplasm, naked DNA - either delivered through physical techniques such as electroporation, or after disassembly of DNA-carrier complexes - should associate with a large number of cellular proteins that mediate subsequent interactions with the microtubule network and move towards the microtubule organizing center and the nuclear envelope. DNA molecules then should enter the nucleus either after the mitotic nuclear envelope reorganization or through nuclear pore complexes in the absence of cell division, using a different set of proteins.

Although nuclear import of DNA molecules is not a normal event in the cell, several mechanisms exist for their transportation. Some of these nuclear transportation mechanisms have evolved over billions of years by viruses and other pathogens, allowing them to successfully infect their hosts, while others appear to be fortuitous "hijackings", as is the case of the SV40 virus genome which spontaneously associates to proteins while on their way to the nucleus. Regardless, the mechanism is the same: a DNA molecule containing Nuclear Targeting Sequences (NTS), which are short consensus motifs recognized by specific transcription factors (TFs) carrying nuclear localization signals (NLS) (either provided by the host or the pathogen), is directed to the nucleus by interacting with such proteins. Thus, these NTS sequences bind to TFs and target the molecules into the nucleus by an importing-mediated process.

Several nuclear importation techniques for nucleic acids are currently being developed. Some of these techniques include the modification of DNA vectors with a covalent linkage of proteins bearing NLS, and attachment of import receptors such as Karyopherins. Engineering of DNA with NTS seems to result in highly efficient translocation of linear DNA molecules but not supercoiled circular closed ones. This difference provides an interesting approach for *in vivo* uses of linear DNA vectors for medical applications.

Nowadays, cutting-edge technology is focusing on a group of novel DNA molecules: hairpin loop ended self-complementary double-stranded covalently closed linear DNA vectors. These offer a different approach compared to conventional circular covalently closed plasmid DNA molecules, posing great promises regarding efficacy and safety. Besides topological differences with circular covalently closed plasmid DNA molecules, hairpin loop ended linear covalently closed DNA molecules are torsion free, as they are not subjected to gyrase-mediated supercoiling. Moreover, as well as classic DNA plasmids, they are also not subjected to exonuclease activity in prokaryotes nor eukaryotes. They are also protected from mammalian nucleases present in sera due to their hairpin loop covalently closed ends, thus preventing degradation. Therefore, this type of DNA molecules has clear advantages over minicircles, and can also compete with viral vector systems in terms of safety, design and redesign versatility, and scale production.

There are several *in vitro* strategies for the generation of linear covalently closed DNA molecules. These include capping of PCR amplification products with self-complementary oligonucleotides, enzymatic digestion of previously amplified and purified backbones followed by ligation with hairpin sequences for telomeric end-closing (doggyBones), and generation of minimalistic immunologically defined gene expression (MiDGE) vectors using Phi26 and Telomerase enzymes. These technologies have shown promising results in several applications including the development of vaccines and therapeutic molecules. Also, these vectors have demonstrated up to 25-fold improved transgene expression profile when compared to classical circular counterparts. Thus, hairpin loop ended linear covalently closed DNA molecules outperform circular covalently closed DNA molecules in expression efficiency, and bio-/immune-compatibility. However, large scale production of linear covalently closed DNA molecules using the aforementioned *in vitro* technologies is expensive and time-consuming because several recombinant enzymes have to be produced and purified prior to manufacturing the linear DNA molecules. Considering these concerns, improvements in production processes are necessary.

As mentioned above, biological mechanisms associated to the production of linear covalently closed double-stranded DNA molecules occur in nature. Linear DNA molecules, as plasmids and chromosomes, were initially unknown in prokaryotes but have now been found in spirochaetes, Gram-positive, and Gram-negative bacteria. At least, two structural types of bacterial linear DNA have been characterized: a) linear plasmids of the spirochaete *Borrelia* with a covalently closed hairpin loop at each end; and b) the linear plasmids of the Gram-positive filamentous Streptomyces with a covalently attached protein at each end. Replicons with similar structures are more frequent in eukaryotic cells than in prokaryotes. Also, several bacteriophages have linear genomes with covalently closed telomeres, such as Bacteriophages N15, PY54, VP882, ΦHAP-1, ϕKO2, Bam35, VP58.5, vB_VpaM_MAR, VHML, amongst others.

All these bacterial and phage linear hairpin-ended DNA genomes are maintained in a linear fashion by enzymes called *recombinases* that are able to perform double-stranded DNA cuts and rearrangements, generating hairpin ends in the linear molecules.

Lyme disease-causing bacteria *Borrelia burgdorferi* has about a dozen of linear DNA molecules that carry covalently closed hairpin ends generated by specialized mechanisms for dealing with the end-replication problem. The hairpin telomeres are generated from replicative intermediates through a unique reaction: breakage of two phosphodiester bonds in a single DNA duplex (one on each strand) and joining of each end with the opposite DNA strand to form a covalently closed hairpin end. This process occurs through a two-step transesterification reaction by ResT, involving the formation of a covalent protein-DNA intermediate at a position three nucleotides from the axis of symmetry in each strand of the substrate.

As well, the N15 phage shows a similar DNA end pattern. N15 phage is a lambdoid virus of *Escherichia coli* whose prophage is not integrative but is a linear DNA with covalently closed ends. The virus DNA has single-stranded cohesive ends, named cosL and cosR, that allow the formation of a circular double-stranded DNA within the cell, which in turn generates an inverted repeat (teIRL). A virus-encoded protelomerase then recognizes that sequence and joins the phosphodiester bonds, producing covalently closed ends (telL and teIR) and, in consequence, a linear hairpin-ended DNA genome.

There have been previous efforts to generate hairpin ended linear covalently closed double-stranded DNA molecules *in vivo,* but there are still several concerns in terms of yield and scalability to address application requirements. Some *in vivo* methods for producing linear DNA molecules rely on the amplification of conventional circular covalently closed DNA plasmid vectors with relaxed origins of replication that generate between 200 and 700 copies per cell and cutting of such plasmid vectors with a protelomerase to generate linear DNA molecules. Said linear DNA molecules are co-purified with their parental DNA backbone, thus needing several additional purification steps to get rid of the parental DNA sequence that contains the antibiotic resistance cassette and the bacterial origin of replication.

None of the existing solutions is capable of providing an efficient and safe hairpin loop ended self-complementary double-stranded covalently closed linear DNA vector manufacturing system and process that allows a large-scale production without the resource consumption caused by the systems and processes known so far. Therefore, there is a need for a hairpin loop ended self-complementary double-stranded covalently closed linear DNA vector manufacturing system and process that is safer, simpler, more efficient and that results in a higher productivity compared to the systems and processes known in the state of the art.

### SUMMARY

The present disclosure provides, in a first aspect, an engineered parental circular covalently closed synthetic plasmid DNA comprising:
a synthetic DNA backbone comprising at least a first, second, and third recombinase recognition sequence, at least one endonuclease recognition sequence, at least one DNA replication enzyme recognition sequence, at least one bacterial selection system, and at least one bacterial origin of replication;
a linear DNA module scaffold comprising at least one DNA nuclear targeting sequence and at least one nuclear matrix-association sequence; and
one or more synthetic transcriptional units comprising at least two multiple cloning sites including endonuclease recognition sequences, at least one promoter sequence, at least one coding sequence of interest, and at least one polyadenylation signal sequence.

In a second aspect of the present invention, a helper plasmid comprising a first and a second synthetic genetic construction is disclosed, wherein the first synthetic genetic construction comprises at least a first repressor protein under at least a first constitutive promoter, at least a second repressor coding sequence under at least a second constitutive promoter, at least a third repressor coding sequence under at least a third constitutive promoter, at least one endonuclease recognition sequence, and wherein the second synthetic genetic construction comprises at least one DNA replicating protein under the control of a first inducible promoter, at least one recombinase protein coding sequence under the control of a second inducible promoter, and at least one endonuclease coding sequence under the control of a third inducible promoter.

In a third aspect, a recombinant cell comprising the helper plasmid is provided.

According to a fourth aspect of the invention, a hairpin loop ended self-complementary double-stranded linear covalently closed DNA vector production system is provided, wherein such system comprises:
the recombinant cell; and
at least two of the engineered parental circular covalently closed synthetic plasmids DNA, housed in the recombinant cell, wherein the synthetic transcriptional units of the at least two engineered parental circular covalently closed synthetic plasmids DNA are cloned in opposite directions.

In a fifth aspect of this invention, a hairpin loop ended self-complementary double-stranded linear covalently closed DNA vector production process is provided, starting from the aforementioned system, wherein said process comprises the stages of:
a. cloning the desired synthetic transcriptional unit/s between two of the multiple cloning sites in two of the engineered parental circular covalently closed synthetic plasmids DNA in order to generate plasmids pDNAv_Ac and pDNAv_Bc;
b. in order to generate the cointegrate plasmid pDNAv_ABc, alternatively subjecting both pDNAv_Ac and pDNAv_Bc to a first *in vitro* recombination using the first recombinase recognition sequence, or subjecting both pDNAv_Ac and pDNAv_Bc to a first *in vitro* recombination using restriction enzyme digestion in the first recombinase recognition sequence and subjecting digested pDNAv_Ac and pDNAv_Bc to ligation, or subjecting both pDNAv_Ac and pDNAv_Bc to a recombination including PCR amplification of each plasmid and the use of *in vitro* recombination systems;
c. transforming the cointegrate plasmid pDNAv_ABc with rolling circle and theta replication capacity into the recombinant cell and selecting recombinant clones;
d. alternatively subjecting the recombinant cell to incubation in growth conditions to allow theta replication of plasmid pDNAv_ABc, inducing the expression of the second recombinase in the recombinant cell to allow the generation of the Linear DNA molecules and inducing the expression of the endonuclease to allow the degradation and elimination of a resulting parental recombination product and the helper plasmid, or subjecting the cointegrate plasmid pDNAv_ABc to a second *in vivo* recombination using the second recombinase recognition sequence in order to generate pDNA_ABc1 and pDNA_ABc2, inducing the expression of the DNA replicating protein and endonuclease in order to allow "rolling circle" replication of pDNA_ABc1 and self-annealing of the single-stranded DNA products of the rolling circle replicated-DNA into hairpin loop ended double-stranded DNA molecules and allow the degradation of pDNAv_ABc2 by the endonuclease activity and inducing the expression of the second recombinase in the recombinant cell to allow the cleavage and elimination of the loop; and
e. isolating the resulting hairpin loop ended self-complementary double-stranded linear covalently closed DNA molecules from the recombinant cell.

In a sixth aspect of this invention, a hairpin loop ended self-complementary double-stranded linear covalently closed DNA vector is provided, wherein such vector is produced by the aforementioned process.

### BRIEF DESCRIPTION OF THE DRAWINGS

The invention is depicted in the Detailed Description with reference to the accompanying drawings.
Figure 1 is a schematic diagram showing a detailed representation of the parental plasmid, according to the first aspect of the present invention.
Figure 2 is a schematic diagram showing a detailed representation of the helper plasmid, according to the second aspect of the present invention.
Figure 3 is a schematic diagram showing a recombinant *E. coli* cell harboring the helper plasmid.
Figure 4 is a schematic diagram depicting the two main components of the system for manufacturing the molecule of the present invention, namely the recombinant *E. coli* strain of Figure 3, and a set of two plasmid vectors (pDNAv_A and pDNAv_B).
Figure 5 is a schematic diagram showing the parental plasmid pDNAv_A, according to one embodiment of the present invention.
Figure 6 is a schematic diagram showing the parental plasmid pDNAv_B, according to one embodiment of the present invention.
Figure 7 is a schematic diagram of the production process for the manufacturing of hairpin loop ended self-complementary double-stranded linear covalently closed DNA molecules, according to one embodiment of the present invention.
Figure 8 is a schematic diagram of the parental plasmid pDNAv_Ac with the expression cassette inserted between MCS1 and MCS2, according to one embodiment of the present invention.
Figure 9 is a schematic diagram of the parental plasmid pDNAv_Bc with the expression cassette inserted between MCS1 and MCS2, according to one embodiment of the present invention.
Figure 10 is a schematic diagram of the cointegrated plasmid DNA (pDNAv_ABc) with its features, according to one embodiment of the present invention.
Figure 11 is a schematic diagram of the splitted plasmid pDNAv_ABc1 suitable for "rolling circle" amplification, according to one embodiment of the present invention.
Figure 12 is a schematic diagram of the splitted plasmid pDNAv_ABc2 susceptible to endonuclease degradation, according to one embodiment of the present invention.
Figure 13 is a schematic representation of the "rolling circle" replication, the generation of the single stranded covalently closed "rolling circle" amplification product, and the self-assembly of the hairpin loop-ended DNA, according to one embodiment of this invention.
Figure 14 is a schematic representation of the endonuclease-mediated degradation of the pDNAv_ABc2.
Figure 15 is a schematic representation of the hairpin loop-ended DNA molecules produced by self-assembly of the rolling circle amplification product, just before polishing of the hairpin loops by means of expression of the 2nd recombinase.
Figure 16 is a schematic diagram of an alternative production process for the manufacturing of hairpin loop ended self-complementary double-stranded linear covalently closed DNA molecules, described in this invention.
Figure 17 is a schematic representation of the SYTE_{L}-DNA molecule produced after the polishing reactions catalyzed by the second recombinase acting upon the hairpin loop-ended DNA molecule of Figure 15.
Figure 18 is a schematic representation of the SYTE_{L}-DNA molecule encoding the EGFP protein in the transcriptional cassette.
Figure 19 is a schematic representation of hairpin loop ended self-complementary covalently closed linear DNA molecule suitable for gene therapy, anchored to the nuclear matrix scaffold by means of interactions with S/MAR sequences present in said DNA molecule, according to one embodiment of the present invention.
Figure 20 is an agarose-gel electrophoresis showing the amplicon DNA molecules generated by PCR that validates the integration of the complete DNA construction into the *E. coli* genome, or the presence of said DNA construction in episomal plasmid vectors, according to one embodiment of the present invention.
Figure 21 is an agarose-gel electrophoresis of the cointegrated DNA (pDNAv_ABc) resolution that validates the activity of PhiC31 protein expressed in the genome-recombinant or episomically-recombinant *E. coli,* according to one embodiment of the present invention, as can be depicted in Figures 10, 11 and 12.
Figure 22 is an agarose-gel electrophoresis of the cointegrated DNA (pDNAv_ABc) resolution and degradation of susceptible DNA sequences that validates the activity of PhiC31 protein and I- Scel endonuclease expressed in the genome-recombinant or episomically-recombinant *E. coli,* according to one embodiment of the present invention.
Figure 23 is the qPCR result that validates the copy number per cell of a pDNA control and the rolling circle-replicated pDNAv_AB, according to one embodiment of the present invention.
Figure 24 is an agarose-gel electrophoresis of the hairpin loop-ended self-complementary covalently closed linear DNA molecules subjected to exonucleases in lane number two, and with a preferred restriction enzyme BamHl, followed by exonuclease treatment, where complete degradation can be seen in lane number 3.
Figure 25 is a picture showing the GFP expression efficiency of different SYTE_{L}-DNA linear DNA molecules with and without the DNTS compared to plasmid DNA, according to one embodiment of this invention.
Figure 26 illustrates the replication of the linear DNA molecule in HEK-293 cells four weeks after transfection with the Linear DNA molecule encoding EGFP (SYTE_{L}-EGFP) . As can be seen, EGFP expression is maintained after ten replatings of the cell culture, demonstrating successful replication and maintenance of the construct in dividing cells.
Figure 27 is a micrograph showing a validation of *in vivo* expression of EGFP (SYTE_{L}-EGFP) in the mouse *biceps femoris* muscle, six days after the administration of 20 ug of the Linear DNA molecule in combination with non-targeted lipid nanoparticles.
Figure 28 illustrates the function of a safety switch abolishing expression of a SYTE_{L}-EGFP-TMD DNA molecule which also comprises a TALEN-mediated specific Linear DNA degradation module, 48h after doxycycline administration to transfected HEK-293 cells.
Figure 29 illustrates the function of a safety switch which induces apoptosis in HEK-293 cells transfected with a SYTE_{L}-ECFP-Kill DNA molecule which also comprises the coding sequence for the pro-apoptotic protein Bax, 48h after doxycycline administration.

Although the exemplary embodiments described herein are susceptible to various modifications and alternative forms, specific embodiments have been shown by way of example in the drawings and will be described in detail below. However, the exemplary embodiments described herein are not intended to be limited to the particular forms disclosed.

### DETAILED DESCRIPTION OF THE INVENTION

The general operating characteristics and advantages of the present invention will now be described in greater detail in this section in connection with the preferred embodiments, which should be considered as exemplary only and not limited to the present invention.

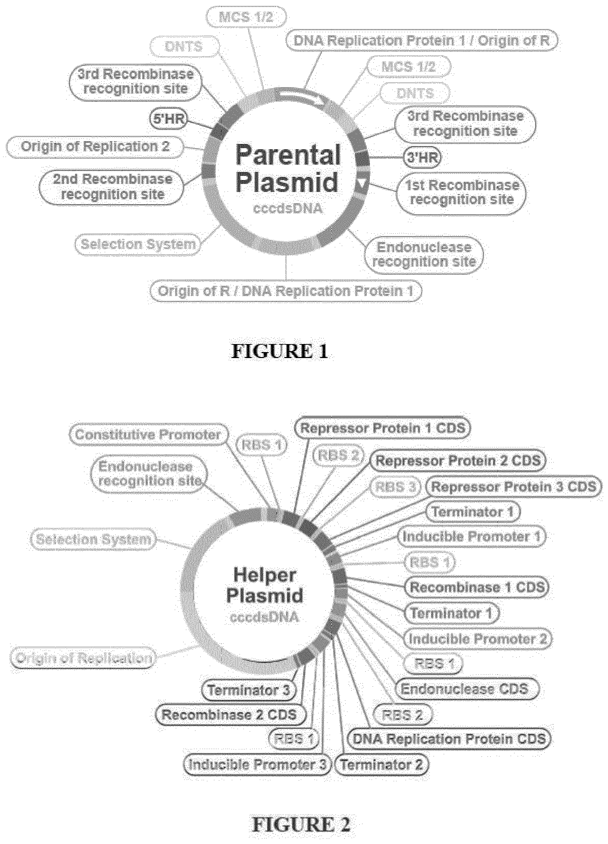

According to a first aspect of the present invention an engineered parental circular covalently closed synthetic plasmid DNA is provided ("Parental Plasmid", shown in Figure 1, SEQ ID NO 1), which comprises a synthetic DNA backbone comprising at least a first, second, and third recombinase recognition sequence, at least one endonuclease recognition sequence, at least one DNA replication enzyme recognition sequence, at least one bacterial selection system, and at least one bacterial origin of replication; a linear DNA module scaffold comprising at least one DNA nuclear targeting sequence, at least one nuclear matrix-association sequence; one or more synthetic transcriptional units comprising at least two multiple cloning sites including endonuclease recognition sequences, at least one promoter sequence, at least one coding sequence of interest, and at least one polyadenylation signal sequence.

In accordance with some embodiments of the present invention, the linear DNA module scaffold additionally comprises eukaryotic origins of replication such as, but not limited to, SV40 Origin of Replication.

In a preferred embodiment, the recombinase recognition sequence is selected from the group consisting of PhiC31 attP recombinase recognition sequence, phiC31 attP recombinase recognition site, CRE recombinase recognition sequence (LoxP), Flp recombinase recognition sequence, Lambda recombinase recognition sequence, ResT recombinase recognition sequence, ϕpK02 telRL site, the FRT site, TelN recombinase recognition sequence, PY54 Tel recombinase recognition sequence, VP882 TelK recombinase recognition sequence, ΦHAP-1 Tel recombinase recognition sequence, B. burgdorferi Tel recombinase recognition sequence, B. hermsii Tel recombinase recognition sequence, B. parkeri Tel recombinase recognition sequence, B. recurrentis Tel recombinase recognition sequence, B. turicatae Tel recombinase recognition sequence, B. anserine Tel recombinase recognition sequence, A. tumefaciens C58 ResT recombinase recognition sequence, and combinations thereof.

In a preferred embodiment, the endonuclease recognition sequence is selected from the group consisting of I- SceI endonuclease recognition sequence, F-SceI endonuclease recognition sequence, F-SceII endonuclease recognition sequence, I-AniI endonuclease recognition sequence, I-CeuI endonuclease recognition sequence, I-ChuI endonuclease recognition sequence, I-CpaI endonuclease recognition sequence, I-CpaII endonuclease recognition sequence, I-CreI endonuclease recognition sequence, I-CsmI endonuclease recognition sequence, I-DmoI endonuclease recognition sequence, I-PorI endonuclease recognition sequence, I-ScaI endonuclease recognition sequence, I-SceII endonuclease recognition sequence, I-SceIII endonuclease recognition sequence, I-SceIV endonuclease recognition sequence, PI-Scel endonuclease recognition sequence, Pi-Pspi endonuclease recognition sequence, PI-Tlil endonuclease recognition sequence, F-Suvl endonuclease recognition sequence, F-Tevl endonuclease recognition sequence, F-Tevll endonuclease recognition sequence, I-DirI, I-HmuI endonuclease recognition sequence, I-HmuII endonuclease recognition sequence, I-PpoI endonuclease recognition sequence, I-TevIII recognition sequence, and combinations thereof.

In a preferred embodiment, the DNA replication enzyme recognition sequence is selected from the group consisting of Phi29 DNA replication enzyme recognition sequence and P2-A replication enzyme recognition sequence.

In a preferred embodiment, the bacterial selection system is selected from the group consisting of antibiotics resistance selection systems such as, but not limited to Ampicillin, Kanamycin, Gentamicin and Chloramphenicol, dapD complementation selection system, infA complementation selection system, amino acid auxotrophy complementation selection system, RNA-OUT selection systems, and combinations thereof.

In a preferred embodiment, wherein the bacterial replication origin is selected from the group consisting of pUC19-derived replication origin, pBR322-derived replication origin, p15A-derived replication origin, pSC101-derived replication origin, Pir-R6K replication origin, trfA-oriV replication origin, M13 Phage replication origin, f1 Phage replication origin, PhiX174/ProteinA Ori replication origin, and pT181 (RepC) replication origin.

In a preferred embodiment, the DNA nuclear targeting sequence is selected from the group consisting of SV40 enhancer nuclear targeting DNA sequence, NF-kB nuclear targeting DNA sequence, GRE nuclear targeting DNA sequence, smooth muscle-specific nuclear targeting DNA sequence, smooth muscle γ-actin (SMGA) promoter nuclear targeting DNA sequence, Sox2 regulatory region 2 (SRR2) nuclear targeting DNA sequence, Karyopherin (importin) β1, isoform 1 (Kpnβ1) nuclear targeting DNA sequence, RanBP7/importin 7 (Ipo7) nuclear targeting DNA sequence, RAN nuclear targeting DNA sequence, Ran binding protein 1 (RanBP1) nuclear targeting DNA sequence, Ran binding protein 3 (RanBP3) nuclear targeting DNA sequence, SYTE-DNTS1 nuclear targeting DNA sequence, and combinations thereof.

In a preferred embodiment, the nuclear matrix-association sequence is selected from the group consisting of human b-interferon gene-derived S/MAR, human immunoglobulin heavy chain-derived S/MAR, human apolipoprotein B-derived S/MAR, chicken lysozyme-derived S/MAR, other vertebrate-derived S/MAR, SV40T mutated sequence, β-Globin Replicator sequence, Epstein-Barr virus (EBV) elements oriP, and EBNA1.

In a preferred embodiment, the Linear DNA Module Scaffold further comprises one or more eukaryotic origins of replication.

In further embodiments, the eukaryotic origin of replication is selected from the group consisting of *Homo sapiens* LOC107198088 ORI, *Homo sapiens* LOC1 07133510 ORI, SV40 Origin of replication, Epstein Barr Origin of replication, Bovine Papilloma Virus Origin of replication, and combinations thereof.

In a preferred embodiment, the promoter sequence is selected from the group consisting of RNA polymerase II constitutive mammalian promoters, such as the human cytomegalovirus (CMV) immediate-early enhancer and promoter (pCMV), the early promoter of the simian virus 40 (pSV40), the polyubiquitin gene promoter (pUBC), the hybrid construct of the cytomegalovirus (CMV) enhancer fused to the chicken beta-actin promoter (pCAGG), the human elongation factor-1 alpha promoter (pEF1A), the human phosphoglycerate kinase 1 promoter (pGK), inducible promoters constituted by the minimal CMV promoter and different response elements such as bacterial operators (e.g., lac repressor, tetracycline repressor) and other DNA binding sequences where artificial transcription factors can be associated, and RNA polymerase III promoters such as the H1 RNA promoter (pH1), the U6 promoter or the 7SK promoter.

In embodiments of the present invention, the transcription terminator sequence is selected from the group consisting of human α-globin polyadenylation signal sequence, human β-globin polyadenylation signal sequence, rabbit α-globin polyadenylation signal sequence, rabbit β-globin polyadenylation signal sequence, mouse k-light chain polyadenylation signal sequence, chicken ovalbumin polyadenylation signal sequence and small polyT stretch (5-6) for RNA pol III promoters.

In a preferred embodiment, the synthetic transcriptional unit can comprise at least one of the following genetic elements:

### IRES (Internal Ribosome Entry Site)

Internal Ribosome Entry Sites (IRES) are natural or artificial RNA sequences typically present in the 5' untranslated region of mRNAs that undergo intracatenary folding to render a three-dimensional structure that resembles that of eukaryotic factors involved in the initiation of translation. Hence, IRES make certain posttranscriptional modifications of the mRNA (such as capping) and said initiation factors superfluous, allowing the mRNA to enter directly into a translationally active state. When placed between different open reading frames in an mRNA, IRES can be used to achieve polycistronic translation in eukaryotes to render several proteins from a single mRNA molecule. Some IRES can even enhance translation of a downstream coding sequence.

In some embodiments, the IRES element is derived from a DNA sequence from an organism including, but not limited to, an animal, a plant, a fungus, a bacterium, a virus, an alga or a protozoan.

In some embodiments, viral IRES are sequences at least partially derived from a virus including, but not limited to, encephalomyocarditis virus (ECMV), Coxackievirus B3 (CVB3), Taura Syndrome Virus (TSV), Triatoma Virus (TV), Human mastadenovirus C (HAdV-C), Human adenovirus 5 (HAdV5), Human adenovirus 7 (HAdV7), Human mastadenovirus B (HAdV-B), Hepatitis GB virus B (HGBV-B), HPV31, Human Poliovirus 3 (HPV-3), Human papillomavirus type 11 (HPV11), Human immunodeficiency virus 1 (HIV-1), Human immunodeficiency virus 2 (HIV-2), Simian T-lymphotropic virus 1 (STLVs-1), Human Parvovirus B19 (HPB19), Human betaherpesvirus 6B (HHV-6B), Human alphaherpesvirus 3 (HHV-3), Human papillomavirus type 41 (HPV41), Human papillomavirus type 6b (HPV6b), Alphapapillomavirus 7, Friend murine leukemia virus, Heilovirus (ThV), Rous sarcoma virus (RSV), Human mastadenovirus F (HAdv-F), Human papillomavirus type 4 (HPV4), Human papillomavirus type 63 (PHV63), Human mastadenovirus A, Feline immunodeficiency virus (FIV), Human T-lymphotropic virus 2 (HTLV-2), Jaagsiekte sheep retrovirus (JSRV), Spleen focus-forming virus (SFFV), Mouse mammary tumor virus (MMTV), Murine osteosarcoma virus (MOV), Ovine lentivirus (OLV/OvLV), Squirrel monkey retrovirus (SMRV), Human papillomavirus type 16 (HPV16), Human papillomavirus type 5 (HPV5), Human polyomavirus 1, Rabies lyssavirus, Simian immunodeficiency virus (SIV), Human papillomavirus type 10 (HPV10), Human papillomavirus type 26 (HPV26), Human papillomavirus type 32 (HPV32), Human papillomavirus type 34 (HPV34), Marburg margurgvirus, Enterovirus A, Rhinovirus A, Human betaherpesvirus 6A (HHV-6A), Macaca mulatta polyomavirus 1, Snakehead retrovirus (SnRV), Bovine foamy virus (BFV), Drosophila C virus (DCV), Hendra henipavirus (HeV), Aichi virus 1 (AiV-1), Influenza B virus (IBV), Zaire ebolavirus (ZEBOV), Human coronavirus 229E (HCoV-229E), Nipah henipavirus (NiV), Human respirovirus 1 (HPIV-1), Modoc virus (MODV), Sudan ebolavirus, Human parvovirus 4 G1, Rotavirus C, Human gammaherpesvirus 4 (Epstein-Barr virus).

In some embodiments, eukaryotic IRES are sequences at least partially derived from mRNAs from different species including, but not limited to, Homo sapiens IRES, Aplysia californica IRES, Canis lupus familiaris IRES, Drosophila melanogaster IRES, Gallus gallus IRES, Mus pahari IRES, Mus musculus IRES, Saccharomyces cerevisiae IRES, Zea mays IRES, Rattus norvegicus IRES, Ovis aries IRES.

In some embodiments, the IRES element comprises a novel synthetic proprietary IRES, for instance: SYTE-IRES1, SYTE-IRES2, SYTE-IRES3, SYTE-IRES4, amongst others.

In some embodiments, the hairpin loop ended self-complementary double stranded covalently closed linear polyribonucleotide includes at least one IRES flanking at least one or more open reading frames. in some embodiments, the IRES flanks both sides of one or more open reading frames. In some embodiments, the hairpin loop ended self-complementary double stranded covalently closed linear polyribonucleotide includes at least one IRES sequence on one or both sides of each open reading frame.

### m6A induced ribosome engagement sites (MIRES)

trM6-methyladenosine (m6A) is a reversible epitranscriptomic modification found in several eukaryotic mRNAs, and when present in the 5' untranslated region (5'UTR), even a single m6A, promotes cap-independent translation. These sites are called m6A induced ribosome engagement sites (MIRES).

MIRES stimulate selective mRNA translation in stress conditions by a mechanism that involves the direct binding of the initiation factor eIF3. MIRES-dependent translation initiation should contain an RRACH (R = Guanine or Adenine, H = Adenine or Cytosine or Thymine) consensus motif of m6A modification close to the start codon.

The m6A machinery allows the hairpin loop ended self-complementary double stranded covalently closed linear polyribonucleotide to be marked as self or endogenous. When using chemical or enzymatic circularization of RNA molecules, it is beneficial to use m6A dependent translation to avoid unwanted immune responses. When m6A RRACH motifs are incorporated in a circular polyribonucleotide, RIG-1 mediated immunogenicity is decreased by 10,000-fold, compared to mutated m6A RRACH motifs.

When placed between different open reading frames in an mRNA, MIRES can be used to achieve polycistronic translation in eukaryotes to render several proteins from a single mRNA molecule. Some MIRES can even enhance translation of a downstream coding sequence.

In some embodiments, the hairpin loop ended self-complementary double stranded covalently closed linear polyribonucleotide may be translated by another cap- independent mechanism. In some embodiments the cap-independent mechanism is mediated by the methylation of the nitrogen at position 6 in the adenosine base, N6-methyladenosine (m6A), within the hairpin loop ended self-complementary double stranded covalently closed linear polyribonucleotide.

In some embodiments, the hairpin loop ended self-complementary double stranded covalently closed linear polyribonucleotide may have one or more m6A sites close to the start codon.

### Untranslated Regions (UTRs)

In some embodiments, the hairpin loop ended self-complementary double stranded covalently closed linear polyribonucleotide can comprise untranslated regions (UTRs), that are genetic sequences that can be transcribed but are not translated.

In some embodiments, one or more UTR sequences may be included upstream of the translation initiation sequence of an open reading frame (5'UTRs). In some embodiments one or more UTR sequences may be included downstream of an expression sequence (3'UTRs).

In some embodiments, the hairpin loop ended self-complementary double stranded covalently closed linear polyribonucleotide comprises at least one UTR sequence with embedded tranches of Adenosines and Thymidine within. In some embodiments, these A-U rich elements (AREs) may increase the expression rates of the hairpin loop ended self-complementary double stranded covalently closed linear polyribonucleotide.

In some embodiments, introduction, removal or modification of UTR AREs may be useful to modulate the stability, immunogenicity and translation rate of the hairpin loop ended self-complementary double stranded covalently closed linear polyribonucleotide.

In some embodiments, the hairpin loop ended self-complementary double stranded covalently closed linear polyribonucleotide may contain one or more copies of AREs that may modulate translation of the coding sequence.

In some embodiments, the hairpin loop ended self-complementary double stranded covalently closed linear polyribonucleotide may be engineered to remove AREs to modulate the intracellular stability and thus the translation of the coding sequence.

it should be understood that any UTR from any gene and any organism may be incorporated into the respective flanking regions of the hairpin loop ended self-complementary double stranded covalently closed linear polyribonucleotide. Furthermore, multiple wild-type UTRs of any known gene may be utilized. it is also within the scope of the present invention to provide novel synthetic proprietary UTRs which are not variants of wild type genes.

In some embodiments, the UTRs or portions thereof, may be placed in the same orientation or altered orientation or location as from where they were selected.

In some embodiments, a 5' or 3' UTR may be inverted, shortened, lengthened and/or chimerized with one or more other 5' or 3' UTRs.

As used in this patent, the term "altered" as it relates to a UTR sequence, means that the UTR sequence has been changed in some way in relation to a reference sequence.

In some embodiments, more than one 5' and/or 3' UTR may be used. For example, a "double" UTR is one in which two copies of the same UTR are encoded in series or substantially in series.

In some embodiments, the UTRs or portions thereof may comprise a group I self-splicing intron system known as PIE (Permuted Intron-Exon) that allows the formation of circular RNA molecules. There are several group I intron precursor RNAs derived from organisms such as *Tetrahymena* spp or *Anabaena* spp, and viruses such as T4 phage, which contain end-to-end fused exons that interrupt half intron sequences that in turn self-splice to generate a circular RNA exon. Exon sequences can be replaced by foreign sequences from a group I intron self-splicing system to make desired circular sequences, and that is possible because exon sequences are not involved in the self-splicing reaction. The Permuted intron Exon method includes two transesterifications at defined splice sites; and, after the splicing, the ends are ligated forming circular RNA molecules. More specifically, the first transesterification releases the 3'-end sequence (5'-half intron) of the sequence. After, the free 3'OH group of the 3'-half exon attacks the 3'-splice site during the second transesterification, generating circRNA molecules. This method has been used to generate RNA aptamers, hammerhead ribozymes and hepatitis delta ribozymes.

In some embodiments, the UTRs or portions thereof may comprise a group II introns that can be modified for inverted splicing and thus generate circular RNA molecules. Yeast self-splicing group II intron can catalyze the formation of circular RNA molecules. This method requires the rearrangement of the exons in a consecutive way, where the branch point has to be positioned upstream and the intronic sequences flanking the exons. This method allows the circularization of the molecules by two transesterifications. This group II method is way more efficient than the group I intron method, however this approach carries 2'-5' phosphodiester bond at the ligation site.

In some embodiments, the UTRs or portions thereof may comprise a Direct back- splicing event wherein the event involves joining the 3'-tail of a downstream exon to the 5'-head of an exon normally located upstream. The downstream splice donor pairs with an unspliced upstream splice acceptor and as a result, the exon becomes circularized.

In some embodiments, the UTRs or portions thereof may comprise a lariat intermediate that contains an exon produced from exon skipping, that subsequently undergoes internal splicing, thereby removing the intron and generating a circularized RNA molecule.

### Sequence of Interest

The sequence of interest may be a coding, translation competent genetic sequence or a non-coding, translation incompetent genetic sequence.

In some embodiments, the sequence of interest might be transcribed into a linear mRNA molecule.

In some embodiments, the sequence of interest might be transcribed into a circular RNA molecule.

### Translation Competent Coding Sequences

### Translation Initiation

In some embodiments, the hairpin loop ended self-complementary double stranded covalently closed linear polyribonucleotide can encode a polypeptide and may comprise a translation initiation sequence such as, but not limited to, start codon. In some embodiments the hairpin loop ended self-complementary double stranded covalently closed linear polyribonucleotide includes at least one translation initiation sequence adjacent to an open reading frame. In some embodiments the start codon is a non-coding start codon. In some embodiments, the translation initiation genetic sequence can include a Shine-Dalgarno or Kozak sequence. In some embodiments the Shine-Dalgarno or Kozak sequence may be adjacent to an open reading frame (ORF). In some embodiments the translation initiation sequence may be placed in either or both sides of each open reading frame. In some embodiments, the translation initiation sequence may provide a conformational flexibility to the hairpin loop ended self-complementary double stranded covalently closed linear polyribonucleotide.

In some embodiments the hairpin loop ended self-complementary double stranded covalently closed linear polyribonucleotide may include at least one start codon and translation may initiate on the first start codon or may initiate downstream the first start codon. In some embodiments the translation of the polyribonucleotide may initiate at an alternative translation initiation sequence, such as, but not limited to, AAG, ACG, AGG, ATA/AUA, ATT/AUU, CTG/CUG, GTG/GUG and TTG/UUG. In some embodiments, the translation may begin under selective conditions, such as, but not limited to, cell type, cell condition and stress conditions. In some embodiments the hairpin loop ended self-complementary double stranded covalently closed linear polyribonucleotide may begin translation at a repeat-associated non-AUG (RAN) sequence.

### Coding Sequence

In some embodiments the hairpin loop ended self-complementary double stranded covalently closed linear polyribonucleotide may encode prokaryotic and/or eukaryotic peptides or polypeptides of interest, selected from, but not limited to, biologics, antibodies, antigens, therapeutic peptides or polypeptides, cell penetrating peptides, secreted proteins, plasma membrane anchored proteins, cytoplasmic proteins, cytoskeletal proteins, intracellular membrane bound proteins, nuclear proteins, proteins associated with any human disease, targeting moieties or those proteins encoded by the human genome for which no therapeutic indication has been identified but which nonetheless have utility in areas of research and discovery.

As indicated herein, "Therapeutic protein" refers to a protein that, when administered to an organism, has a therapeutic, diagnostic and/or prophylactic effect, and/or elicits a desired biological and/or pharmacological effect.

In some embodiments, the hairpin loop ended self-complementary double stranded covalently closed linear polyribonucleotide disclosed herein, may encode one or more biologics. As used herein, a "biologic" is a peptide or polypeptide produced by the methods provided herein and which may be used to prevent, diagnose, mitigate, treat or cure a serious disease or medical condition.

According to the present invention, biologics include, but are not limited to, vaccines, monoclonal antibodies, bi-specific antibodies, tri-specific antibodies, chimeric antigen receptors, allergenics, blood components, gene therapies, mammalian organ, tissue or cellular products, cytokines, immunomodulators, growth factors and enzymes amongst others.

In some embodiments, one or more biologics can be encoded and translated by the hairpin loop ended self-complementary double stranded covalently closed linear polyribonucleotide of the present invention.

In some embodiments, the hairpin loop ended self-complementary double stranded covalently closed linear polyribonucleotide can encode one or more antibody fragments and combinations thereof. The term "antibody" includes monoclonal antibodies, which may include full length antibodies with an Fc region, antibodies with poly epitope specificity, multispecific antibodies such as, but not limited to, bispecific antibodies, diabodies, single chain molecules, VHH antibodies and antibody fragments. As described herein, the term "immunoglobulin" (Ig) is used interchangeably with the term "antibody". As described herein, the term "monoclonal antibody" refers to an antibody obtained from an identical population of antibodies. Monoclonal antibodies are highly specific, and are directed against a single antigenic site.

In some embodiments, monoclonal antibodies include "chimeric" antibodies in which a portion of the heavy and/or light chain is homologous to antibodies derived from a particular species or correspond to a particular antibody class or subclass.

In some embodiments, the hairpin loop ended self-complementary double stranded covalently closed linear polyribonucleotide may include any of the five classes of immunoglobulins, IgA, IgD, IgE, IgG and IgM, including the heavy chains designated alpha, delta, epsilon, gamma and mu respectively. In some embodiments, the hairpin loop ended self-complementary double stranded covalently closed linear polyribonucleotide may include any subclass of antibody such as, but not limited to, IgA1, IgA2, IgG1, IgG2, IgG3 and IgG4.

In some embodiments, the hairpin loop ended self-complementary double stranded covalently closed linear polyribonucleotide may encode whole antibodies or antibody fragments of interest which include, but are not limited to, Fab, Fab', F(ab'), Fv, diabodies, linear antibodies, nanobodies, bi-specific antibodies, tri-specific antibodies, tetra-specific antibodies, single-chain fragment variable, multispecific antibodies or combinations thereof

In some embodiments, the hairpin loop ended self-complementary double stranded covalently closed linear polyribonucleotide may encode one or more antibody sequences that are currently being on the market or in development.

In some embodiments, the hairpin loop ended self-complementary double stranded covalently closed linear polyribonucleotide may encode antibodies to treat or prevent diseases in several therapeutic areas such as, but not limited to, immunology, endocrinology, dermatology, oncology, musculoskeletal, respiratory, central nervous system, gastrointestinal and infectious.

In some embodiments, the hairpin loop ended self-complementary double stranded covalently closed linear polyribonucleotide disclosed herein, may encode one or more vaccine antigens.

As indicated herein, "vaccine antigen" refers to a peptide or polypeptide that improves immunity to a particular disease or infectious agent.

In some embodiments, the hairpin loop ended self-complementary double stranded covalently closed linear polyribonucleotide may encode one or more vaccine antigens. In some embodiments, the antigens encoded may be used to treat or prevent diseases in many therapeutic areas such as, but not limited to, cancer, allergy and infectious diseases.

In some embodiments, the hairpin loop ended self-complementary double stranded covalently closed linear polyribonucleotide of this invention may encode one or more antimicrobial peptides, which have been isolated from a wide range of animals such as, but not limited to, microorganisms, plants, invertebrates, amphibians, birds, fish, and mammals.

In some embodiments, the hairpin loop ended self-complementary double stranded covalently closed linear polyribonucleotide of this invention may encode antimicrobial peptides that may block cell fusion of viral entry. In some embodiments, the antimicrobial peptides may be part of viral encoded proteins. In some embodiments, the hairpin loop ended self-complementary double stranded covalently closed linear polyribonucleotide described in this invention, may encode one or more cell penetrating peptides. As used herein, "cell penetrating peptide" refers to a polypeptide which may facilitate the cellular uptake of other polypeptides or molecules. In some embodiments, the cell penetrating peptide, may be fully or partially labeled. In some embodiments, the cell penetrating peptide may include a signal peptide. As used herein, a "signal sequence" refers to a sequence of amino acid residues bound at the amino terminus of a nascent protein during protein translation, that enables the polypeptide for example to be secreted. In some embodiments, the signal sequence may be used to signal the secretion of the cell penetrating peptide.

In some embodiments, the hairpin loop ended self-complementary double stranded covalently closed linear polyribonucleotide described in this invention may encode a fusion protein. In some embodiments, the fusion protein may be created by operably linking two proteins of interest. As used herein, "operably linked" refers to the connection of two proteins in such a way to permit the expression of the complex when introduced into a cell.

In some embodiments, the hairpin loop ended self-complementary double stranded covalently closed linear polyribonucleotide encodes a plasma membrane anchored protein, a cytoplasmic protein or cytoskeletal protein. In some embodiments, the hairpin loop ended self-complementary double stranded covalently closed linear polyribonucleotide encodes a nuclear protein.

In some embodiments, the hairpin loop ended self-complementary double stranded covalently closed linear polyribonucleotide of this invention encodes and is able to express a protein associated with a mammalian disease. In some embodiments, the mammalian disease is a human disease.

In some embodiments the hairpin loop ended self-complementary double stranded covalently closed linear polyribonucleotide may encode proteins such as, but not limited to, VEGF, Hemoglobin, FIX, cancer-specific antigens, gene editing enzymes, Zinc Fingers, Nucleases, Cas9 and TALENs.

As described herein, a polypeptide may be a single molecule, or a multi-molecular complex such as a dimer, trimer, tetramer or n-mer. In some embodiments the polypeptide may also comprise single chain or multiple chain polypeptides that may be associated or linked.

In some embodiments, the coding sequences embedded in the parental plasmid DNA may be codon optimized to permit an efficient translation of peptides or polypeptides that may be derived from different organisms than the host organism.

In further embodiments, the sequence of interest is selected from the group consisting of peptides, polypeptides, vaccine antigens, monoclonal antibodies, mono-specific antibodies, bi-specific antibodies, tri-specific antibodies, tetra-specific antibodies, chimeric antigen receptors, allergens, blood components, gene therapies, organ or cellular products, cytokines, immunomodulators, growth factors, enzymes, antibody fragments, poly epitopes, multispecific antibodies, poly-epitope-specific antibodies, diabodies, single chain molecules, VHH antibodies, antibodies fragments, chimeric antibodies, fusion proteins, plasma membrane anchored proteins, cytoplasmic proteins, cytoskeletal proteins, nuclear proteins, gene editing proteins, zinc fingers, nucleases, Cas9, TALENs, dimers, trimers, tetramers, single-chain polypeptides, multi-chain polypeptides, and combinations thereof.

### Riboswitches

A riboswitch is an RNA sequence that can directly bind to a small target molecule, and the binding to that molecule directly affects the continuity of transcription of the riboswitch-bearing RNA itself and/or its own translation, thus the hairpin loop ended self-complementary double stranded covalently closed linear polyribonucleotide that includes a riboswitch is directly involved in regulating its own activity depending on the presence or absence of its target molecule.

In some embodiments, the hairpin loop ended self-complementary double stranded covalently closed linear polyribonucleotide comprises one or more riboswitches.

In some embodiments, a riboswitch may have an aptamer-like affinity for a specific molecule, thus, these riboswitches may be used for sequestration of molecules from bulk amounts.

In some embodiments, the riboswitch may have an effect on gene expression, including, but not limited to, transcriptional termination, inhibition of translation initiation, mRNA self-cleavage, alteration of splicing pathways, binding to a trigger molecule to activate or inactivate gene expression.

In some embodiments, the hairpin loop ended self-complementary double stranded covalently closed linear polyribonucleotide may be subjected to conditions that may activate, deactivate or block the gene expression. Expression can be altered as a result of blocking the ribosome binding site.

In some embodiments, the hairpin loop ended self-complementary double stranded covalently closed linear polyribonucleotide binding to a molecule or an analog thereof can, depending on the nature of the riboswitch, reduce or prevent, promote or increase the expression of the RNA molecule

### Aptazymes

An aptazyme is a genetic switch that can be used for conditional gene expression in which the aptamer region is used as an allosteric control element that can be coupled to a catalytic RNA ribozyme sequence.

In some embodiments, the hairpin loop-ended self-complementary double stranded covalently closed linear polyribonucleotide can comprise one or more aptazymes.

In some embodiments, the hairpin loop-ended self-complementary double stranded covalently closed linear polyribonucleotide comprises an aptazyme that is active under specific cell states. In some embodiments, the specific cell state may include, but is not limited to, viral infection, or the presence of viral nucleic acids or proteins.

A ribozyme, or ribonucleic acid enzyme, is an RNA molecule that catalyzes a chemical reaction. Many ribozymes that are found in nature catalyze the hydrolysis of their own phosphodiester bonds or the hydrolysis of other RNA or DNA bonds. Ribozymes are able to catalyze other types of chemical reactions such as, but not limited to, covalent modification of amino acid side chains, tyrosine azido-adenylation, phosphorylation, dephosphorylation, covalent modification of phosphorylated amino acid side chains, amide and ester hydrolysis, diels-alder reaction, glycosylation, porphyrin metalation, RNA cleavage, RNA ligation, thymine-dimer repair, DNA depuration, DNA phosphorylation, DNA capping, DNA cleavage and DNA ligation.

In some embodiments, it may be advantageous to place many copies of a ribozyme and aptamers within a non-coding RNA.

In some embodiments, non-limiting examples of ribozymes may include hammerhead ribozyme, VL ribozyme, leadzyme and hairpin ribozyme.

In some embodiments, the aptazyme encoded in the hairpin loop-ended self-complementary double stranded covalently closed linear polyribonucleotide may also permit the self-regulation of the gene expression. In some embodiments, the polyribonucleotide gene expression may be autoregulated by the presence of an aptazyme that is activated by the product encoded in the same polyribonucleotide sequence. In some embodiments, the activity of the aptazyme can be sensitive to the accumulation of the protein product encoded within the polyribonucleotide or any other cellular macromolecule.

### 2A Peptides

Virus-derived self-cleaving 2A peptides are promising candidates for the production of multicistronic RNA therapeutic molecules due to their small size and self-cleavage ability. Most 2A peptides are composed of 16-20 amino acids and are taken from viral RNA. The most common 2A peptides used to produce multicistronic vectors are F2A (Foot-and-Mouth Disease virus), E2A (Equine Rhinitis Virus), P2A (Porcine Teschovirus-1), and T2A (Thosea Asigna Virus). 2A peptide cleavage is not mediated by proteases, but it is mediated via ribosomal skip mechanism. The cleavage sites of these peptides are located between the C- terminal glycine of 2A and the N-terminal proline of 2B. This process takes place inside the ribosome during protein synthesis, where the formation of a normal peptide bond between these amino acids at the cleavage site is inhibited, but the cleavage does not affect the translation of the subsequent protein, since ribosome dissociation does not occur.

The use of elongated 2A with the additional spacers in the N-terminus of 2A peptide sequence can provide an efficient cleavage reaction, since it leads to a decrease in the inhibition rate of the 2A cleavage reaction. The insertion of additional spacer sequences such as as the furin cleavage site or a glycine-serine- glycine (GSG) spacer flexible linker can yield better cleavage.

The combination of 2A peptide sequences in the following order, namely T2A, P2A, and E2A, is optimal when creating multicistronic vectors containing four genes.

In some embodiments, the hairpin loop ended self-complementary double stranded covalently closed linear polyribonucleotide can comprise one or more 2A peptides.

### Non-Coding Sequences

### Regulatory Nucleic Acids

In some embodiments, the hairpin loop ended self-complementary double stranded covalently closed linear polyribonucleotide may comprise at least one translation competent sequence that encodes a regulatory nucleic acid. In some embodiments the regulatory nucleic acid sequence may modify the expression of an endogenous or exogenous gene. In some embodiments, the regulatory nucleic acid may comprise at least one non-translation competent sequence that encodes a regulatory nucleic acid, such as, but not limited to, exRNA, hnRNA, IncRNA, microRNA, rRNA, shRNA, snoRNA, snRNA, siRNA, scaRNA, Y RNA, piRNA, miRNA, tRNA and rRNA, iRNA. In some embodiments the regulatory non-coding RNA sequences may hibridize to an endogenous genetic sequence or an exogenous genetic sequence such as viral DNA or RNA.

In some embodiments the non-coding regulatory RNA sequences can comprise a guide RNA (gRNA) or a single guide RNA (sgRNA) that may recognize specific DNA sequences. In some embodiments the sgRNA mimics a naturally occurring crRNA-tracrRNA complex and may contain both a tracrRNA that binds to the nuclease, and at least one crRNA that guides the nuclease to the sequence targeted for editing. In some embodiments, the gRNA and sgRNA is used as a part of the CRISPR/Cas system for gene editing.

In some embodiments, the hairpin loop ended self-complementary double stranded covalently closed linear polyribonucleotide can comprise a sequence encoding one or more peptides or polypeptides, one or more regulatory sequences, one or more regulatory nucleic acids, one or more non-coding RNAs, one or more expression sequences, and any combination thereof.

### Safety Switch

In some embodiments, the hairpin loop ended self-complementary double stranded covalently closed linear polyribonucleotide of this invention, may include a "safety switch system". This "safety switch" is represented by a regulatable expression unit comprising at least one inducible promoter such as, but not limited to, tetracycline-inducible promoter, cumate-inducible promoter, rapamycin-inducible promoter, at least one copy of a suitable operator, at least one Kozak sequence, at least a coding sequence encoding a transcriptional activator-like effector nuclease (TALEN)-Fok1 fusion protein, where the DNA recognition domain is designed to target a specific n-mer sequence termed "S4" (*Safety Switch System Sequence*)*,* plus a transcription terminator.

In other embodiments, another expression unit drives the expression of the regulatory rTetT-VP16 fusion protein which regulates the expression of the aforementioned tetracycline-inducible unit, comprising a constitutive promoter, a Kozak' sequence, and the cds corresponding to said regulatory fusion protein, followed by a transcription terminator. The hairpin loop ended self-complementary double-stranded linear DNA molecule bears two mutually facing S4 n-mers strategically placed to direct a double stranded cleavage in the molecule, when expression of TALEN-Fok1 is induced by tetracycline or doxycycline. In summary, administration of tetracycline/doxycycline will induce the expression of a TALEN-Fok1 fusion protein that selectively cleaves the hairpin loop ended self-complementary double-stranded linear DNA molecule in the region between the two S4 n-mers, rendering dsDNA free 5' and 3' ends which are susceptible to exonucleolytic degradation by naturally occurring cellular DNAses, thus, ending the physiological activity of the therapeutic DNA molecule.

In an alternative approach, the inducible promoter drives the expression of a cytotoxic or pro-apoptotic gene which promotes cell death upon induction by administration of a drug such as -but not limited to- tetracycline/doxycycline, cumate or rapamycin, thus ending the physiological activity of the therapeutic strategy. One example of these cytotoxic proteins is *Herpes simplex'*s thymidine-kinase, which metabolizes the nontoxic drug ganciclovir into ganciclovir-triphosphate, which incorporates into DNA in replicative cells, inhibiting DNA synthesis and resulting in cell death.

In a preferred embodiment, the enhancer sequence is selected from the group consisting of constitutive mammalian enhancers.

In another preferred embodiment, the post-transcriptional regulatory element is selected from the group consisting of Hepatitis B virus (HPRE) regulatory element and Woodchuck Hepatitis virus (WPRE) regulatory element.

In a preferred embodiment, the synthetic or wild-type IRES sequence is selected from the group consisting of encephalomyocarditis virus (ECMV) IRES, Coxackievirus B3 (CVB3) IRES, Taura Syndrome Virus (TSV) IRES, Triatoma Virus (TV), Human mastadenovirus C (HAdV-C), Human adenovirus 5 (HAdV5), Human adenovirus 7 (HAdV7), Human mastadenovirus B (HAdV-B), Hepatitis GB virus B (HGBV-B), HPV31, Human Poliovirus 3 (HPV-3), Human papillomavirus type 11 (HPV11), Human immunodeficiency virus 1 (HIV-1), Human immunodeficiency virus 2 (HIV-2), Simian T-lymphotropic virus 1 (STLVs-1), Human Parvovirus B19 (HPB19), Human betaherpesvirus 6B (HHV-6B), Human alphaherpesvirus 3 (HHV-3), Human papillomavirus type 41 (HPV41), Human papillomavirus type 6b (HPV6b), Alphapapillomavirus 7, Friend murine leukemia virus, Heilovirus (ThV), Rous sarcoma virus (RSV), Human mastadenovirus F (HAdv-F), Human papillomavirus type 4 (HPV4), Human papillomavirus type 63 (PHV63), Human mastadenovirus A, Feline immunodeficiency virus (FIV), Human T-lymphotropic virus 2 (HTLV-2), Jaagsiekte sheep retrovirus (JSRV), Spleen focus-forming virus (SFFV), Mouse mammary tumor virus (MMTV), Murine osteosarcoma virus (MOV), Ovine lentivirus (OLV/OvLV), Squirrel monkey retrovirus (SMRV), Human papillomavirus type 16 (HPV16), Human papillomavirus type 5 (HPV5), Human polyomavirus 1, Rabies lyssavirus, Simian immunodeficiency virus (SIV), Human papillomavirus type 10 (HPV10), Human papillomavirus type 26 (HPV26), Human papillomavirus type 32 (HPV32), Human papillomavirus type 34 (HPV34), Marburg marburgvirus, Enterovirus A, Rhinovirus A, Human betaherpesvirus 6A (HHV-6A), Macaca mulatta polyomavirus 1, Snakehead retrovirus (SnRV), Bovine foamy virus (BFV), Drosophila C virus (DCV), Hendra henipavirus (HeV), Aichi virus 1 (AiV-1), Influenza B virus (IBV), Zaire ebolavirus (ZEBOV), Human coronavirus 229E (HCoV-229E), Nipah henipavirus (NiV), Human respirovirus 1 (HPIV-1), Modoc virus (MODV), Sudan ebolavirus, Human parvovirus 4 G1, Rotavirus C, Human gammaherpesvirus 4 (Epstein-Barr virus), eukaryotic IRES derived from *Homo sapiens, Aplysia californica, Canis lupus familiaris, Drosophila melanogaster, Gallus gallus, Mus pahari*, *Mus musculus, Saccharomyces cerevisiae, Zea mays, Rattus norvegicus, Ovisaries,* SYTE-IRES1, SYTE-IRES2, and combinations thereof.

The synthetic transcriptional unit may further comprise a plurality of permuted Intron-Exon sequences.

In a further preferred embodiment, the permuted Intron-Exon sequence is selected from the group consisting of self-splicing group I intron fragment, group II intron fragment introns, and combinations thereof.

In a preferred embodiment, the at least one DNA nuclear targeting sequence included in the parental plasmid, and/or plasmids pDNAv_A and pDNAv_B is an SV40 enhancer DNA nuclear targeting sequence (SEQ ID NO 2).

In another embodiment, the at least one DNA nuclear targeting sequence included in plasmids pDNAv_A and pDNAv_B is the NFkB importin DNA nuclear targeting sequence (SEQ ID NO 3).

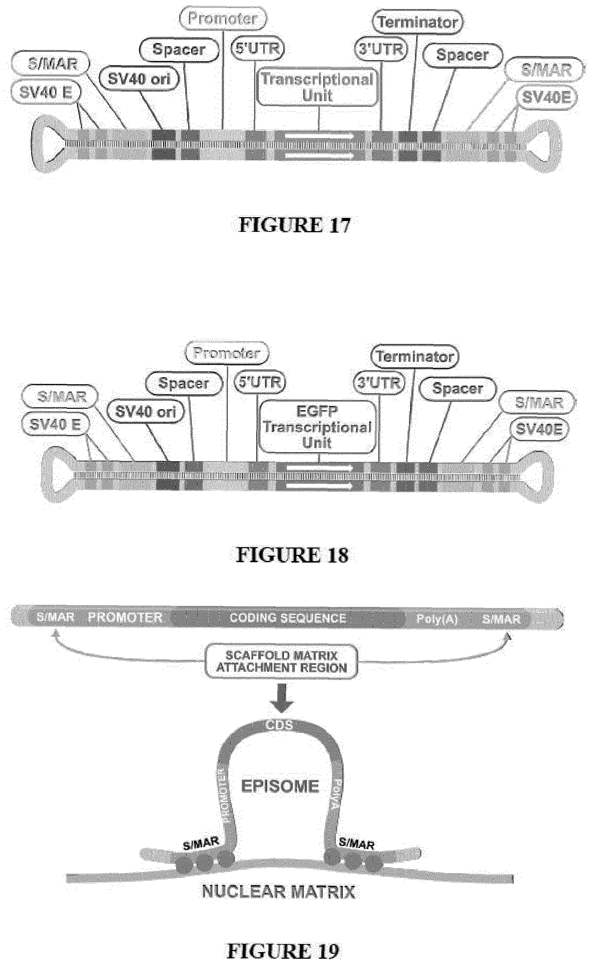

In order to obtain a long-term expression of a protein of interest, DNA molecules can be engineered to maintain a certain stability in the cell nucleus. For that purpose, several viral-derived and autologous DNA sequences can be added to the linear DNA molecules. Chromatin of eukaryotic cells have Matrix Attachment Regions (MARs) that are operationally defined as A/T-rich DNA elements that bind specifically to the protein nuclear matrix. They are frequently located in gene-rich areas associated with transcribed units and there is evidence that they can reduce or eliminate some forms of gene silencing. Genes flanked by MAR sequences have been shown to exhibit less variation in expression among independent transgenic plants or cell culture lines. In particular, MARs appear to prevent silencing caused by multiple transgene copies *in cis.* There is conjecture as to the precise role of MAR sequences in transgene cassettes, but current hypothesis suggest that they may act as boundary elements facilitating the formation of decondensed chromatin loops that in turn enhance transgene transcription and reduce some forms of gene silencing (Figure 19).

Therefore, it is contemplated the addition of S/MAR sequences to the parental plasmids of the present invention (Figure 17 and 18).

In a preferred embodiment, the S/MAR sequence is SEQ ID NO 4.

The placement of an SV40 origin of replication in the vicinity of an S/MAR sequence in a genetic construct has the capability of driving replication of said genetic construct at very low copy numbers, in a cell cycle-synchronized fashion, and is stably maintained without selection for more than 100 generations. This becomes especially relevant when considering a therapeutic approach that requires transfection of replicative cells with the hairpin loop ended self-complementary double stranded covalently closed linear DNA molecule of this invention.

Therefore, the addition of an SV40 origin of replication to the parental plasmid of the present invention is contemplated.

MCS1 and MCS2 are composed by at least one restriction enzyme recognition sequence known in the art, such as, but not limited to, EcoRl, Xbal, BamHl, Xhol, Xbal, Hindlll, EcoRV, Pstl, Kpnl, Stul, Sall, Ncol, Ndel, Notl, Spel and Bstel.

In a preferred embodiment the restriction recognition sites for MCS1 are Sail (SEQ ID NO 5), Pstl (SEQ ID NO 6) and Nsel (SEQ ID NO 7).

In a preferred embodiment the restriction recognition sites for MCS2 are EcoRI (SEQ ID NO 8), Sdal (SEQ ID NO 9) and Xhol (SEQ ID NO 10).

Origin of replication 1 can be selected from any inducible origin of replication such as, but not limited to, Pir-R6K, trfA-oriV, etc.

In a preferred embodiment of this invention, the selected Origin of replication is R6K and the replication protein 1 is Pi protein coding sequence (SEQ ID NO 11).

Appropriate Origin of replication 2 derived from a bacteria, phage, or combinations can be selected from any "rolling circle" replication system such as, but not limited to, M13 Ori (M13 g2p), PhiX174 Ori (protein A), pT181 (RepC).

In a preferred embodiment, the origin of replication of this invention comprises the M13 origin of replication and, in particular, the M13 Ori(+) (SEQ ID NO 12), to enable the generation of single stranded covalently closed circular DNA molecules.

In some embodiments, a recombinant transcriptional unit comprises a promoter operably linked to a second nucleic acid sequence that may be an open reading frame that encodes a genetic product. In some embodiments, a recombinant transcriptional unit may comprise nucleic acids combined in such a way that these nucleic acids are extremely unlikely to be found in nature.

In some embodiments, the transcriptional unit comprises a terminator or termination sequence operably linked to the open reading frame.

In some embodiments, the transcriptional unit comprises at least one synthetic or non-natural promoter, at least one synthetic or non-natural coding sequence and at least one synthetic or non-natural terminator.

In some embodiments the promoters and regulatory elements may include novel proprietary sequences.

According to a second aspect of the present invention, a helper plasmid (illustrated in Figure 2, SEQ ID NO 13) comprising a first and a second synthetic genetic construction is disclosed, wherein the first synthetic genetic construction comprises at least a first repressor protein under at least a first constitutive promoter, at least a second repressor coding sequence under at least a second constitutive promoter, at least a third repressor coding sequence under at least a third constitutive promoter, at least one endonuclease recognition sequence, and wherein the second synthetic genetic construction comprises at least one DNA replicating protein under the control of a first inducible promoter, at least one recombinase protein coding sequence under the control of a second inducible promoter, and at least one endonuclease coding sequence under the control of a third inducible promoter.

In a preferred embodiment, the helper plasmid also contains an endonuclease recognition sequence to permit the elimination of the Helper Plasmid prior to the purification of the linear DNA molecules.

In some embodiments, constitutive promoters are selected from the group consisting of P(Bla) constitutive promoter, P(Cat) constitutive promoter, P(Kat) constitutive promoter, Reverse lambda cl-regulated promoter, pBAD reverse constitutive promoter, lacQ constitutive promoter, GlnRS constitutive promoter, and combinations thereof.

In some embodiments, the inducible promoters are selected from the group consisting of L-Arabinose inducible promoter, IPTG inducible promoter, Lactose inducible promoter, Glucose inducible promoter, Tetracycline inducible promoter, Temperature inducible promoter, pH inducible promoter, HSL inducible promoter, Maltose inducible promoter, Xylose inducible promoter, Phosphate inducible promoter, Cold-Shock inducible promoter, glnG transcription enhancer inducible promoter, and combinations thereof.

In some embodiments, the Endonuclease Recognition Sequence is selected from the group consisting of I- Scel endonuclease recognition sequence, F-Scel endonuclease recognition sequence, F-Scell endonuclease recognition sequence, I-Anil endonuclease recognition sequence, I-CeuI endonuclease recognition sequence, I-Chul endonuclease recognition sequence, I-CpaI endonuclease recognition sequence, I-Cpall endonuclease recognition sequence, I-Crel endonuclease recognition sequence, I-Csml endonuclease recognition sequence, I-Dmol endonuclease recognition sequence, I-PorI endonuclease recognition sequence, I-Scal endonuclease recognition sequence, I-SceII endonuclease recognition sequence, I-SceIII endonuclease recognition sequence, I-SceIV endonuclease recognition sequence, PI-Scel endonuclease recognition sequence, Pi-Pspi endonuclease recognition sequence, PI-Tlil endonuclease recognition sequence, F-Suvl endonuclease recognition sequence, F-Tevl endonuclease recognition sequence, F-Tevll endonuclease recognition sequence, I-DirI endonuclease recognition sequence, I-HmuI endonuclease recognition sequence, I-HmuII endonuclease recognition sequence, I-PpoI endonuclease recognition sequence, I- TevIII endonuclease recombination sequence, and combinations thereof.

The helper plasmid may further comprise a bacterial genome integration cassette.

Examples of suitable recombinases for use in the present invention can be selected from, but are not limited to, Cre, Flp, phiC31, Lambda, ResT, whose target sequences are: locus of X-over P1 (loxp); short flippase recognition target (FRT); attachment sites B/P/L/R (attB/P/UR); cos site; respectively.

The expression of recombinases, DNA replicating proteins and endonucleases is under the control of inducible promoters selected form the group consisting of *E. coli* σ70 promoters such as -but not limited to- L-Arabinose inducible promoter, IPTG inducible promoter, Lactose inducible promoter, Glucose inducible promoter, Tetracycline inducible promoter, Temperature inducible promoter, pH inducible promoter, HSL inducible promoter, Maltose inducible promoter, Xylose inducible promoter, Phosphate inducible promoter, or where the inducible promoter comprises *E*. *coli* σ32 promoters such as Cold-Shock inducible promoter, or where the inducible promoter comprises *E*. *coli* σ54 promoters such as glnG transcription enhancer inducible promoter, and combinations thereof.

The expression of the repressor proteins is under the control of constitutive promoters selected from the group consisting of *E*. *coli* σ70 promoters such as -but not limited to P(Bla) constitutive promoter, P(Cat) constitutive promoter, P(Kat) constitutive promoter, Reverse lambda cl-regulated promoter, pBAD reverse constitutive promoter, lacQ constitutive promoter, GlnRS constitutive promoter, and combinations thereof.

According to one embodiment of this invention, the constitutive promoter of the repressor protein operon is the constitutive promoter pTac, (SEQ ID NO 14).

In other embodiments of this invention, the first repressor protein CDS is Laclq (SEQ ID NO 15), which represses the first inducible promoter from the first expression cassette and permits the expression of the Recombinase 1 protein.

In further embodiments, the second repressor protein CDS is AraC (SEQ ID NO 16) that represses the second inducible promoter from the second expression cassette and permits the expression of the Endonuclease and DNA Replication proteins.

In alternative embodiments, the third repressor protein CDS is Lambda repressor protein (SEQ ID NO 17) that represses the third inducible promoter from the third expression cassette and permits the expression of the Recombinase 2 protein.

Exemplary "rolling circle" replication proteins from the second expression cassette comprise, M13 g2p, *Staphylococcus aureus* RepC, *Pyrococcus abyssi* pGT5, *Streptococcus pneumoniae* pLS1, amongst others.

In a preferred embodiment of this invention the DNA replication protein is M13 g2p (SEQ ID NO 18).

In accordance to possible embodiments, the Recombinase 2 CDS comprises at least one phage-derived telomerase such as N15 phage telomerase TeIN, PY54 phage telomerase TeI VP882 phage TeIK, ΦHAP-1 phage Tel, or wherein the Recombinase comprises at least one bacteria-derived telomerase such as *B. burgdorferi* telomerase, *B. hermsii* telomerase, *B. parkeri* telomerase, *B. recurrentis* telomerase, *B. turicatae* telomerase, *B. anserine* telomerase, *A. tumefaciens* C58 ResT telomerase, or combinations thereof, and the circular covalently closed plasmid DNA vector incorporates the target binding sequence for at least said recombinase and said system produces a linear covalently closed DNA molecule.

In a further preferred embodiment, the Recombinase 2 coding sequence is TelN (SEQ ID NO 19)

According to some embodiments, the Recombinase 2 coding sequence from the first expression cassette in the recombinant *Escherichia coli* is selected from the group consisting of the ResT site, the IoxP site, ϕpK02 telRL site, the FRT site, phiC31 attP site and the λ attP site.

In a preferred embodiment of this invention, the Recombinase 2 coding sequence corresponds to PhiC31 (SEQ ID NO 20).

In some embodiments, the Endonuclease coding sequence of the second expression cassette comprises at least one of the "LAGLIDADG" homing endonuclease family such as I- Scel endonuclease, F-Scel endonuclease, F-Scell endonuclease, I-Anil endonuclease, I-CeuI endonuclease, I-Chul endonuclease, I-CpaI endonuclease, I-Cpall endonuclease, I-Crel endonuclease, I-Csml endonuclease, I-Dmol endonuclease, I-PorI endonuclease, I-Scal endonuclease, I-SceII endonuclease, I-SceIII endonuclease, I-SceIV endonuclease, PI-Scel endonuclease, Pi-Pspi endonuclease, Pi-Tiii endonuclease, F-Suvl, or the endonuclease comprises at least one the GIY-YIG endonuclease family such as F-Tevl endonuclease, F-Tevll endonuclease, or the endonuclease comprises at least one of the His-Cys-His Box endonuclease family such as I-DirI, I-HmuI endonuclease, I-HmuII endonuclease, I-PpoI endonuclease, I- TevIII, or combinations thereof.

In a preferred embodiment of this invention, the Endonuclease coding sequence of the second expression cassette is I-SceI endonuclease (SEQ ID NO 21).

In a preferred embodiment the genetic sequence of the Helper Plasmid is SEQ ID NO 13.

It is contemplated the use of this helper plasmid within a recombinant bacterium as a system together with parental plasmids, wherein said recombinant bacteria is a non -genome recombinant bacteria.

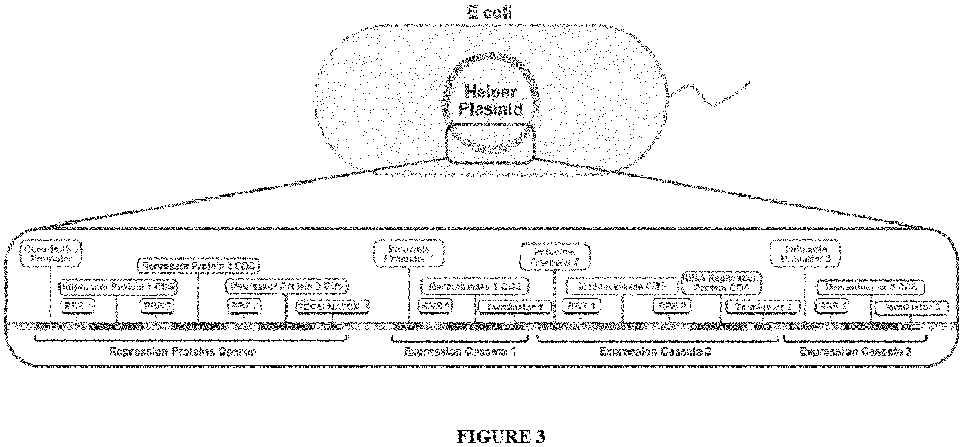

According to a third aspect of the present invention, a recombinant cell is provided, as illustrated in Figure 3.

Suitable cells for use in the present invention may be bacterial cells such as *Escherichia coli* cells, yeast cells such as *Saccharomyces cerevisiae,* and mammalian cells such as HEK-293 cells.

In an embodiment of this invention, the recombinant cells are *Escherichia coli,* K12 strain derivative, as shown in Figure 3.

Genome-Recombinant Cells of the invention may be engineered based on techniques well-established in the art, for example, using bacteriophage integrases, which integrate Helper Plasmid DNA sequences into bacterial chromosomes in a single recombination step.

Alternatively, Episomically-Recombinant Cells of the invention may be engineered based on transformation of the Helper Plasmid using techniques well-established in the art, for example, CaCl₂/heat shock transformation or electroporation.

As depicted in Figures 2 and 3, such engineered recombinant cells harbors the Helper Plasmid which further comprises a repression operon that permits the constitutive expression of at least three repressor proteins (first repressor protein, second repressor protein, and third repressor protein) from at least one constitutive promoter; and further harbors at least three expression cassettes, each cassette comprising at least one inducible promoter, at least one ribosome binding site (RBS), at least one protein coding sequence, and at least one bacterial terminator.

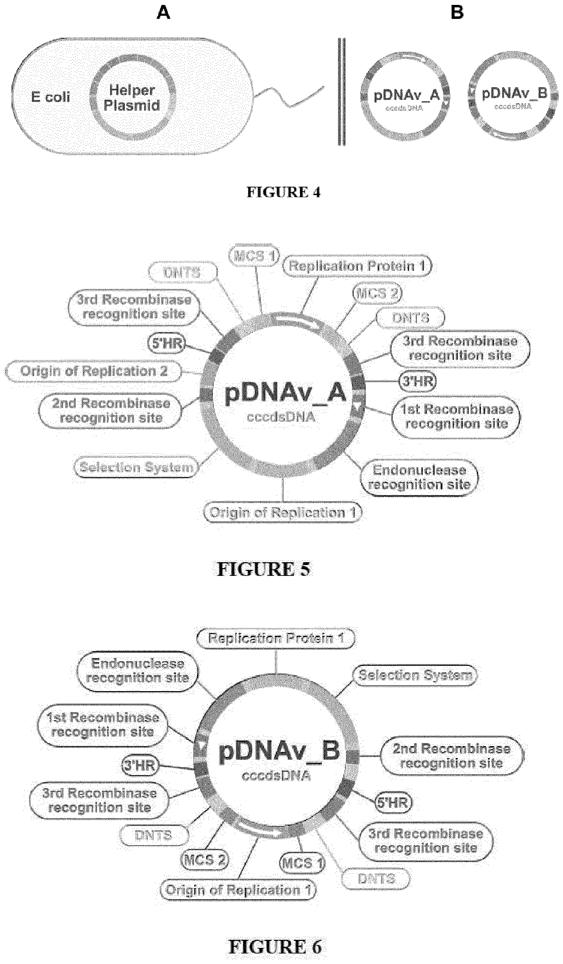

According to a fourth aspect of the invention, a hairpin loop ended self-complementary double-stranded linear covalently closed DNA vector production system (depicted in Figure 4) is provided, wherein such system comprises: the recombinant cell; and at least two of the engineered parental circular covalently closed synthetic plasmids DNA, housed in the recombinant cell, wherein the synthetic transcriptional units of the at least two engineered parental circular covalently closed synthetic plasmids DNA are cloned in opposite directions.

In some specific embodiments, the production system comprises the recombinant cell and the parental plasmids hereinafter referred to as pDNAv_A and pDNAv_B.

Both pDNAv_A (illustrated in Figure 5) and pDNAv_B (illustrated in Figure 6) comprise a first bacterial origin of replication (Origin of replication 1), at least one Replication Protein 1 that acts upon the Origin of Replication 1, at least one bacterial selection system, at least three target sequences for at least three recombinases (1st recombinase recognition site, 2nd recombinase recognition site, 3rd recombinase recognition site), at least two multiple cloning sites (MCS 1, MCS 2) flanked in both ends by at least one recombinase target site (2nd recombinase recognition site), and at least two copies of a DNA nuclear targeting sequence (DNTS), and at least two homology regions (5'HR and 3'HR), wherein at least one expression cassette may be cloned between MCS 1 and MCS 2 sequences to allow the expression of a gene of interest in mammalian cells. Preferably, expression cassettes are cloned in opposite directions for templates pDNAv_A and pDNAv_B. Preferably, both plasmids contain at least one copy of an endonuclease recognition sequence.

In embodiments of the present invention, pDNAv_A and/or pDNAv_B further comprise at least one additional ultra-high copy number bacterial origin of replication that can generate more than 5,000 copies per cell.

In embodiments of the invention, pDNAv_A has two bacterial origins of replication. Preferably, one of the bacterial origins of replication for pDNAv_A is R6K origin of replication (SEQ ID NO 22).

in embodiments of the invention, the selection system for pDNAv_A is Ampicillin Resistance Cassette (SEQ ID NO 23).

In embodiments of the invention, the preferred selection system for pDNAv_B is Chloramphenicol Resistance Cassette (SEQ ID NO 24).

Also preferably, the other bacterial origin of replication for pDNAv_A is M13 origin of replication (SEQ ID NO 12).

In further embodiments, pDNAv_B has one bacterial origin of replication. Preferably, the bacterial origin of replication of pDNAv_B is the R6K origin of replication (SEQ ID NO 22).

In a preferred embodiment the genetic sequence of pDNAv_A is SEQ ID NO 25 (illustrated in figure 5)

In another preferred embodiment the genetic sequence of pDNAv_B is SEQ ID NO 26 (illustrated in figure 6)

According to a fifth aspect of the invention, a hairpin loop ended self-complementary double-stranded linear covalently closed DNA vector production process is provided, starting from the aforementioned system, wherein said process comprises the stages of:
a. cloning the desired synthetic transcriptional unit between two of the multiple cloning sites in two of the engineered parental circular covalently closed synthetic plasmids DNA in order to generate plasmids pDNAv_Ac and pDNAv_Bc;
b. in order to generate the cointegrate plasmid pDNAv_ABc, alternatively subjecting both pDNAv_Ac and pDNAv_Bc to a first *in vitro* recombination using the first recombinase recognition sequence, or subjecting both pDNAv_Ac and pDNAv_Bc to a first *in vitro* recombination using restriction enzyme digestion in the first recombinase recognition sequence and subjecting digested pDNAv_Ac and pDNAv_Bc to ligation, or subjecting both pDNAv_Ac and pDNAv_Bc to a recombination including PCR amplification of each plasmid and the use of *in vitro* recombination systems;
c. transforming the cointegrate plasmid pDNAv_ABc with rolling circle and theta replication capacity into the recombinant cell and selecting recombinant clones;
d. alternatively subjecting the recombinant cell to incubation in growth conditions to allow theta replication of plasmid pDNAv_ABc, inducing the expression of the second recombinase in the recombinant cell to allow the generation of the Linear DNA molecules and inducing the expression of the endonuclease to allow the degradation and elimination of a resulting parental recombination product and the helper plasmid, or subjecting the cointegrate plasmid pDNAv_ABc to a second *in vivo* recombination using the second recombinase recognition sequence in order to generate pDNA_ABc1 and pDNA_ABc2, inducing the expression of the DNA replicating protein and endonuclease in order to allow "rolling circle" replication of pDNA_ABc1 and self-annealing of the single-stranded DNA products of the rolling circle replicated-DNA into hairpin loop ended double-stranded DNA molecules and allow the degradation of pDNAv_ABc2 by the endonuclease activity and inducing the expression of the second recombinase in the recombinant cell to allow the cleavage and elimination of the loop; and
e. isolating the resulting hairpin loop ended self-complementary double-stranded linear covalently closed DNA molecules from the recombinant cell.

In possible embodiments of the invention, the process particularly comprises the steps of:
1. cloning the desired synthetic transcriptional unit between two of the multiple cloning sites in two of the engineered parental circular covalently closed synthetic plasmids DNA in order to generate plasmids pDNAv_Ac and pDNAv_Bc;
2. in order to generate the cointegrate plasmid pDNAv_ABc, alternatively subjecting both pDNAv_Ac and pDNAv_Bc to a first recombination using the first recombinase recognition sequence, or subjecting both pDNAv_Ac and pDNAv_Bc to a first recombination using restriction enzyme digestion in the first recombinase recognition sequence and subjecting digested pDNAv_Ac and pDNAv_Bc to ligation, or subjecting both pDNAv_Ac and pDNAv_Bc to a recombination including PCR amplification of each plasmid and the use of in vitro recombination systems;
3. transforming the cointegrate plasmid pDNAv_ABc with rolling circle and theta replication capacity into the recombinant cell and selecting recombinant clones;
4. subjecting the cointegrate plasmid pDNAv_ABc to a second recombination in vivo using the second recombinase recognition sequence to generate pDNA_ABc1 and pDNA_ABc2;
5. inducing the DNA replicating protein in order to allow "rolling circle" replication of pDNA_ABc1 and self-annealing of the single-stranded DNA products of the rolling circle replicated-DNA into hairpin loop ended double-stranded DNA molecules, and inducing the endonuclease coding sequence to allow the degradation of pDNAv_ABc2 by the endonuclease activity;
6. inducing the third recombinase recognition sequence in the recombinant cell to allow the cleavage and elimination of the loop; and
7. isolating the resulting hairpin loop ended self-complementary double-stranded linear covalently closed DNA molecules from the recombinant cell.

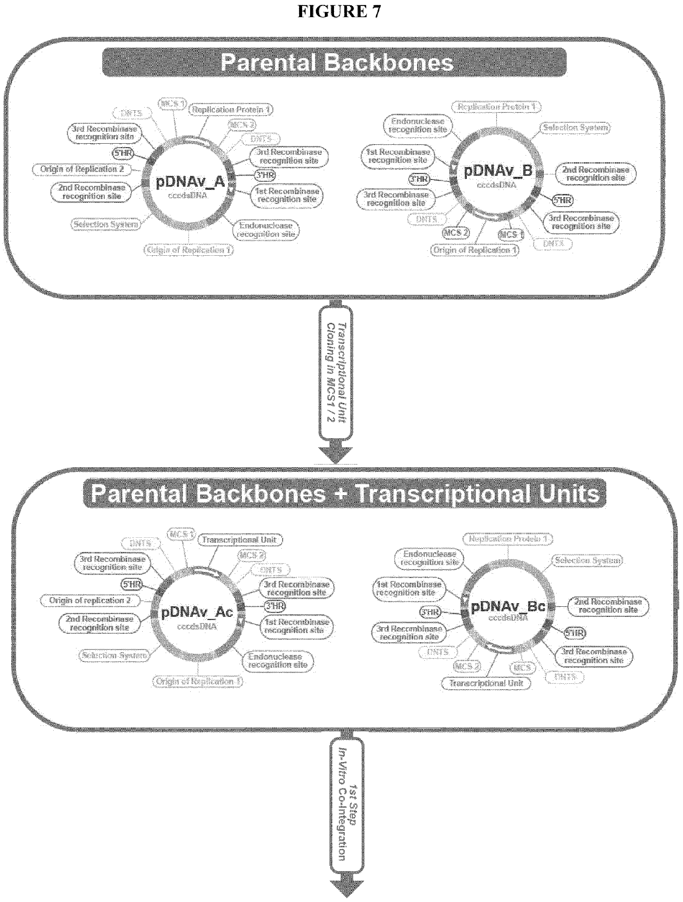

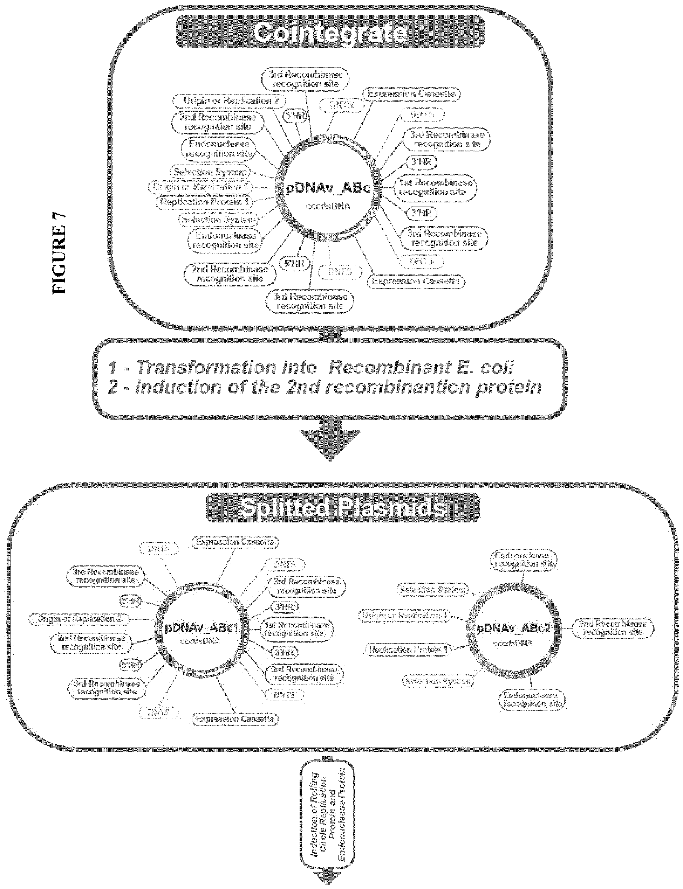

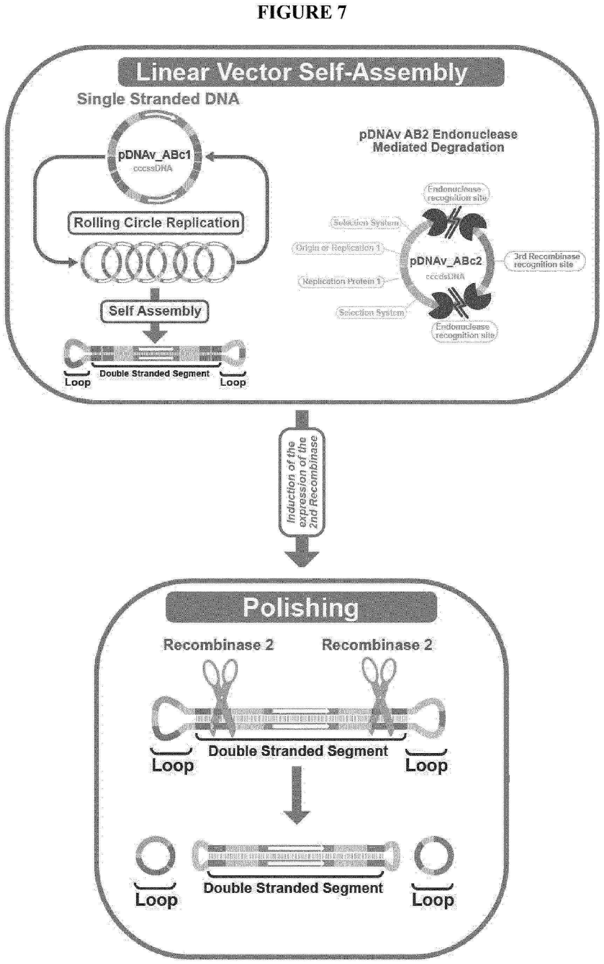

The above process is depicted in Figure 7 and its steps further described below.

### 1. Generation of pDNAv_Ac and pDNA Av_Bc from parental plasmid

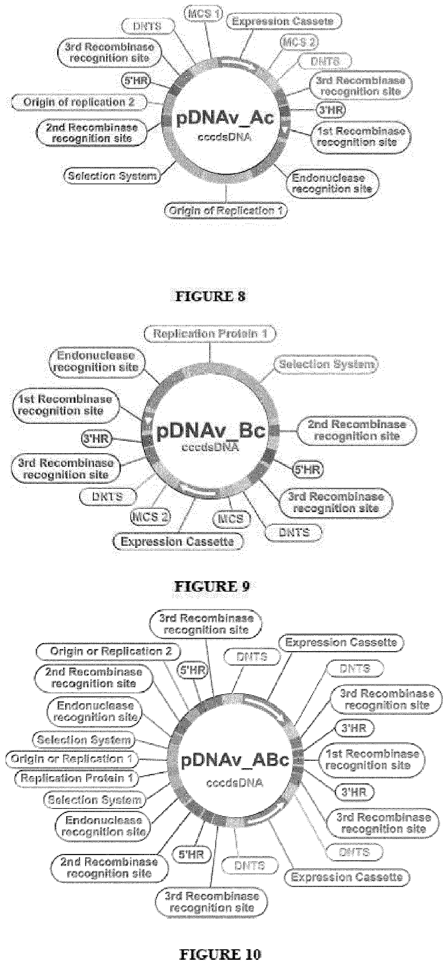

In preferred embodiments, pDNAv_A (Figure 5) and pDNAv_B (Figure 6) are subjected to a cloning step where the desired synthetic transcriptional unit is inserted between the two multiple cloning sites in two of the engineered parental circular covalently closed synthetic plasmid DNA molecules in order to generate plasmids pDNAv_Ac (Figure 8) and pDNAv_Bc (Figure 9).

### 2. First recombination (in vitro):generation of the cointegrate plasmid

After the selection of the correct clones pDNAv_Ac and pDNAv_Bc, both are subjected to a first *in vitro* recombination step using the first recombinase recognition sequence. Alternatively, pDNAv_Ac and pDNAv_Bc can be subjected to a first *in vitro* recombination by using restriction enzyme digestion of the endonuclease recognition sequence embedded in the first recombinase recognition sequence, and subjecting digested pDNAv_Ac and pDNAv_Bc to ligation. Alternatively, pDNAv_Ac and pDNAv_Bc can be subjected to a first *in vitro* recombination by performing a PCR amplification of each plasmid and subjecting the amplicons generated to *in vitro* commercial recombination systems, in order to generate the cointegrate plasmid pDNAv_ABc (Figure 10).

### 3. Bacterial transformation and selection of recombinant clones

After performing the cointegration step *in vitro,* the cointegrate vector pDNAv_ABc is transformed in the recombinant cell which harbors the Helper Plasmid, and incubated to allow the theta replication and expression of antibiotic resistance genes. After appropriate incubation, recombinant clones are selected.

### 4. Second Recombination (In Vivo): generation of pDNA_ABc1

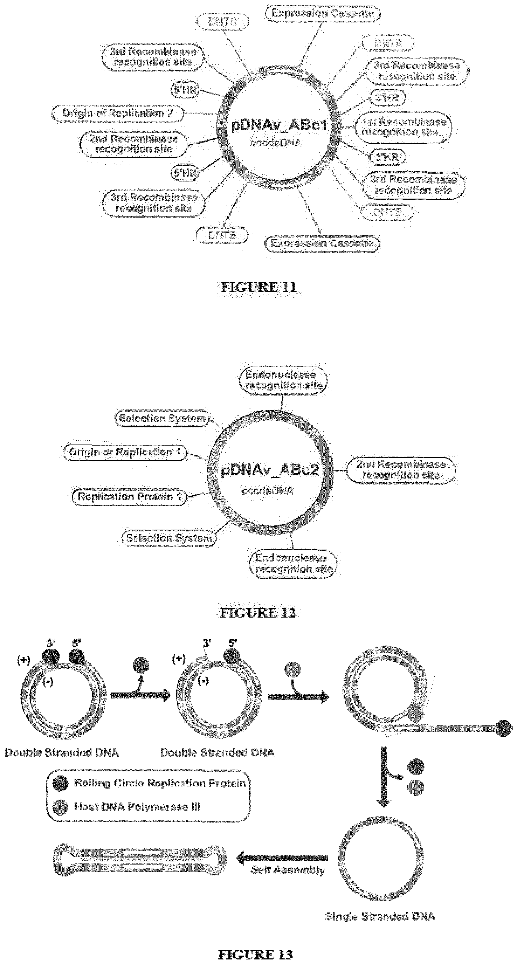

Once the recombinant clones are selected, the cointegrate plasmid pDNAv_ABc is subjected to a second *in vivo* recombination by inducing the expression of the First Recombinase Protein that recognizes the Second Recombinase Recognition Sequence in the co-integrate, in order to generate two derived circular covalently closed DNA molecules: pDNAv_ABc1 (Figure 11) and pDNAv_ABc2 (Figure 12). Plasmid pDNAv_ABc1 comprises the first origin of replication, DNA nuclear localization sequences (DNTS), Nuclear Matrix Attachment Regions (S/MARs), optionally a eukaryotic origin of replication and the transcriptional unit, flanked by the second recombinase recognition sequence and the first recombinase recognition site. On the other hand, pDNAv_ABc2 comprises at least one endonuclease recognition sequence, the bacterial selection system and the second origin of replication.

### 5. Rolling Circle Replication amplification, self-annealing, and degradation of parental DNA

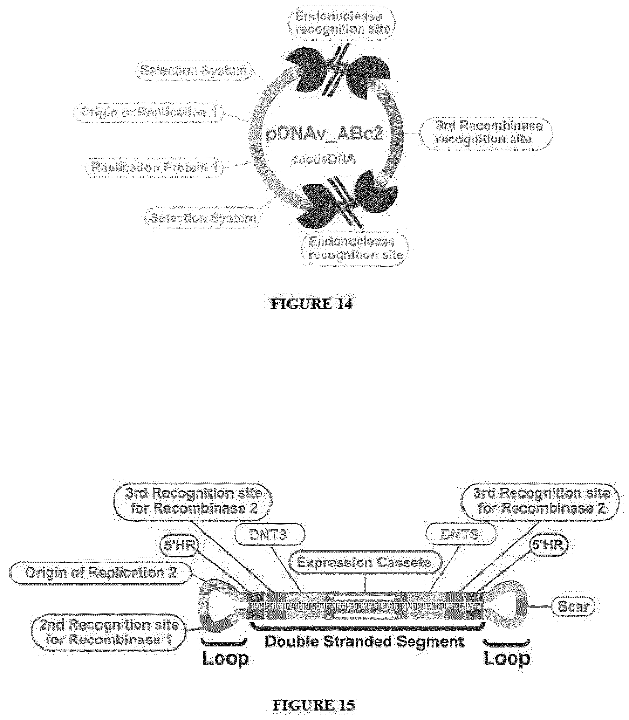

After the generation of pDNAv_ABc1 and pDNAv_ABc2 circular covalently closed DNA molecules, the posterior induction of the DNA replicating protein expression in this step allows the initiation of the "rolling circle" replication of pDNA_ABc1 (Figure 13), which produces circular covalently closed single stranded self-complementary DNA molecules that will spontaneously auto-assemble into hairpin loop ended double-stranded linear DNA molecules (Figure 15). On the other hand, the expression of the endonuclease protein, of which coding sequence is located under the same promoter as the DNA Replicating Protein, allows the degradation of pDNAv_ABc2 and the helper plasmid by the endonuclease activity (Figure 14).

### 6. Hairpin loop ended double-stranded linear DNA molecules polishing

Once pDNAv_ABc2 and the helper plasmid are degraded, the third recombinase protein is induced in order to cleavage and eliminate the loops at both ends of the hairpin loop-ended double-stranded linear DNA molecules, producing the final hairpin loop-ended self-complementary double-stranded linear covalently closed DNA molecules denoted as SYTE_{L}-DNA (Figure 17).

### 7. Isolation of the resulting Linear DNA Molecule

This last step involves the isolation of the resulting hairpin loop-ended self-complementary double-stranded linear covalently closed DNA molecules (SYTE_{L}-DNA) from the recombinant cell, in order to proceed with the formulation for final administration into the target organism or cell.

The isolation can be carried out by methods known in the art for plasmid DNA purification such as Alkaline Lysis, Boiling Lysis, Affinity Column Based Methods among others.

In other embodiments of the invention, the process particularly comprises the steps of:
1. cloning the desired synthetic transcriptional unit between two of the multiple cloning sites in two of the engineered parental circular covalently closed synthetic plasmids DNA in order to generate plasmids pDNAv_Ac and pDNAv_Bc;
2. in order to generate the cointegrate plasmid pDNAv_ABc, alternatively subjecting both pDNAv_Ac and pDNAv_Bc to a first recombination using the first recombinase recognition sequence, or subjecting both pDNAv_Ac and pDNAv_Bc to a first recombination using restriction enzyme digestion in the first recombinase recognition sequence and subjecting digested pDNAv_Ac and pDNAv_Bc to ligation, or subjecting both pDNAv_Ac and pDNAv_Bc to a recombination including PCR amplification of each plasmid and the use of in vitro recombination systems;
3. transforming the cointegrate plasmid pDNAv_ABc with rolling circle and theta replication capacity into the recombinant cell and selecting recombinant clones;
4. subjecting the recombinant cell to incubation in growth conditions to allow theta replication of plasmid pDNAv_ABc;
5. inducing the third recombinase recognition sequence in the recombinant cell to allow the cleavage and elimination of the loop;
6. inducing the endonuclease coding sequence to allow the degradation and elimination of a resulting parental recombination product and the helper plasmid; and
7. isolating the resulting hairpin loop ended self-complementary double-stranded linear covalently closed DNA molecules from the recombinant cell.

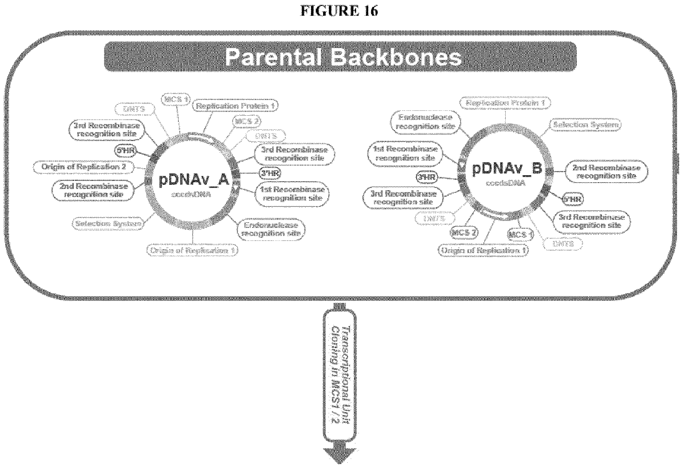

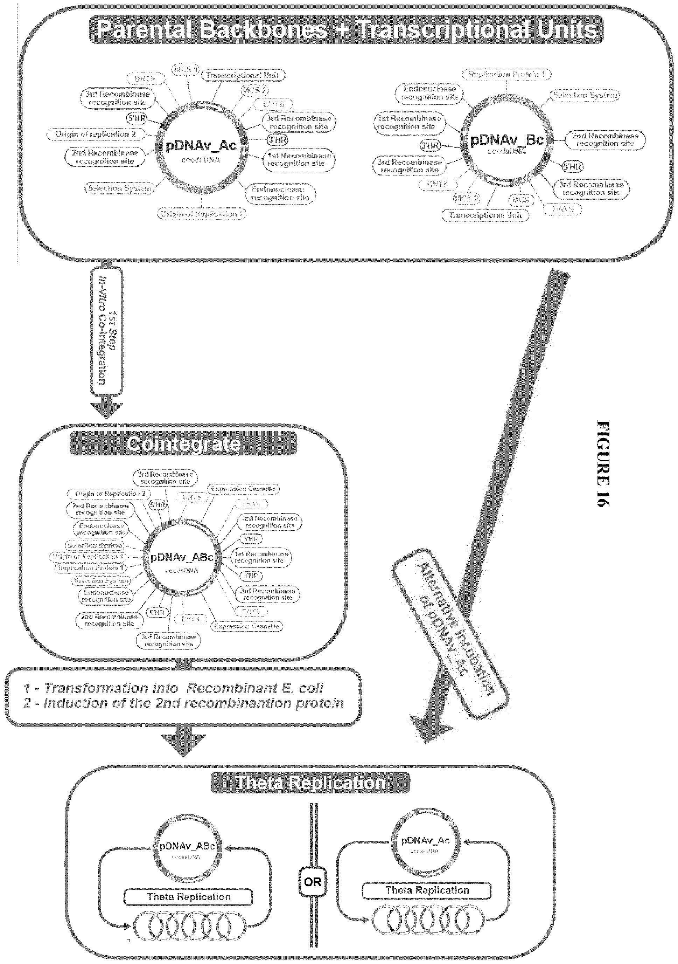

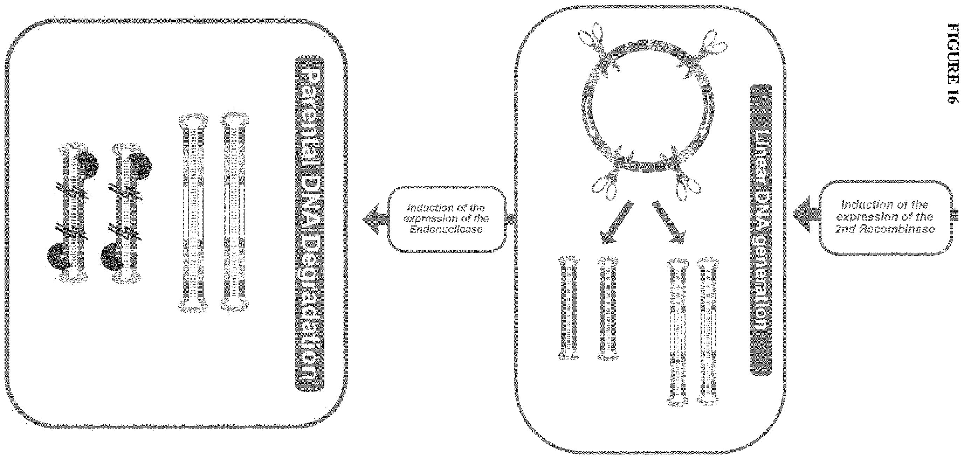

The above process is depicted in Figure 16 and its steps further described below.

### 1. Generation of pDNAv_AC and pDNA Av_Bc from parental plasmid

In preferred embodiments, pDNAv_A (Figure 5) and pDNAv_B (Figure 6) are subjected to a cloning step where the desired synthetic transcriptional unit is inserted between the two multiple cloning sites in two of the engineered parental circular covalently closed synthetic plasmid DNA molecules in order to generate plasmids pDNAv_Ac (Figure 8) and pDNAv_Bc (Figure 9).

### 2. First recombination (in vitro): generation the cointegrate plasmid

After the selection of the correct clones pDNAv_Ac and pDNAv_Bc, both are subjected to a first *in vitro* recombination step using the first recombinase recognition sequence. Alternatively, pDNAv_Ac and pDNAv_Bc can be subjected to a first *in vitro* recombination by using restriction enzyme digestion of the endonuclease recognition sequence embedded in the first recombinase recognition sequence, and subjecting digested pDNAv_Ac and pDNAv_Bc to ligation. Alternatively, pDNAv_Ac and pDNAv_Bc can be subjected to a first *in vitro* recombination by performing a PCR amplification of each plasmid and subjecting the amplicons generated to *in vitro* commercial recombination systems, in order to generate the cointegrate plasmid pDNAv_ABc (Figure 10).

### 3. Bacterial transformation and selection of recombinant clones

After performing the cointegration step *in vitro,* the cointegrate vector pDNAv_ABc is transformed in the recombinant cell which harbors the helper plasmid, and incubated to allow the theta replication and expression of antibiotic resistance genes. After appropriate incubation, recombinant clones are selected.

### 4. Theta replication of the cointegrate

Once proper recombinant clones are selected, the recombinant bacteria are subjected to an incubation step in order to allow theta replication of plasmid pDNAv_ABc.

### 5. Linear DNA molecule generation

After a suitable incubation time, the expression of the second recombination protein is induced in order to produce the hairpin loop-ended double-stranded linear DNA molecules denoted as SYTE_{L}-DNA (Figure 17).

### 6. Degradation of parental DNA

To degrade the resulting residual parental recombination product and the Helper Plasmid, the endonuclease protein expression is induced in order to allow the cleavage of the corresponding endonuclease recognition sites located in the aforementioned DNA molecules.

### 7. Isolation of the resulting Linear DNA Molecule

This last step involves the isolation of the resulting hairpin loop-ended self-complementary double-stranded linear covalently closed DNA molecules from the recombinant cell, in order to proceed with the formulation for final administration into the target organism or cell.

The isolation can be carried out by methods known in the art for plasmid DNA purification such as Alkaline Lysis, Boiling Lysis, Affinity Column Based Methods among others.

In some embodiments of the invention the entire process is carried out *in vivo.*

In other possible embodiments, the entire process is carried out *in vitro.*

In preferred embodiments, all the steps are performed *in vivo,* except from the step of subjecting both parental plasmids (such as pDNAv_Ac and pDNAv_Bc) to a first recombination with the first recombination site in order to generate the co-integrated plasmid pDNAv_ABc that is performed *in vitro.*

In a sixth aspect of this invention, a hairpin loop ended self-complementary double-stranded linear DNA molecule is provided, wherein such molecule is produced by any of the aforementioned processes.

In some embodiments, as illustrated in Figure 15, the hairpin loop ended self-complementary double-stranded linear DNA molecule is composed of non-complementary loops, a homology region to permit the correct self-assembly of the single stranded precursor, at least two copies of a DNA Nuclear Localization Sequence, at least one Transcriptional Unit, and is devoid of bacterial derived antibiotic genes and origins of replication.

In still a further embodiment and with reference to Figures 17 and 18, hairpin loop ended self-complementary double-stranded linear DNA molecule is composed of non-complementary loops, an homology region to permit the correct self-assembly of the single stranded precursor, at least two copies of a DNA Nuclear Localization Sequence, at least one transcriptional unit, at least two copies of episomal maintenance sequences that permit the expression of a coding sequence of interest during a long period of time, optionally at least one mammalian origin of replication, and is devoid of bacterial derived antibiotic genes and origins of replication

Preferably, the genetic elements of the DNA vector are operably linked in the following order: a single-stranded DNA loop, optionally at least one DNA nuclear targeting sequence, optionally at least one S/MAR sequence, optionally at least one eukaryotic origin of replication, at least one promoter, optionally at least one 5' untranslated region sequence, at least one coding sequence or sequence of interest, optionally one or more IRES, MIRES and/or 2A-peptide sequences, optionally at least one 3' untranslated region sequence, at least one transcriptional terminator sequence, optionally at least one S/MAR sequence, optionally at least one eukaryotic origin of replication, optionally at least one DNA nuclear targeting sequence, a single-stranded DNA loop, with suitable DNA spacer sequences optionally placed between at least two of the aforementioned elements.

In a most preferred embodiment, as shown in Figure 17, the genetic elements of the DNA vector are operably linked in the following order: a single-stranded DNA loop, at least two SV40E sequences, at least one S/MAR sequence, at least one SV40 origin of replication, at least one promoter, at least one 5' untranslated region sequence, at least one coding sequence or sequence of interest, at least one 3' untranslated region sequence, at least one transcriptional terminator sequence, at least one S/MAR sequence, at least two SV40E sequences, a single-stranded DNA loop, with suitable DNA spacer sequences optionally placed between at least two of the aforementioned elements.

By way of example, episomal maintenance sequences can be selected from S/MAR sequences, SV40T mutated sequence, β-Globin Replicator sequence, Epstein-Barr virus (EBV) elements oriP and EBNA1.

Preferably, the episomal maintenance sequence is β-Interferon S/MAR sequence (SEQ ID NO 4).

The resulting hairpin loop ended self-complementary double-stranded covalently closed linear DNA vectors may be used as a DNA-based platform for the expression of a wide range of genetic constructs such as, but not limited to, conventional mRNA molecules, self-amplifying RNA molecules, small interfering RNA (siRNA), circular RNA molecules (circRNA), microRNA Sponges, and combinations thereof.

In an embodiment of this invention, the molecules expressed may be used for the prevention or treatment of:
- Oncology related diseases, such as, but not limited to, solid tumors such as carcinomas, sarcomas, myelomas, myomas, melanomas, mixed types, lymphomas and liquid tumors such as leukemias.
- infectious diseases such as, but not limited to diseases derived from viruses, bacteria, fungi or parasites. Non-limiting examples of viral infectious diseases are COVID-19, Dengue, Hantavirus, Chikungunya, Zika, Influenza, Lassa fever, Ebola and Nipah.
- Genetic disorders, with Replacement Gene Therapy (RGT) approach for long term expression of coding sequences of interest for genetic diseases such as, but not limited to, Liver Monogenic Diseases such as Hemophilia A, Hemophilia B, Diabetes; Retina Genetic Disorders such as Leber Congenital Amaurosis, Age-related Macular Degeneration and Stargardt.
- Diseases that may be treated by cell therapy approaches such as Chimeric Antigen Receptor (CAR)-T Cell, Chimeric Antigen Receptor (CAR)-NK Natural Killer Cell Therapies, induced Pluripotent Stem Cells (iPSC), Gene Editing such as Clustered Regularly Interspersed Short Palindromic Repeats (CRISPR)/Cas9 gene editing (or its variants), Transcriptional Activator-Like Effector Nuclease (TALEN) gene editing, Zinc Finger Nuclease (ZFN) gene editing, Meganuclease gene editing.
- Neurodegenerative disorders such as, but not limited to, Alzheimer's disease, ataxia, Huntington's disease, Parkinson's disease, Motor neuron disease, Multiple system atrophy, Amyotrophic Lateral Sclerosis and Progressive supranuclear palsy.

In some embodiments, in order to achieve the expected efficacy, the hairpin loop ended self-complementary double stranded covalently closed linear polyribonucleotide may be formulated with suitable delivery vehicles through known methods. These delivery vehicles include but are not limited to: polymers, polymeric carriers, membrane lipid bilayers, liposomes, lipid nanoparticles, protein nanoparticles, viral-derived envelopes and/or capsids. In some embodiments, delivery methods may include transfection, electroporation and or fusion.

In some embodiments a pharmaceutical composition or formulation refers to a form of administration, e.g., systemic, local, intratumoral, peritumoral, subcutaneous, intramuscular. Suitable forms, in part, depend upon the use or the route of entry, for example oral, inhalation or injection. These compositions or formulations are prepared according to any method known in the art for the manufacture of pharmaceutical compositions.

This invention articulates with other available technologies to produce hairpin loop ended linear covalently closed DNA molecules since it can generate them in great quantity and quality for their administration to mammalian cells, tissues and organs. But, in addition, it overcomes the obstacles posed by such other available technologies. Particularly, because this solution uses a bacterial biological system to produce the hairpin-loop ended linear covalently-closed DNA molecules. This mainly avoids the need for large amounts of recombinant enzymes for use *in vitro,* which are expensive and represent a limiting step to sustain a qualitative and quantitative competitive production with a standardizable, reproducible and transferable procedure.

Regarding to the distinctive characteristics of this invention, it is important to highlight that the disclosed technology is more easily scalable to virtually all volumes in which it has been possible to cultivate *Escherichia coli* in high densities, without requiring an expensive infrastructure (widely used in the traditional biotech industry), consequently reducing time, human resources, dependence on important supplies (which are irreplaceable in the other procedures, such as recombinant enzymes), and procedural steps, since our biological system performs all the necessary tasks to generate the molecules of interest. Thus, the recombinant biological system proposed here, in combination with the particular and specific design of the plasmid vectors of this invention, contains all the engineering that replaces the infrastructure required in other extant systems producing similar DNA molecules. Thus, our system provides a fast and simple way to generate different active ingredients for the development of gene therapies or vaccines, which can then be rapidly produced on a scale suitable for conceptual evaluation (laboratory scale), for its testing in animal models (pre-clinical scale), for its clinical trial in humans or its direct formulation for products registered and approved by regulatory agencies (clinical GMP scale).

Adding to the originality of the present invention, we propose the incorporation of several specific modular genetic elements to the final functional hairpin loop ended linear covalently closed DNA molecules, such as SV40 enhancer, S/MAR and SV40 origin of replication sequences. The combination of these elements allows the encoded transcriptional unit in the molecule to achieve an unsurpassed therapeutic potential by maximizing its nuclear localization, and enhancing its transcriptional activity and stability through the attachment of the molecule to the nuclear matrix. In addition, the functional combination of an SV40 origin of replication with the S/MAR sequence, provides continuous replicative capability in a cell cycle-synchronized fashion, which is essential to avoid the gradual dilution of the hairpin loop ended linear covalently closed DNA molecule as the transfected cells proliferate, thus maintaining the therapeutic effect unaltered as the replication process continues, maximizing the chances for each cell of the progeny of receiving at least one copy of the therapeutic molecule. This feature is of the utmost importance in strategies such as CAR-T or CAR-NK cell therapies, wherein the transformed cells (T-cells or NK lymphocytes) undergo active proliferation upon activation, or for the treatment of diseases like atopic dermatitis or psoriasis, where the transformed cells (keratinocytes, in this example) also proliferate constantly as the skin peels off.

The aforementioned original innovations ensure the achievement of the maximum expression potential of the hairpin loop ended linear covalently closed DNA molecule of this invention as a therapeutic approach, potentially minimizing the need for high concentrations of nucleic acids administered per dose, or even the need for redosings after a single administration.

The assays described below are valid for all the aforementioned aspects of the invention.

### ASSAYS AND EXPERIMENTS

The expression cassettes and the repressor proteins' operon were designed *in-silico* and chemically synthesized, molecularly cloned and sequenced by GeneUniversal Inc. Previous to the full sequence insertion into the *Escherichia coli* genome, a full genetic construction was achieved (e.g., Helper Plasmid; Figure 2). After the full construction sequencing and verification, the latter was inserted into the *Escherichia coli* K12 genome by methods well known in the art. In particular, for this invention, a PhiC31 att method was chosen. To demonstrate the insertion of the full construction into the *Escherichia coli* genome, two PCR assays were performed.

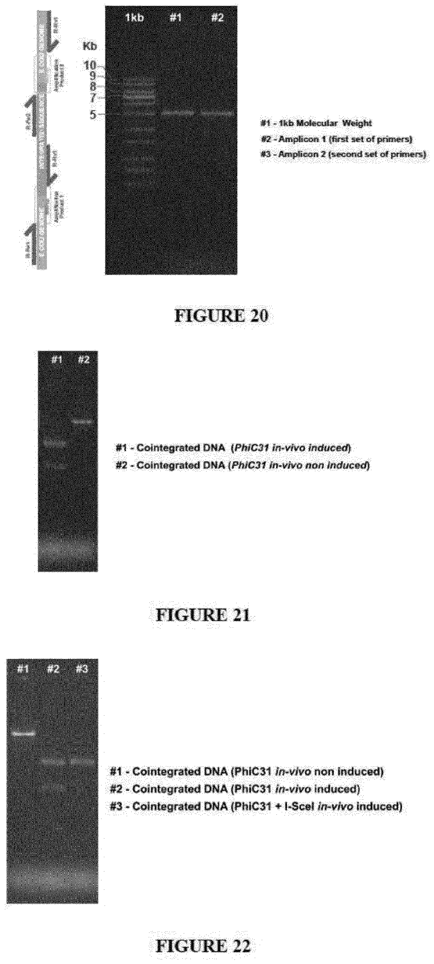

After the PCR was performed, a gel-agarose electrophoresis was carried out, as can be appreciated in Figure 20, two amplicons were obtained (and subsequently Sanger-sequenced), meaning that the full DNA construction was correctly inserted into the *Escherichia coli* genome.

To further demonstrate the functioning of the bacteria, template plasmids were subjected to enzymatic activity from the genes that were inserted in the bacterial genome.

In addition, in order to demonstrate the function of the PhiC31 enzyme, a cointegrated plasmid was generated from pDNAv_A (Figure 5) and pDNAv_B (Figure 6) plasmids and after the induction with IPTG and the appropriate incubation, resulting DNA molecules were extracted via alkaline lysis method. After DNA purification, an agarose-gel electrophoresis was performed showing two circular covalently closed DNA molecules, as appreciated in Figure 21.

To demonstrate the function of the I-SceI enzyme, genome-recombinant *Escherichia coli* having the co-integrate plasmid were subjected to an inductor and after appropriate incubation, extraction, and purification of DNA by alkaline lysis, this was subjected to an agarose gel electrophoresis and stained with ethidium bromide. Only the linear DNA was present as can be seen in Figure 22.

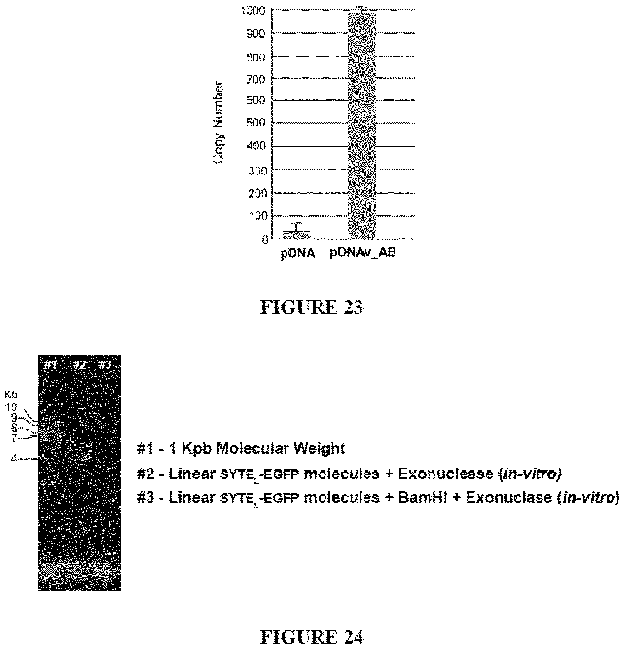

To further test the recombinant bacterial strain, the "rolling circle" replication was tested using a proper DNA template, showing the expected increment in the copies of DNA molecules (Figure 23).

To further validate generation of hairpin loop ended self-complementary double-stranded covalently closed linear DNA vectors, resulting linear DNA molecules from the process described in this invention, were subjected to exonuclease and/or endonuclease activity. Figure 24 shows an agarose gel electrophoresis of the linear DNA molecules *in vitro* treated with exonuclease or exonuclease + endonuclease, where it can be seen that in the no endonuclease reaction there was no degradation, while the reaction that had the endonuclease added as a pretreatment rendered completely degraded DNA after the exonuclease addition.

### Transfection Efficiency Assay of the Linear DNA Vector

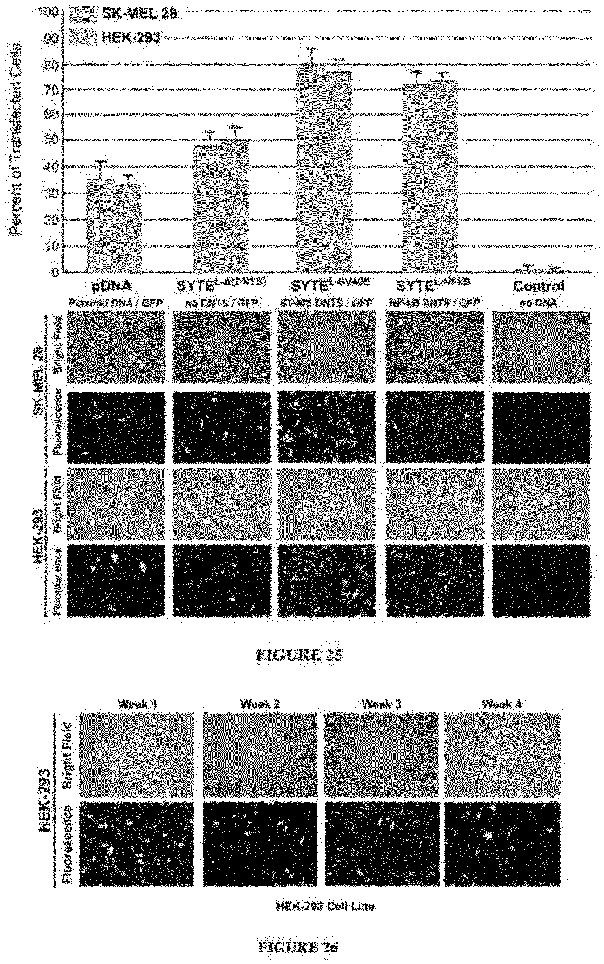

A transformation efficiency assay of the linear DNA molecules was performed (Figure 25). A reporter hairpin loop ended self-complementary double-stranded covalently closed linear DNA molecules containing a EGFP expression cassette was used (Figure 18). Polyethylenimine reagent was obtained from Polysciences, and Lipofectamine 2000 was obtained from Invitrogen. HEK-293 (human embryonic kidney) cells used for this experiment were cultured in Dulbecco's Modified Eagle Medium (DMEM) with 10% FBS at 37° in 5% v/v CO2 and in a humidified atmosphere. All experiments were performed with cells in passage 3. Briefly, one hour before transfection, complete culture medium was replaced with serum-free culture medium, and DNA- Polyethylenimine (DNA-P) or DNA-Lipofectamine (DNA-L) complexes were formed. DNA-P was incubated for 10 minutes at room temperature and DNA-L was incubated for 25 minutes at room temperature. After incubation, serum free medium was removed, DNA-P or DNA-L were added dropwise, and the plates were incubated at 37 °C. After a 2h incubation, the cells were washed, and complete medium was added to each well. The percentage of transfection was assessed after 48 h post transfection as GFP-positive cells/total cells, and the results are shown in Figure 26.

### In Vivo Transfection and Expression Efficiency of EGFP-coding Linear DNA vector

To validate the *in vivo* expression of the Linear DNA molecules, 20 µg of SYTE_{L}-EGFP were formulated with non-targeted lipid nanoparticles and injected in mice *biceps femoris* muscles. Six days after the administration, the muscle was harvested, properly dissected and subjected to a fluorescence microscopy survey.

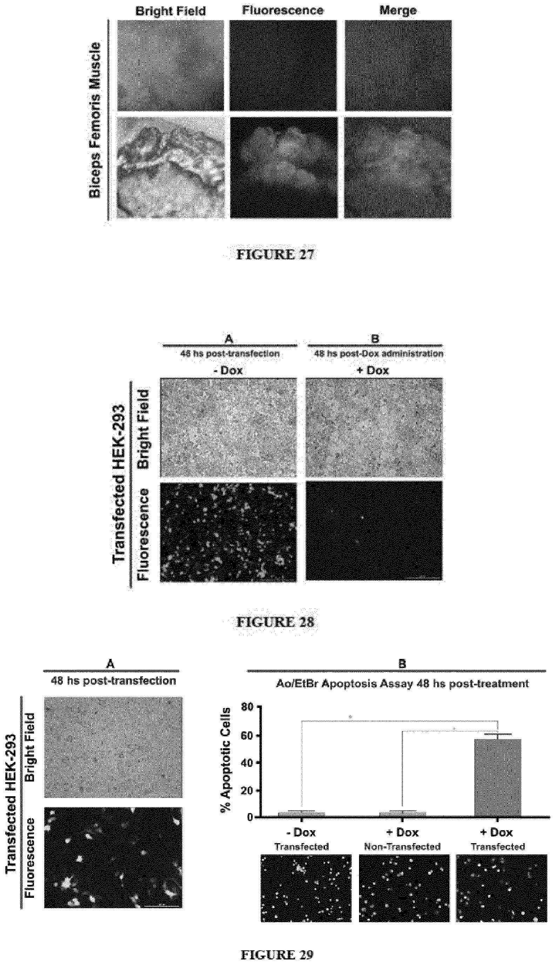

SYTE_{L}-EGFP *in vivo* transfection leads to the expression of the recombinant protein EGFP as can be visualized in (Figure 27).

### Linear DNA Replication and Maintenance in Mammalian Cells

Eukaryotic cell replication follows a molecular schedule known as the cell cycle, which is controlled by a set of replication proteins such as cyclins and cyclin-dependent kinases, among others. Besides the expression of said replication proteins, the replication of DNA molecules in the cell requires a sequence within the DNA molecule that serves as an origin of replication, where the process starts by the formation of a replication bubble.

A replication competent linear DNA molecule was designed, synthesized and manufactured using the method described in this patent. The replication competent linear DNA was engineered to contain at least one origin of replication that is able to replicate the molecules inside mammalian cells. In a preferred embodiment of this patent, the origin of replication is selected from the group consisting of, but not limited to the SV40 origin of replication. This genetic element orchestrates the replication of the DNA molecule where the SV40 origin is located, provided that it is located in the vicinity of an appropriate S/MAR sequence, in a fashion that shows complete synchronization with the host cell cycle. This allows the linear DNA molecule to be stably maintained in the progeny of the originally transformed cell.

To demonstrate the replication of the linear DNA molecule inside mammalian cells, a set of experiments were performed. First, 1 µg of Linear DNA encoding the reporter protein EGFP (denoted as SYTE_{L}-EGFP; Figure 18) formulated with Lipofectamine was used to transfect a low-confluency HEK-293 monolayer culture. HEK-293 (human embryonic kidney) cells used for this experiment were cultured in Dulbecco's Modified Eagle Medium (DMEM) with 10% FBS at 37° in 5% v/v CO2 and in a humidified atmosphere. All experiments were performed with cells in passage 3. The cells then were subjected to incubation for four weeks, with replating every three days, and then analyzed using fluorescence microscopy to demonstrate that the daughter cells were capable of expressing EGFP after several cell-replication cycles, as depicted in Figure 26.

### Safety Switch Assays

The safety switch assay was performed in cell lines to see if the expression of the recombinant protein can be stopped and the molecule can be self-eliminated in a safe way. For this assay, a proteasome-tagged EGFP-encoding linear DNA molecule was used. The expression of proteasome-tagged EGFP proteins is preferred over the classical EGFP protein because the former one can be recycled in a rapid fashion, so that if the safety switch works, the fluorescence will rapidly fade in the cells as can be visualized by fluorescence microscopy.

For the assay, 1 µg of SYTE_{L}-TgEGFP was complexed with Lipofectamine and administered to HEK-293 cells according to the manufacturer's instructions. After 48hs, transfected cells were visualized under fluorescence microscopy to validate the correct expression of EGFP (Figure 28a). Then, the safety switch was induced using doxycycline and the EGFP was measured after 24 hs, confirming depletion of the fluorescence signal in the totality of the treated cells (Figure 28b).

In an alternative approach, a second SYTE_{L}-DNA molecule expressing CFP (Cerulean Fluorescent Protein) was used. In this case, a conditional Bax expressing cassette was built into the linear DNA molecule. When doxycycline is administered externally, the expression of the pro-apoptotic protein Bax is induced, triggering the apoptotic cascade, leading to the death of the transfected cell.

For this second assay, 1 µg of SYTE_{L}-CFP (Bax-armed) was complexed with Lipofectamine and administered to HEK-293 (human embryonic kidney) cells. This cell line was cultured in Dulbecco's Modified Eagle Medium (DMEM) with 10% FBS at 37° in 5% v/v CO2 and in a humidified atmosphere. All experiments were performed with cells in passage 3 cells according to the manufacturer's instructions. After 48hs, transfected cells were visualized under fluorescence microscopy to validate the correct expression of CFP (Figure 29a). Then, the safety switch was induced using doxycycline and cells were stained with an acridine orange/ethidium bromide mix. This staining protocol allows the visualization of green nuclei when cells are viable, and red nuclei when cells are apoptotic. Apoptotic cells were visualized after 48hs of doxycycline administration, confirming the killing of transfected cells when the safety switch is activated (Figure 29b).

### TERMINOLOGY

As used herein, the article "a", "an ", and "the'" means "at least one", unless the context in which the article is used clearly indicates otherwise. The term "nucleic acid" as used herein means a polymer composed of nucleotides, e.g., deoxyribonucleotides or ribonucleotides.

The terms "ribonucleic acid", "RNA" an "polyribonucleotide" as used herein mean a polymer composed of ribonucleotides.

The terms "circular polyribonucleotide", "circRNA" and "circular RNA" are used interchangeably throughout this patent.

The terms "deoxyribonucleic acid", "DNA" an "polydeoxyribonucleotide" as used herein mean a polymer composed of deoxyribonucleotides.

The term "recombinase", as used herein, refers to an enzymatic protein able to catalyze DNA exchange at specific target sites.

The term "circular covalently closed DNA", "circular covalently closed plasmid DNA", "pDNA" or "plasmid DNA" are used interchangeably throughout this patent.

The term "hairpin loop ended self-complementary double stranded covalently closed linear polyribonucleotide", "Linear DNA", "Loop-Ended Linear DNA" or "L-DNA" are used interchangeably throughout this patent.

The term "oligonucleotide" denotes single or double stranded nucleotide multimers that goes from about 2 to up to about 200 nucleotides in length. Suitable oligonucleotides may be prepared by a chemical method such as the phosphoramidite method or an enzymatic method.

The term "polynucleotide" refers to a single or double stranded polymer composed of nucleotide monomers.

The term "polypeptide" refers to a compound made up of a single chain of D-configured or L- configured amino acids joined by peptide bonds.

The term "complementary" refers to an interacting surface of a molecule and its target. Thus, the probe and the target can be described as complementary.

The term "anneal" refers to the process by which a single-stranded nucleic sequence pairs by hydrogen bonds to a complementary sequence, thus forming a double stranded nucleic acid sequence. This includes the reformation of complementary strands that were thermally denatured, known as renaturation.

The term "target" refers to a molecule with an affinity for a given probe. Targets may be natural or man-made molecules.

The term "promoter", "regulatory element" or "regulatory sequence" refers to a region or genetic sequence located upstream or downstream from the start of the transcription site and which are involved in the recognition and binding of RNA polymerase and other proteins to initiate the transcription process. Selected promoters may include, but are not limited to, bacterial promoters, viral promoters, mammalian promoters, yeast promoters or combinations thereof.

The term "regulatory element" is intended to include promoters, enhancers, internal ribosome entry sites (IRES) and other expression control elements such as, but not limited to, transcription termination signals, polyadenylation signals and poly-U sequences. Regulatory elements may include those that direct constitutive expression of a nucleic acid sequence in a wide variety of cell types, tissues or organs and those that direct expression of a nucleic acid sequence only in certain cell types, tissues or organs, such as, but not limited to, cell specific regulatory sequences, tissue specific regulatory sequences and organ specific regulatory sequences.

A cell specific promoter may direct the expression of a nucleic acid sequence in a desired cell type of interest such as, but not limited to, lymphocytes, muscle cells, keratinocytes and fibroblasts. A tissue specific promoter may direct the expression of a nucleic acid sequence in a desired tissue of interest such as, but not limited to, muscle, neuron, bone, skin and blood. An organ specific promoter may direct the expression of a nucleic acid sequence in a desired organ such as, but not limited to, liver, brain, heart, lungs and kidney.

Regulatory elements may also direct expression in a temporal dependent manner such as, but not limited to, cell cycle and developmental stage.

Also encompassed by the term "regulatory element" are enhancer elements that include, but are not limited to, WPRE enhancer, CMV enhancer and HTLV-LTR enhancer.

The term "recombinant" refers to a manipulated nucleic acid. In reference to a protein ("recombinant protein"), a protein encoded by a recombinant nucleic acid.

The term "transcriptional unit" refers to a nucleic acid construct, which when introduced into a cell, results in transcription and / or translation to an RNA or polypeptide.

The term "codon optimized" refers to the alteration of codons in the open reading frame of nucleic acid sequences to reflect the typical codon usage of a selected organism without altering the polypeptide encoded by the sequence. The optimization includes replacing at least one, or more than one, or a significant number of codons with one or more codons that are more frequently used in the genes of that selected organism.

A nucleic acid sequence is "operably linked" when it is placed into a functional relation with another nucleic acid sequence.

As used herein a "host" means an individual, and can include domesticated animals such as, but not limited to, cats and dogs; livestock such as but not limited to, cattle, horses, pigs, sheep and goats; laboratory animals such as, but not limited to, mouse, rabbit, rat and guinea pig; mammalians such as, but not limited to, humans, non-human primates and primates; other animals such as, but not limited to, rodents, birds, reptiles, amphibians and fish.

### SEQUENCES

**SEQ ID NO 1**
   LENGTH: 5.951
   TYPE: DNA
   ORGANISM: Artificial
   FEATURE: Parental Plasmid
   OTHER INFORMATION: Parental Plasmid / pDNAv_A
   SEQUENCE: 1 
GGATCCACTAGTATCTCGACGCATTCATTACCCTGTTATCCCTAGATACAAGATACATTACCCTGTTATCCCAGAT
GACATACCCTGTTATCCCTAGATGACATTACCCTGTTATCCCAGATGACATTACCCTGTTATCCCTAGATACATTA
CCCTGTTATCCCAGATGACATACCCTGTTATCCCTAGATGACATTACCCTGTTATCCCAGATGACATTACCCTGTT
ATCCCTAGATACATTACCCTGTTATCCCAGATGACATACCCTGTTATCCCTAGATGACATTACCCTGTTATCCCAG
ATGACATTACCCTGTTATCCCTAGATACATTACCCTGTTATCCCAGATGACATACCCTGTTATCCCTAGATGACAT
TACCCTGTTATCCCAGATGACATTACCCTGTTATCCCTAGATACATTACCCTGTTATCCCAGATGACATACCCTGT
TATCCCTAGATGACATTACCCTGTTATCCCAGATGACATTACCCTGTTATCCCTAGAAGATCCTTTGATGCGGCC
GCATAACTTCGTATAGCATACATTATACGAAGTTATCTCGAGGTCAGTGTCAAGTCAGTGACTACTGTACTGAACT
GTACGATATCGGTACCTAGATGTCAACTTCAGCACACAATTGCCATTATACGCGCGTATAATGGACTATTGTGTG
CTGATACGATCGCAGACCATGGTCCTTCGATTGGTGTGGAAAGTCCCCAGGCTCCCCAGCAGGCAGAAGTATG
CAAAGCATGCATCTCAATTAGTCAGCAACCAGCATGCATGCATTGGTGTGGAAAGTCCCCAGGCTCCCCAGCAG
GCAGAAGTATGCAAAGCATGCATCTCAATTAGTCAGCAACCAGCATGCATGCATCTATCCATAGCTGATTGGTCT
AAAATGAGATACATCAACGCTCCTCCATGTTTTTTGTTTTCTTTTTAAATGAAAAACTTTATTTTTTAAGAGGAGTTT
CAGGTTCATAGCAAAATTGAGAGGAAGGTACATTCAAGCTGAGGAAGTTTTCCTCTATTCCTAGTTTACTGAGAG
ATTGCATCATGAATGGGTGTTAAATTTTGTCAAATGCTTTTTCTGTGTCTATCAATATGACCATGTGATTTTCTTCT
TTAACCTGTTGATGGGACAAATTACGTTAATTGATTTTCAAACGTTGAACCACCCTTACATATCTGGAATAAATTCT
ACTTGGTTGTGGTGTATATTTTTTGATACATTCTTGGATTCTTTTTGCTAATATTTTGTTGAAAATGTTTGTATCTTT
GTTCATGAGAGATATTGGTCTGTTGTTTTCTTTTCTTGTAATGTCATTTTCTAGTTCCGGTATTAAGGTAATGCTGG
CCTAGTTGAATGATTTAGGAAGTATTCCCTCTGCTTCTGTCTTCTGAAAGAGATTGTAGAAAGTTGATACAATTTTT
TTTTCTTTAAATATCTTGATAGGTGGCTATGGCAGGGCTTGCCGCCCCGACGTTGGCTGCGAGCCCTGGGCCTT
CACCCGAACTTGGGGGTTGGGGTGGGGAAAAGGAAGAAACGCGGGCGTATTGGTCCCAATGGGGTCTCGGTG
GGGTATCGACAGAGTGCCAGCCCTGGGACCGAACCCCGCGTTTATGAACAAACGACCCAACACCCGTGCGTTT
TATTCTGTCTTTTTATTGCCGTCATAGCGCGGGTTCCTTCCGGTATTGTCTCCTTCCGTGTTTCAGTTAGCCTCCC
CCATCTCCCGCTGCAGGAATTCCGCGGAACCCCTATTTGTTTATTTTTCTAAATACATTCAAATATGTATCCGCTC
ATGAGACAATAACCCTGATAAATGCTTCAATAATATTGAAAAAGGAAGAGTATGCGTTTGAAGGTGATGATGGAT
GTCAATAAAAAAACCAAGATTCGTCATCGTAATGAGCTTAATCATACCTTAGCTCAGCTGCCTTTACCAGCAAAAC
GCGTGATGTATATGGCCCTTGCCCCCATTGACAGTAAGGAGCCGTTAGAACGCGGTCGTGTATTTAAGATTCGT
GCCGAAGACTTAGCGGCGCTTGCAAAAATTACTCCTAGTTTGGCCTACCGTCAACTGAAGGAGGGAGGCAAATT
GCTGGGCGCCTCGAAGATCAGCTTGCGTGGAGACGATATCATCGCCCTTGCTAAGGAGTTGAACTTGCCATTCA
CTGCGAAAAATTCCCCGGAGGAATTAGACCTGAATATCATTGAGTGGATCGCTTACAGCCCTGACGAGGGCTAC
TTGAGTCTGAAGTTCACGCGTACTATTGAGCCTTATATTTCTAGTCTTATCGGGAAAAAGAACAAATTTACCACGC
AATTATTGACTGCATCCTTGCGCCTGTCGTCACAGTATTCTTCATCACTGTATCAACTGATCCGCAAACATTATAG
TAATTTCAAAAAAAAAAACTATTTTATCATTAGCGTGGACGAGCTTAAAGAAGAACTGATTGCCTATACTTTTGATA
AAGACGGGAACATCGAATATAAATACCCGGACTTCCCAATTTTTAAACGCGATGTTTTAAACAAAGCGATTGCAG
AAATCAAAAAGAAAACTGAGATTTCGTTTGTTGGTTTTACAGTACACGAGAAGGAGGGGCGCAAAATTTCCAAAT
TAAAATTTGAATTTGTCGTTGATGAGGACGAGTTTAGTGGAGATAAAGACGACGAGGCTTTCTTTATGAACTTGTC
GGAGGCCGATGCGGCATTTTTAAAAGTGTTCGATGAAACCGTTCCGCCCAAAAAAGCCAAGGGCTAAAAGCCCG
 
AAAGGAAGCTGAGTTGGCTGCTGCCACCGCTGAGCAATAACTAGCATAACCCCTTGGGGCCTCTAAACGGGTCT
TGAGGGGTTTTTTGGCTAGCTCTAGAGTCGACGATCATAATCAGCCATACCACATTTGTAGAGGTTTTACTTGCTT
TAAAAAACCTCCCACACCTCCCCCTGAACCTGAAACATAAAATGAATGCAATTGTTGTTGTTAACTTGTTTATTGC
AGCTTATAATGGTTACAAATAAAGCAATAGCATCACAAATTTCACAAATAAAGCATTTTTTTCACTGCATTCTAGTT
GTGGTTTGTCCAAACTCATCAATGTATCTTATCATGTCTGGATCCTATCCATAGCTGATTGGTCTAAAATGAGATA
CATCAACGCTCCTCCATGTTTTTTGTTTTCTTTTTAAATGAAAAACTTTATTTTTTAAGAGGAGTTTCAGGTTCATAG
CAAAATTGAGAGGAAGGTACATTCAAGCTGAGGAAGTTTTCCTCTATTCCTAGTTTACTGAGAGATTGCATCATGA
ATGGGTGTTAAATTTTGTCAAATGCTTTTTCTGTGTCTATCAATATGACCATGTGATTTTCTTCTTTAACCTGTTGA
TGGGACAAATTACGTTAATTGATTTTCAAACGTTGAACCACCCTTACATATCTGGAATAAATTCTACTTGGTTGTG
GTGTATATTTTTTGATACATTCTTGGATTCTTTTTGCTAATATTTTGTTGAAAATGTTTGTATCTTTGTTCATGAGAG
ATATTGGTCTGTTGTTTTCTTTTCTTGTAATGTCATTTTCTAGTTCCGGTATTAAGGTAATGCTGGCCTAGTTGAAT
GATTTAGGAAGTATTCCCTCTGCTTCTGTCTTCTGAAAGAGATTGTAGAAAGTTGATACAATTTTTTTTTCTTTAAA
TATCTTGATAGGCACGCATGCATTGGTGTGGAAAGTCCCCAGGCTCCCCAGCAGGCAGAAGTATGCAAAGCATG
CATCTCAATTAGTCAGCAACCAGCATGCATGCATTGGTGTGGAAAGTCCCCAGGCTCCCCAGCAGGCAGAAGTA
TGCAAAGCATGCATCTCAATTAGTCAGCAACCAGCATGCATGCATGCGAAGTACGAGCTCCGATCTCCAATTTAT
CAGCACACAATAGTCCATTATACGCGCGTATAATGGCAATTGTGTGCTGACTACGTACGTACACTACTACGAACT
CAGAGTCAGTAGATCTGATATCATCGTCTACACTTGCCAGCGCCCTAGCGCCCGCTCCTTTCGCTTTCTTCCCTT
CCTTTCTCGCCACGTTCGCCGGCTTTCCCCGTCAAGCTCTAAATCGGGGGCTCCCTTTAGGGTTCCGATTTAGT
GCTTTACGGCACCTCGACCCCAAAAAACTTGATTTGGGTGATGGTTCACGTAGTGGGCCATCGCCCTGATAGAC
GGTTTTTCGCCCTTTGACGTTGGAGTCCACGTTCTTTAATAGTGGACTCTTGTTCCAAACTGGAACAACACTCAA
CCCTATCTCGGGCTATTCTTTTGATTTATAAGGGATTTTGCCGATTTCGGGAAGATCCTTTGATGGTGCCAGGGC
GTGCCCTTGGGCTCCCCGGGCGCGCTTTTCTACGGGGTTGACGCTCAGTCCTCGCGGAACCCCTATTTGTTTAT
TTTTCTAAATACATTCAAATATGTATCCGCTCATGAGACAATAACCCTGATAAATGCTTCAATAATATTGAAAAAGG
AAGAGTATGAGTATTCAACATTTCCGTGTCGCCCTTATTCCCTTTTTTGCGGCATTTTGCCTTCCTGTTTTTGCTC
ACCCAGAAACGCTGGTGAAAGTAAAAGATGCTGAAGATCAGTTGGGTGCACGAGTGGGTTACATCGAACTGGAT
CTCAACAGCGGTAAGATCCTTGAGAGTTTTCGCCCCGAAGAACGTTTTCCAATGATGAGCACTTTTAAAGTTCTG
CTATGTGGCGCGGTATTATCCCGTATTGACGCCGGGCAAGAGCAACTCGGTCGCCGCATACACTATTCTCAGAA
TGACTTGGTTGAGTACTCACCAGTCACAGAAAAGCATCTTACGGATGGCATGACAGTAAGAGAATTATGCAGTGC
TGCCATAACCATGAGTGATAACACTGCGGCCAACTTACTTCTGACAACGATCGGAGGACCGAAGGAGCTAACCG
CTTTTTTGCACAACATGGGGGATCATGTAACTCGCCTTGATCGTTGGGAACCGGAGCTGAATGAAGCCATACCA
AACGACGAGCGTGACACCACGATGCCTGTAGCAATGGCAACAACGTTGCGCAAACTATTAACTGGCGAACTACT
TACTCTAGCTTCCCGGCAACAATTAATAGACTGGATGGAGGCGGATAAAGTTGCAGGACCACTTCTGCGCTCGG
CCCTTCCGGCTGGCTGGTTTATTGCTGATAAATCTGGAGCCGGTGAGCGTGGGTCTCGCGGTATCATTGCAGCA
CTGGGGCCAGATGGTAAGCCCTCCCGTATCGTAGTTATCTACACGACGGGGAGTCAGGCAACTATGGATGAAC
GAAATAGACAGATCGCTGAGATAGGTGCCTCACTGATTAAGCATTGGTAACTGTCAGACCAAGTTTACTCATATA
TACTTTAGATTGATTTAAAACTTCATTTTTAATTTAAAAGGATCTAGGTGAAGATCCTTTTTGATAATCTCATGACCA
AAATCCCTTAACGTGAGTTTTCGTTCCACTGAGCGTCAGACCCCGTAGAAAAGATCAAAGGATCTTCCGGACCGA
AAGAGTTTGTAGAAACGCATGTCAGCCGTTAAGTGTTCCTGTGTCACTCAAAATTGCTTTGAGAGGCTCTAAGGG
CTTCTCAGTGCGTTACATCCCTGGCTTGTTGTCCACAACCGTTAAACCTTAAAAGCTTTAAAAGCCTTATATATTC
TTTTTTTTCTTATAAAACTTAAAACCTTAGAGGCTATTTAAGTTGCTGATTTATATTAATTTTATTGTTCAAACATGA
GAGCTTAGTACGTGAAACATGAGAGCTTAGTACGTTAGCCATGAGAGCTTAGTACGTTAGCCATGAGGGTTTAGT
TCGTTAAACATGAGAGCTTAGTACGTTAAACTTGAGAGCTTAGTACGTGAAACATGAGAGCTTAGTACGTAC
**SEQ ID NO 2**
   LENGTH: 73
   TYPE: DNA
   ORGANISM: Artificial
   FEATURE: DNA Nuclear Localization Sequence
   OTHER INFORMATION: SV40E
   SEQUENCE: 2
      TGGTGTGGAAAGTCCCCAGGCTCCCCAGCAGGCAGAAGTATGCAAAGCATG CATCTCAATTAGTCAGCAACCA
**SEQ ID NO 3**
   LENGTH: 42
   TYPE: DNA
   ORGANISM: Artificial
   FEATURE: DNA Nuclear Localization Sequence
   OTHER INFORMATION: NF-kB importin
   SEQUENCE: 3
      TGGGGACTTTCCGCTGGGGACTTTCCGCTGGGGACTTTCCGC
**SEQ ID NO 4**
   LENGTH: 2.268
   TYPE: DNA
   ORGANISM: Artificial
   FEATURE: DNA Scaffold Matrix Attachment Region
   OTHER INFORMATION: β-Interferon Scaffold Matrix Attachment Region
   SEQUENCE: 4 
GGGACGTAGCAGAAGGACGTCCCGCGCAGAATCCAGGTGGCAACACAGGC
GAGCAGCCAAGGAAAGGACGATGATTTCCCCGACAACACCACGGAATTGTC
AGTGCCCAACAGCCGAGCCCCTGTCCAGCAGCGGGCAAGGCAGGCGGCGAT
GAGTTCCGCCGTGGCAATAGGGAGGGGGAAAGCGAAAGTCCCGGAAAGGA
GCTGACAGGTGGTGGCAATGCCCCAACCAGTGGGGGTTGCGTCAGCAAACA
CAGTGCACACCACGCCACGTTGCCTGACAACGGGCCACAACTCCTCATAAA
GAGACAGCAACCAGGATTTATACAAGGAGGAGAAAATGAAAGCCATACGG
GAAGCAATAGCATGATACAAAGGCATTAAAGCAGCGTATCCACATAGCGTA
AAAGGAGCAACATAGTTAAGAATACCAGTCAATCTTTCACAAATTTTGTAAT
CCAGAGGTTGATCTAGCAGCAAGGTCGCCACGCACAAGATCAATATTAACA
ATCAGTCATCTCTCTTTAGCAATAAAAAGGTGAAAAATTACATTTTAAAAAT
GACACCATAGACGATGTATGAAAATAATCTACTTGGAAATAAATCTAGGCA
AAGAAGTGCAAGACTGTTACCCAGAAAACTTACAAATTGTAAATGAGAGGT
TAGTGAAGATTTAAATGAATGAAGATCTAAATAAACTTATAAATTGTGAGA
GAAATTAATGAATGTCTAAGTTAATGCAGAAACGGAGAGACATACTATATT
CATGAACTAAAAGACTTAATATTGTGAAGGTATACTTTCTTTCCACATAAAT
TTGTAGTCAATATGTTCACCCCAAAAAAGCTGTTTGTTAACTTGCCAACCTC
ATTCTAAAATGTATATAGAAGCCCAAAAGACAATAACAAAAATATTCTTGT
AGAACAAAATGGGAAAGAATGTTCCACTAAATATCAAGATTTAGAGCAAAG
CATGAGATGTGTGGGGATAGACAGTGAGGCTGATAAAATAGAGTAGAGCTC
AGAAACAGACCCATTGATATATGTAAGTGACCTATGAAAAAAATATGGCAT
TTTACAATGGGAAAATGATGATCTTTTTCTTTTTTAGAAAAACAGGGAAATA
TATTTATATGTAAAAAATAAAAGGGAACCCATATGTCATACCATACACACA
AAAAAATTCCAGTGAATTATAAGTCTAAATGGAGAAGGCAAAACTTTAAAT
 
CTTTTAGAAAATAATATAGAAGCATGCCATCATGACTTCAGTGTAGAGAAA
AATTTCTTATGACTCAAAGTCCTAACCACAAAGAAAAGATTGTTAATTAGAT
TGCATGAATATTAAGACTTATTTTTAAAATTAAAAAACCATTAAGAAAAGTC
AGGCCATAGAATGACAGAAAATATTTGCAACACCCCAGTAAAGAGAATTGT
AATATGCAGATTATAAAAAGAAGTCTTACAAATCAGTAAAAAATAAAACTA
GACAAAAATTTGAACAGATGAAAGAGAAACTCTAAATAATCATTACACATG
AGAAACTCAATCTCAGAAATCAGAGAACTATCATTGCATATACACTAAATT
AGAGAAATATTAAAAGGCTAAGTAACATCTGTGGCAATATTGATGGTATAT
AACCTTGATATGATGTGATGAGAACAGTACTTTACCCCATGGGCTTCCTCCC
CAAACCCTTACCCCAGTATAAATCATGACAAATATACTTTAAAAACCATTAC
CCTATATCTAACCAGTACTCCTCAAAACTGTCAAGGTCATCAAAAATAAGA
AAAGTCTGAGGAACTGTCAAAACTAAGAGGAACCCAAGGAGACATGAGAA
TTATATGTAATGTGGCATTCTGAATGAGATCCCAGAACAGAAAAAGAACAG
TAGCTAAAAAACTAATGAAATATAAATAAAGTTTGAACTTTAGTTTTTTTTA
AAAAAGAGTAGCATTAACACGGCAAAGCCATTTTCATATTTTTCTTGAACAT
TAAGTACAAGTCTATAATTAAAAATTTTTTAAATGTAGTCTGGAACATTGCC
AGAAACAGAAGTACAACAGCTATCTGTGCTGTCGCCTAACTATCCATAGCT
GATTGGTCTAAAATGAGATACATCAACGCTCCTCCATGTTTTTTGTTTTCTTT
TTAAATGAAAAACTTTATTTTTTAAGAGGAGTTTCAGGTTCATAGCAAAATT
GAGAGGAAGGTACATTCAAGCTGAGGAAGTTTTCCTCTATTCCTAGTTTACT
GAGAGATTGCATCATGAATGGGTGTTAAATTTTGTCAAATGCTTTTTCTGTG
TCTATCAATATGACCATGTGATTTTCTTCTTTAACCTGTTGATGGGACAAATT
ACGTTAATTGATTTTCAAACGTTGAACCACCCTTACATATCTGGAATAAATT
CTACTTGGTTGTGGTGTATATTTTTTGATACATTCTTGGATTCTTTTTGCTAAT
ATTTTGTTGAAAATGTTTGTATCTTTGTTCATGAGAGATATTGGTCTGTTGTT
TTCTTTTCTTGTAATGTCATTTTCTAGTTCCGGTATTAAGGTAATGCTGGCCT
AGTTGAATGATTTAGGAAGTATTCCCTCTGCTTCTGTCTTCTGAAAGAGATT
GTAGAAAGTTGATACAATTTTTTTTTCTTTAAATATTTGATAG
**SEQ ID NO 5**
   LENGTH: 6
   TYPE: DNA
   ORGANISM: Artificial
   FEATURE: Restriction Site
   OTHER INFORMATION: Sail
   SEQUENCE: 5
      GTCGAC
**SEQ ID NO 6**
   LENGTH: 6
   TYPE: DNA
   ORGANISM: Artificial
   FEATURE: Restriction Site
   OTHER INFORMATION: Pstl
   SEQUENCE: 6
      CTG CAG
**SEQ ID NO 7**
   LENGTH: 6
   TYPE: DNA
   ORGANISM: Artificial
   FEATURE: Restriction Site
   OTHER INFORMATION: Nsel
   SEQUENCE: 7
      GCTAGC
**SEQ ID NO 8**
   LENGTH: 6
   TYPE: DNA
   ORGANISM: Artificial
   FEATURE: Restriction Site
   OTHER INFORMATION: EcoRI
   SEQUENCE: 8
      GAATTC
**SEQ ID NO 9**
   LENGTH: 8
   TYPE: DNA
   ORGANISM: Artificial
   FEATURE: Restriction Site
   OTHER INFORMATION: Sdal
   SEQUENCE: 9
      CCTGCAGG
**SEQ ID NO 10**
   LENGTH: 6
   TYPE: DNA
   ORGANISM: Artificial
   FEATURE: Restriction Site
   OTHER INFORMATION: Xhol
   SEQUENCE: 10
      CTCGAG
**SEQ ID NO 11**
   LENGTH: 918
   TYPE: DNA
   ORGANISM: Artificial
   FEATURE: DNA Replication Protein
   OTHER INFORMATION: PI Protein
   SEQUENCE: 11 
ATGCGCCTGAAAGTGATGATGGATGTGAACAAAAAAACCAAAATTCGCCAT
CGCAACGAACTGAACCATACCCTGGCGCAGCTGCCGCTGCCGGCGAAACGC
 
GTGATGTATATGGCGCTGGCGCCGATTGATAGCAAAGAACCGCTGGAACGC
GGCCGCGTGTTTAAAATTCGCGCGGAAGATCTGGCGGCGCTGGCGAAAATT
ACCCCGAGCCTGGCGTATCGCCAGCTGAAAGAAGGCGGCAAACTGCTGGGC
GCGAGCAAAATTAGCCTGCGCGGCGATGATATTATTGCGCTGGCGAAAGAA
CTGAACCTGCCGTTTACCGCGAAAAACAGCCCGGAAGAACTGGATCTGAAC
ATTATTGAATGGATTGCGTATAGCCCGGATGAAGGCTATCTGAGCCTGAAAT
TTACCCGCACCATTGAACCGTATATTAGCAGCCTGATTGGCAAAAAAAACA
AATTTACCACCCAGCTGCTGACCGCGAGCCTGCGCCTGAGCAGCCAGTATA
GCAGCAGCCTGTATCAGCTGATTCGCAAACATTATAGCAACTTTAAAAAAA
AAAACTATTTTATTATTAGCGTGGATGAACTGAAAGAAGAACTGATTGCGT
ATACCTTTGATAAAGATGGCAACATTGAATATAAATATCCGGATTTTCCGAT
TTTTAAACGCGATGTGCTGAACAAAGCGATTGCGGAAATTAAAAAAAAAAC
CGAAATTAGCTTTGTGGGCTTTACCGTGCATGAAAAAGAAGGCCGCAAAAT
TAGCAAACTGAAATTTGAATTTGTGGTGGATGAAGATGAATTTAGCGGCGA
TAAAGATGATGAAGCGTTTTTTATGAACCTGAGCGAAGCGGATGCGGCGTT
TCTGAAAGTGTTTGATGAAACCGTGCCGCCGAAAAAAGCGAAAGGCTAA
**SEQ ID NO 12**
   LENGTH: 179
   TYPE: DNA
   ORGANISM: Artificial
   FEATURE: Phage Origin of Replication
   OTHER INFORMATION: M13 Origin of Replication (+)
   SEQUENCE: 12 
TCTAAATCGGGGGCTCCCTTTAGGGTTCCGATTTAGTGCTTTACGGCACCTCGACCCCAAAAAACTTGATTTGGG
TGATGGTTCACGTAGTGGGCCATCGCCCTGATAGACGGTTTTTCGCCCTTTGACGTTGGAGTCCACGTTCTTTAA
TAGTGGACTCTTGTTCCAAACTGGAACAA
**SEQ ID NO 13**
   LENGTH: 14.657
   TYPE: DNA
   ORGANISM: Artificial
   FEATURE: Helper Plasmid
   OTHER INFORMATION: pSYTE_{L}-Helper
   SEQUENCE: 13 
TCGCGCGTTTCGGTGATGACGGTGAAAACCTCTGACACATGCAGCTCCCGGAGACGGTCACAGCTTGTCTGTAA
GCGGATGCCGGGAGCAGACAAGCCCGTCAGGGCGCGTCAGCGGGTGTTGGCGGGTGTCGGGGCTGGCTTAA
CTATGCGGCATCAGAGCAGATTGTACTGAGAGTGCACCATATGCGGTGTGAAATACCGCACAGATGCGTAAGGA
GAAAATACCGCATCAGGCGCCATTCGCCATTCAGGCTGCGCAACTGTTGGGAAGGGCGATCGGTGCGGGCCTC
TTCGCTATTACGCCAGCTGGCGAAAGGGGGATGTGCTGCAAGGCGATTAAGTTGGGTAACGCCAGGGTTTTCC
CAGTCACGACGTTGTAAAACGACGGCCAGTGAATTGGAGATCGGTACTTCGCGAATGCGTCGAGATGTAGTGGC
CAGGACCCAACGCTGCCCGAAATTGGCCAGGACCCAACGCTGCCCGAAATTCCGACACCATCGAATGGTGCAA
AACCTTTCGCGGTATGGCATGATAGCGCCCGGAAGAGAGTCAATTCAGGGTGGTGAATGTGAAACCAGTAACGT
TATACGATGTCGCAGAGTATGCCGGTGTCTCTTATCAGACCGTTTCCCGCGTGGTGAACCAGGCCAGCCACGTT
TCTGCGAAAACGCGGGAAAAAGTGGAAGCGGCGATGGCGGAGCTGAATTACATTCCCAACCGCGTGGCACAAC
AACTGGCGGGCAAACAGTCGTTGCTGATTGGCGTTGCCACCTCCAGTCTGGCCCTGCACGCGCCGTCGCAAAT
 
TGTCGCGGCGATTAAATCTCGCGCCGATCAACTGGGTGCCAGCGTGGTGGTGTCGATGGTAGAACGAAGCGGC
GTCGAAGCCTGTAAAGCGGCGGTGCACAATCTTCTCGCGCAACGCGTCAGTGGGCTGATCATTAACTATCCGCT
GGATGACCAGGATGCCATTGCTGTGGAAGCTGCCTGCACTAATGTTCCGGCGTTATTTCTTGATGTCTCTGACCA
GACACCCATCAACAGTATTATTTTCTCCCATGAAGACGGTACGCGACTGGGCGTGGAGCATCTGGTCGCATTGG
GTCACCAGCAAATCGCGCTGTTAGCGGGCCCATTAAGTTCTGTCTCGGCGCGTCTGCGTCTGGCTGGCTGGCA
TAAATATCTCACTCGCAATCAAATTCAGCCGATAGCGGAACGGGAAGGCGACTGGAGTGCCATGTCCGGTTTTC
AACAAACCATGCAAATGCTGAATGAGGGCATCGTTCCCACTGCGATGCTGGTTGCCAACGATCAGATGGCGCTG
GGCGCAATGCGCGCCATTACCGAGTCCGGGCTGCGCGTTGGTGCGGATATTTCGGTAGTGGGATACGACGATA
CCGAAGACAGCTCATGTTATATCCCGCCGTTAACCACCATCAAACAGGATTTTCGCCTGCTGGGGCAAACCAGC
GTGGACCGCTTGCTGCAACTCTCTCAGGGCCAGGCGGTGAAGGGCAATCAGCTGTTGCCCGTCTCACTGGTGA
AAAGAAAAACCACCCTGGCGCCCAATACGCAAACCGCCTCTCCCCGCGCGTTGGCCGATTCATTAATGCAGCTG
GCACGACAGGTTTCCCGACTGGAAAGCGGGCAGTGAGAATTCAATAATTTTGTTTAACTTTAAGAAGGAGATATA
CATATGGCTGAAGCGCAAAATGATCCCCTGCTGCCGGGATACTCGTTTAATGCCCATCTGGTGGCGGGTTTAAC
GCCGATTGAGGCCAACGGTTATCTCGATTTTTTTATCGACCGACCGCTGGGAATGAAAGGTTATATTCTCAATCT
CACCATTCGCGGTCAGGGGGTGGTGAAAAATCAGGGACGAGAATTTGTTTGCCGACCGGGTGATATTTTGCTGT
TCCCGCCAGGAGAGATTCATCACTACGGTCGTCATCCGGAGGCTCGCGAATGGTATCACCAGTGGGTTTACTTT
CGTCCGCGCGCCTACTGGCATGAATGGCTTAACTGGCCGTCAATATTTGCCAATACGGGGTTCTTTCGCCCGGA
TGAAGCGCACCAGCCGCATTTCAGCGACCTGTTTGGGCAAATCATTAACGCCGGGCAAGGGGAAGGGCGCTAT
TCGGAGCTGCTGGCGATAAATCTGCTTGAGCAATTGTTACTGCGGCGCATGGAAGCGATTAACGAGTCGCTCCA
TCCACCGATGGATAATCGGGTACGCGAGGCTTGTCAGTACATCAGCGATCACCTGGCAGACAGCAATTTTGATA
TCGCCAGCGTCGCACAGCATGTTTGCTTGTCGCCGTCGCGTCTGTCACATCTTTTCCGCCAGCAGTTAGGGATT
AGCGTCTTAAGCTGGCGCGAGGACCAACGTATCAGCCAGGCGAAGCTGCTTTTGAGCACCACCCGGATGCCTA
TCGCCACCGTCGGTCGCAATGTTGGTTTTGACGATCAACTCTATTTCTCGCGGGTATTTAAAAAATGCACCGGGG
CCAGCCCGAGCGAGTTCCGTGCCGGTTGTGAAGAAAAAGTGAATGATGTAGCCGTCAAGTTGTCATAATGTACA
AAGCTTCGCCAGGGTTTTCCCAGTCGAGCTCAATAATTTTGTTTAACTTTAAGAAGGAGATATACATATGAGCACA
AAAAAGAAACCATTAACACAAGAGCAGCTTGAGGACGCACGTCGCCTTAAAGCAATTTATGAAAAAAAGAAAAAT
GAACTTGGCTTATCCCAGGAATCTGTCGCAGACAAGATGGGGATGGGGCAGTCAGGCGTTGGTGCTTTATTTAA
TGGCATCAATGCATTAAATGCTTATAACGCCGCATTGCTTGCAAAAATTCTCAAAGTTAGCGTTGAAGAATTTAGC
CCTTCAATCGCCAGAGAAATCTACGAGATGTATGAAGCGGTTAGTATGCAGCCGTCACTTAGAAGTGAGTATGA
GTACCCTGTTTTTTCTCATGTTCAGGCAGGGATGTTCTCACCTGAGCTTAGAACCTTTACCAAAGGTGATGCGGA
GAGATGGGTAAGCACAACCAAAAAAGCCAGTGATTCTGCATTCTGGCTTGAGGTTGAAGGTAATTCCATGACCG
CACCAACAGGCTCCAAGCCAAGCTTTCCTGACGGAATGTTAATTCTCGTTGACCCTGAGCAGGCTGTTGAGCCA
GGTGATTTCTGCATAGCCAGACTTGGGGGTGATGAGTTTACCTTCAAGAAACTGATCAGGGATAGCGGTCAGGT
GTTTTTACAACCACTAAACCCACAGTACCCAATGATCCCATGCAATGAGAGTTGTTCCGTTGTGGGGAAAGTTAT
CGCTAGTCAGTGGCCTGAAGAGACGTTTGGCTGAGCGCAACGCAATTAATGTAAGTTAGCTCACTCATTAGGCA
CAATTCTCATGTTTGACAGCTTATCATCGACTGCACGGTGCACCAATGCTTCTGGCGTCAGGCAGCCATCGGAA
GCTGTGGTATGGCTGTGCAGGTCGTAAATCACTGCATAATTCGTGTCGCTCAAGGCGCACTCCCGTTCTGGATA
ATGTTTTTTGCGCCGACATCATAACGGTTCTGGCAAATATTCTGAAATGAGCTACGGCCTCAACCTACTACTGGG
CTGCTTCCTAATGCAGGATTTACACTTTATGCTTCCGGCTCGTATGTTGTGTGGAATTGTGAGCGGATAACAATTT
CACACATACTAGAGTCACACAGGAAAGTACTAGGTGGACACGTACGCGGGTGCTTACGACCGTCAGTCGCGCG
AGCGCGAGAATTCGAGCGCAGCAAGCCCAGCGACACAGCGTAGCGCCAACGAAGACAAGGCGGCCGACCTTC
AGCGCGAAGTCGAGCGCGACGGGGGCCGGTTCAGGTTCGTCGGGCATTTCAGCGAAGCGCCGGGCACGTCG
GCGTTCGGGACGGCGGAGCGCCCGGAGTTCGAACGCATCCTGAACGAATGCCGCGCCGGGCGGCTCAACATG
ATCATTGTCTATGACGTGTCGCGCTTCTCGCGCCTGAAGGTCATGGACGCGATTCCGATTGTCTCGGAATTGCT
CGCCCTGGGCGTGACGATTGTTTCCACTCAGGAAGGCGTCTTCCGGCAGGGAAACGTCATGGACCTGATTCAC
CTGATTATGCGGCTCGACGCGTCGCACAAAGAATCTTCGCTGAAGTCGGCGAAGATTCTCGACACGAAGAACCT
 
TCAGCGCGAATTGGGCGGGTACGTCGGCGGGAAGGCGCCTTACGGCTTCGAGCTTGTTTCGGAGACGAAGGA
GATCACGCGCAACGGCCGAATGGTCAATGTCGTCATCAACAAGCTTGCGCACTCGACCACTCCCCTTACCGGAC
CCTTCGAGTTCGAGCCCGACGTAATCCGGTGGTGGTGGCGTGAGATCAAGACGCACAAACACCTTCCCTTCAAG
CCGGGCAGTCAAGCCGCCATTCACCCGGGCAGCATCACGGGGCTTTGTAAGCGCATGGACGCTGACGCCGTG
CCGACCCGGGGCGAGACGATTGGGAAGAAGACCGCTTCAAGCGCCTGGGACCCGGCAACCGTTATGCGAATC
CTTCGGGACCCGCGTATTGCGGGCTTCGCCGCTGAGGTGATCTACAAGAAGAAGCCGGACGGCACGCCGACC
ACGAAGATTGAGGGTTACCGCATTCAGCGCGACCCGATCACGCTCCGGCCGGTCGAGCTTGATTGCGGACCGA
TCATCGAGCCCGCTGAGTGGTATGAGCTTCAGGCGTGGTTGGACGGCAGGGGGCGCGGCAAGGGGCTTTCCC
GGGGGCAAGCCATTCTGTCCGCCATGGACAAGCTGTACTGCGAGTGTGGCGCCGTCATGACTTCGAAGCGCGG
GGAAGAATCGATCAAGGACTCTTACCGCTGCCGTCGCCGGAAGGTGGTCGACCCGTCCGCACCTGGGCAGCAC
GAAGGCACGTGCAACGTCAGCATGGCGGCACTCGACAAGTTCGTTGCGGAACGCATCTTCAACAAGATCAGGC
ACGCCGAAGGCGACGAAGAGACGTTGGCGCTTCTGTGGGAAGCCGCCCGACGCTTCGGCAAGCTCACTGAGG
CGCCTGAGAAGAGCGGCGAACGGGCGAACCTTGTTGCGGAGCGCGCCGACGCCCTGAACGCCCTTGAAGAGC
TGTACGAAGACCGCGCGGCAGGCGCGTACGACGGACCCGTTGGCAGGAAGCACTTCCGGAAGCAACAGGCAG
CGCTGACGCTCCGGCAGCAAGGGGCGGAAGAGCGGCTTGCCGAACTTGAAGCCGCCGAAGCCCCGAAGCTTC
CCCTTGACCAATGGTTCCCCGAAGACGCCGACGCTGACCCGACCGGCCCTAAGTCGTGGTGGGGGCGCGCGT
CAGTAGACGACAAGCGCGTGTTCGTCGGGCTCTTCGTAGACAAGATCGTTGTCACGAAGTCGACTACGGGCAG
GGGGCAGGGAACGCCCATCGAGAAGCGCGCTTCGATCACGTGGGCGAAGCCGCCGACCGACGACGACGAAGA
CGACGCCCAGGACGGCACGGAAGACGTAGCGGCGTAGTTAACCTAGGCTGCTGCCACCGCTGAGCAATAACTA
GCATAACCCCTTGGGGCCTCTAAACGGGTCTTGAGGGGTTTTTTGATGGTGGCAGGCCCCGTGGCCGGGGGAC
TGTTGGGCGCCATCTCCTACTTTTCATACTCCCGCCATTCAGAGAAGAAACCAATTGTCCATATTGCATCAGACA
TTGCCGTCACTGCGTCTTTTACTGGCTCTTCTCGCTAACCAAACCGGTAACCCCGCTTATTAAAAGCATTCTGTAA
CAAAGCGGGACCAAAGCCATGACAAAAACGCGTAACAAAAGTGTCTATAATCACGGCAGAAAAGTCCACATTGA
TTATTTGCACGGCGTCACACTTTGCTATGCCATAGCATTTTTATCCATAAGATTAGCGGATCCTACCTGACGCTTT
TTATCGCAACTCTCTACTGTTTCTCCATACCCGTTTTTTTGGGCTAGCGAATTCGAGCTCAAGAGGATACCATATG
AAAAACATTAAAAAAAACCAGGTGATGAACCTGGGCCCGAACAGCAAACTGCTGAAAGAATATAAAAGCCAGCTG
ATTGAACTGAACATTGAACAGTTTGAAGCGGGCATTGGCCTGATTCTGGGCGATGCGTATATTCGCAGCCGCGA
TGAAGGCAAAACCTATTGCATGCAGTTTGAATGGAAAAACAAAGCGTATATGGATCATGTGTGCCTGCTGTATGA
TCAGTGGGTGCTGAGCCCGCCGCATAAAAAAGAACGCGTGAACCATCTGGGCAACCTGGTGATTACCTGGGGC
GCGCAGACCTTTAAACATCAGGCGTTTAACAAACTGGCGAACCTGTTTATTGTGAACAACAAAAAAACCATTCCG
AACAACCTGGTGGAAAACTATCTGACCCCGATGAGCCTGGCGTATTGGTTTATGGATGATGGCGGCAAATGGGA
TTATAACAAAAACAGCACCAACAAAAGCATTGTGCTGAACACCCAGAGCTTTACCTTTGAAGAAGTGGAATATCT
GGTGAAAGGCCTGCGCAACAAATTTCAGCTGAACTGCTATGTGAAAATTAACAAAAACAAACCGATTATTTATATT
GATAGCATGAGCTATCTGATTTTTTATAACCTGATTAAACCGTATCTGATTCCGCAGATGATGTATAAACTGCCGA
ACACCATTAGCAGCGAAACCTTTCTGAAATGAGAATTCAATAATTTTGTTTAACTTTAAGAAGGAGATATACATATG
ATTGACATGCTAGTTTTACGATTACCGTTCATCGATTCTCTTGTTTGCTCCAGACTCTCAGGCAATGACCTGATAG
CCTTTGTAGATCTCTCAAAAATAGCTACCCTCTCCGGCATTAATTTATCAGCTAGAACGGTTGAATATCATATTGA
TGGTGATTTGACTGTCTCCGGCCTTTCTCACCCTTTTGAATCTTTACCTACACATTACTCAGGCATTGCATTTAAA
ATATATGAGGGTTCTAAAAATTTTTATCCTTGCGTTGAAATAAAGGCTTCTCCCGCAAAAGTATTACAGGGTCATA
ATGTTTTTGGTACAACCGATTTAGCTTTATGCTCTGAGGCTTTATTGCTTAATTTTGCTAATTCTTTGCCTTGCCTG
TATGATTTATTGGATGTTAATGCTACTACTATTAGTAGAATTGATGCCACCTTTTCAGCTCGCGCCCCAAATGAAA
ATATAGCTAAACAGGTTATTGACCATTTGCGAAATGTATCTAATGGTCAAACTAAATCTACTCGTTCGCAGAATTG
GGAATCAACTGTTATATGGAATGAAACTTCCAGACACCGTACTTTAGTTGCATATTTAAAACATGTTGAGCTACAG
CATTATATTCAGCAATTAAGCTCTAAGCCATCCGCAAAAATGACCTCTTATCAAAAGGAGCAATTAAAGGTACTCT
CTAATCCTGACCTGTTGGAGTTTGCTTCCGGTCTGGTTCGCTTTGAAGCTCGAATTAAAACGCGATATTTGAAGT
CTTTCGGGCTTCCTCTTAATCTTTTTGATGCAATCCGCTTTGCTTCTGACTATAATAGTCAGGGTAAAGACCTGAT
 
TTTTGATTTATGGTCATTCTCGTTTTCTGAACTGTTTAAAGCATTTGAGGGGGATTCAATGAATATTTATGACGATT
CCGCAGTATTGGACGCTATCCAGTCTAAACATTTTACTATTACCCCCTCTGGCAAAACTTCTTTTGCAAAAGCCTC
TCGCTATTTTGGTTTTTATCGTCGTCTGGTAAACGAGGGTTATGATAGTGTTGCTCTTACTATGCCTCGTAATTCC
TTTTGGCGTTATGTATCTGCATTAGTTGAATGTGGTATTCCTAAATCTCAACTGATGAATCTTTCTACCTGTAATAA
TGTTGTTCCGTTAGTTCGTTTTATTAACGTAGATTTTTCTTCCCAACGTCCTGACTGGTATAATGAGCCAGTTCTTA
AAATCGCATAATCTAGAGTCGACCTGCAGGCATGCAAGCTTGGCTGTTTTGGCGGATGAGAGAAGATTTTCAGC
CTGATACAGATTAAATCAGAACGCAGAAGCGGTCTGATAAAACAGAATTTGCCTGGCGGCAGTAGCGCGGTGGT
CCCACCTGACCCCATGCCGAACTCAGAAGTGAAACGCCGTAGCGCCGATGGTAGTGTGGGGTCTCCCCATGCG
AGAGTAGGGAACTGCCAGGCATCAAATAAAACGAAAGGCTCAGTCGAAAGACTGGGCCTTTCGTTTTATCTGTT
GTTTGTCGGTGAACGCTCTCCTGAGTAGGACAAATCCGCCGGGAGCGGATTTGAACGTTGCGAAGCAACGGCC
CGGAGGGTGGCGGGCAGGACGCCCGCCATAAACTGCCAGGCATCAAATTAAGCAGAAGGCCATCCTGACGGAT
GGCCTTTTTGCGTTTCTACAAACTCGTGGCCGGCATCACCGGCGCCACAGGTGCGGTTGCTGTCTCACCTACCA
AACAATGCCCCCCTGCAAAAAATAAATTCATATAAAAAACATACAGATAACCATCTGCGGTGATAAATTATCTCTG
GCGGTGTTGACATAAATACCACTGGCGGTGATACTGAGCACATCAGCAGGACGCACTGACCACCATGAAGGTGA
CGCTCTTAAAAATTAAGCCCTGAAGAAGGGCAGCATTCAAAGCAGAAGGCTTTGGGGTGTGTGATACGAAACGA
AGCATTGGCCGTAAGTGCGATTCCGGATTAGCTGCCAATGTGCCAATCGCGGGGGGTTTTCGTTCAGGACTACA
ACTGCCACACACCACCAAAGCTAACTGACAGGAGAATCCAGATGAGCAAGGTAAAAATCGGTGAGTTGATCAAC
ACGCTTGTGAATGAGGTAGAGGCAATTGATGCCTCAGACCGCCCACAAGGCGACAAAACGAAGAGAATTAAAGC
CGCAGCCGCACGGTATAAGAACGCGTTATTTAATGATAAAAGAAAGTTCCGTGGGAAAGGATTGCAGAAAAGAA
TAACCGCGAATACTTTTAACGCCTATATGAGCAGGGCAAGAAAGCGGTTTGATGATAAATTACATCATAGCTTTG
ATAAAAATATTAATAAATTATCGGAAAAGTATCCTCTTTACAGCGAAGAATTATCTTCATGGCTTTCTATGCCTACG
GCTAATATTCGCCAGCACATGTCATCGTTACAATCTAAATTGAAAGAAATAATGCCGCTTGCCGAAGAGTTATCAA
ATGTAAGAATAGGCTCTAAAGGCAGTGATGCAAAAATAGCAAGACTAATAAAAAAATATCCAGATTGGAGTTTTGC
TCTTAGTGATTTAAACAGTGATGATTGGAAGGAGCGCCGTGACTATCTTTATAAGTTATTCCAACAAGGCTCTGC
GTTGTTAGAAGAACTACACCAGCTCAAGGTCAACCATGAGGTTCTGTACCATCTGCAGCTAAGCCCTGCGGAGC
GTACATCTATACAGCAACGATGGGCCGATGTTCTGCGCGAGAAGAAGCGTAATGTTGTGGTTATTGACTACCCA
ACATACATGCAGTCTATCTATGATATTTTGAATAATCCTGCGACTTTATTTAGTTTAAACACTCGTTCTGGAATGGC
ACCTTTGGCCTTTGCTCTGGCTGCGGTATCAGGGCGAAGAATGATTGAGATAATGTTTCAGGGTGAATTTGCCGT
TTCAGGAAAGTATACGGTTAATTTCTCAGGGCAAGCTAAAAAACGCTCTGAAGATAAAAGCGTAACCAGAACGAT
TTATACTTTATGCGAAGCAAAATTATTCGTTGAATTATTAACAGAATTGCGTTCTTGCTCTGCTGCATCTGATTTCG
ATGAGGTTGTTAAAGGATATGGAAAGGATGATACAAGGTCTGAGAACGGCAGGATAAATGCTATTTTAGCAAAAG
CATTTAACCCTTGGGTTAAATCATTTTTCGGCGATGACCGTCGTGTTTATAAAGATAGCCGCGCTATTTACGCTCG
CATCGCTTATGAGATGTTCTTCCGCGTCGATCCACGGTGGAAAAACGTCGACGAGGATGTGTTCTTCATGGAGA
TTCTCGGACACGACGATGAGAACACCCAGCTGCACTATAAGCAGTTCAAGCTGGCCAACTTCTCCAGAACCTGG
CGACCTGAAGTTGGGGATGAAAACACCAGGCTGGTGGCTCTGCAGAAACTGGACGATGAAATGCCAGGCTTTG
CCAGAGGTGACGCTGGCGTCCGTCTCCATGAAACCGTTAAGCAGCTGGTGGAGCAGGACCCATCAGCAAAAAT
AACCAACAGCACTCTCCGGGCCTTTAAATTTAGCCCGACGATGATTAGCCGGTACCTGGAGTTTGCCGCTGATG
CATTGGGGCAGTTCGTTGGCGAGAACGGGCAGTGGCAGCTGAAGATAGAGACACCTGCAATCGTCCTGCCTGA
TGAAGAATCCGTTGAGACCATCGACGAACCGGATGATGAGTCCCAAGACGACGAGCTGGATGAAGATGAAATTG
AGCTCGACGAGGGTGGCGGCGATGAACCAACCGAAGAGGAAGGGCCAGAAGAACATCAGCCAACTGCTCTAAA
ACCCGTCTTCAAGCCTGCAAAAAATAACGGGGACGGAACGTACAAGATAGAGTTTGAATACGATGGAAAGCATT
ATGCCTGGTCCGGCCCCGCCGATAGCCCTATGGCCGCAATGCGATCCGCATGGGAAACGTACTACAGCTAATT
AACCTAGGCTGCTGCCACCGCTGAGCAATAAATTCTCACCAATAAAAAACGCCCGGCGGCAACCGAGCGTTCTG
AACAAATCCAGATGGAGTTCTGAGGTCATTACTGGATCTATCAACAGGAGTCCACTCAAAGGCGGTAATACGGTT
ATTACATCGGATGCCGGGACCGACGAGTGCAGAGGCGTGCAAGCGAGCTTGGCGTAATCATGGTCATAGCTGT
TTCCTGTGTGAAATTGTTATCCGCTCACAATTCCACACAACATACGAGCCGGAAGCATAAAGTGTAAAGCCTGGG
 
GTGCCTAATGAGTGAGCTAACTCACATTAATTGCGTTGCGCTCACTGCCCGCTTTCCAGTCGGGAAACCTGTCG
TGCCAGCTGCATTAATGAATCGGCCAACGCGCGGGGAGAGGCGGTTTGCGTATTGGGCGCTCTTCCGCTTCCT
CGCTCACTGACTCGCGGCCGCGCTGCGCTCGGTCGTTCGGCTGCGGCGAGCGGTAGGGATAACAGGGTAATG
TCATCTGGGATAACAGGGTAATGTCATCTAGGGATAACAGGGTATGTCATCTGGGATAACAGGGTAATGTATCTA
GGGATAACAGGGTAATGTCATCTGGGATAACAGGGTAATGTCATCTAGGGATAACAGGGTATGTCATCTGGGAT
AACAGGGTAATGTATCTAGGGATAACAGGGTAATGTCATCTGGGATAACAGGGTAATGTCATCTAGGGATAACAG
GGTATGTCATCTGGGATAACAGGGTAATGTATCTAGGGATAACAGGGTAATGTCATCTGGGATAACAGGGTAAT
GTCATCTAGGGATAACAGGGTATGTCATCTGGGATAACAGGGTAATGTATCTAGGGATAACAGGGTAATGTCATC
TGGGATAACAGGGTAATGTCATCTAGGGATAACAGGGTATGTCATCTGGGATAACAGGGTAATGTATCTTGTATC
TAGGGATAACAGGGTAATTATCAGCTCACTCAAAGGCGGTAATACGGTTATCCACAGAATCAGGGGATAACGCA
GGAAAGAACATGTGAGCAAAAGGCCAGCAAAAGGCCAGGAACCGTAAAAAGGCCGCGTTGCTGGCGTTATGGA
ATAGACTGGATGGAGGCGGATAAAGTTGCAGGACCACTTCTGCGCTCGGCCCTTCCGGCTGGCTGGTTTATTGC
TGATAAATCTGGAGCCGGTGAGCGTGGGTCTCGCGGTATCATTGCAGCACTGGGGCCAGATGGTAAGCCCTCC
CGTATCGTAGTTATCTACACGACGGGGAGTCAGGCAACTATGGATGAACGAAATAGACAGATCGCTGAGATAGG
TGCCTCACTGATTAAGCATTGGTAACTGTCAGACCAAGTTTACTCATATATACTTTAGATTGATTTAAAACTTCATT
TTTAATTTAAAAGGATCTAGGTGAAGATCCTTTTTGATAATCTCATGACCAAAATCCCTTAACGTGAGTTTTCGTTC
CACTGAGCGTCAGACCCCTTAATAAGATGATCTTCTTGAGATCGTTTTGGTCTGCGCGTAATCTCTTGCTCTGAA
AACGAAAAAACCGCCTTGCAGGGCGGTTTTTCGAAGGTTCTCTGAGCTACCAACTCTTTGAACCGAGGTAACTG
GCTTGGAGGAGCGCAGTCACCAAAACTTGTCCTTTCAGTTTAGCCTTAACCGGCGCATGACTTCAAGACTAACTC
CTCTAAATCAATTACCAGTGGCTGCTGCCAGTGGTGCTTTTGCATGTCTTTCCGGGTTGGACTCAAGACGATAGT
TACCGGATAAGGCGCAGCGGTCGGACTGAACGGGGGGTTCGTGCATACAGTCCAGCTTGGAGCGAACTGCCTA
CCCGGAACTGAGTGTCAGGCGTGGAATGAGACAAACGCGGCCATAACAGCGGAATGACACCGGTAAACCGAAA
GGCAGGAACAGGAGAGCGCACGAGGGAGCCGCCAGGGGAAACGCCTGGTATCTTTATAGTCCTGTCGGGTTTC
GCCACCACTGATTTGAGCGTCAGATTTCGTGATGCTTGTCAGGGGGGCGGAGCCTATGGAAAAACGGCTTTGCC
GCGGCCCTCTCACTTCCCTGTTAAGTATCTTCCTGGCATCTTCCAGGAAATCTCCGCCCCGTTCGTAAGCCATTT
CCGCTCGCCGCAGTCGAACGACCGAGCGTAGCGAGTCAGTGAGCGAGGAAGCGGAATATATCCTGTATCACAT
ATTCTGCTGACGCACCGGTGCAGCCTTTTTTCTCCTGCCACATGAAGCACTTCACTGACACCCTCATCAGTGCCA
ACATAGTAAGCCAGTATACACTCCGCTAGCGCTGAGGTCTGCCTCGTGAAGAAGGTGTTGCTGACTCATACCAG
GCCTGAATCGCCCCATCATCCAGCCAGAAAGTGAGGGAGCCACGGTTGATGAGAGCTTTGTTGTAGGTGGACC
AGTTGGTGATTTTGAACTTTTGCTTTGCCACGGAACGGTCTGCGTTGTCGGGAAGATGCGTGATCTGATCCTTCA
ACTCAGCAAAAGTTCGATTTATTCAACAAAGCCACGTTGTGTCTCAAAATCTCTGATGTTACATTGCACAAGATAA
AAATATATCATCATGAACAATAAAACTGTCTGCTTACATAAACAGTAATACAAGGGGTGTTGAAGATCCTTTGAGC
GGCCGCTCTTTTCTACGGGGTCTGACGCTCAGTGGAACGAAAACTCACGTTAAGGGATTTTGGTCATGAGATTAT
CAAAAAGGATCTTCACCTAGATCCTTTTAAATTAAAAATGAAGTTTTAAATCAATCTAAAGTATATATGAGTAAACT
TGGTCTGACAGTTACCAATGCTTAATCAGTGAGGCACCTATCTCAGCGATCTGTCTATTTCGTTCATCCATAGTTG
CCTGACTCCCCGTCGTGTAGATAACTACGATACGGGAGGGCTTACCATCTGGCCCCAGTGCTGCAATGATACCG
CGAGACCCACGCTCACCGGCTCCAGATTTATCAGCAATAAACCAGCCAGCCGGAAGGGCCGAGCGCAGAAGTG
GTCCTGCAACTTTATCCGCCTCCATCCAGTCTATTAATTGTTGCCGGGAAGCTAGAGTAAGTAGTTCGCCAGTTA
ATAGTTTGCGCAACGTTGTTGCCATTGCTACAGGCATCGTGGTGTCACGCTCGTCGTTTGGTATGGCTTCATTCA
GCTCCGGTTCCCAACGATCAAGGCGAGTTACATGATCCCCCATGTTGTGCAAAAAAGCGGTTAGCTCCTTCGGT
CCTCCGATCGTTGTCAGAAGTAAGTTGGCCGCAGTGTTATCACTCATGGTTATGGCAGCACTGCATAATTCTCTT
ACTGTCATGCCATCCGTAAGATGCTTTTCTGTGACTGGTGAGTACTCAACCAAGTCATTCTGAGAATAGTGTATG
CGGCGACCGAGTTGCTCTTGCCCGGCGTCAATACGGGATAATACCGCGCCACATAGCAGAACTTTAAAAGTGCT
CATCATTGGAAAACGTTCTTCGGGGCGAAAACTCTCAAGGATCTTACCGCTGTTGAGATCCAGTTCGATGTAACC
CACTCGTGCACCCAACTGATCTTCAGCATCTTTTACTTTCACCAGCGTTTCTGGGTGAGCAAAAACAGGAAGGCA
AAATGCCGCAAAAAAGGGAATAAGGGCGACACGGAAATGTTGAATACTCATACTCTTCCTTTTTCAATATTATTGA
 
AGCATTTATCAGGGTTATTGTCTCATGAGCGGATACATATTTGAATGTATTTAGAAAAATAAACAAATAGGGGTTC
CGCGCACATTTCCCCGAAAAGTGCCACCTGACGTCTAAGAAACCATTATTATCATGACATTAACCTATAAAAATAG
GCGTATCACGAGGCCCTTTCGTC
**SEQ ID NO 14**
   LENGTH: 29
   TYPE: DNA
   ORGANISM: Artificial
   FEATURE: Promoter
   OTHER INFORMATION: pTac
   SEQUENCE: 14
      TTGACAATTAATCATCGGCTCGTATAATG
**SEQ ID NO 15**
   LENGTH: 1.083
   TYPE: DNA
   ORGANISM: Artificial
   FEATURE: Repressor Protein
   OTHER INFORMATION: Laclq
   SEQUENCE: 15 
ATGAAACCAGTAACGTTATACGATGTCGCAGAGTATGCCGGTGTCTCTTATCAGACCGTTTCCCGCGTGGTGAA
CCAGGCCAGCCACGTTTCTGCGAAAACGCGGGAAAAAGTGGAAGCGGCGATGGCGGAGCTGAATTACATTCCC
AACCGCGTGGCACAACAACTGGCGGGCAAACAGTCGTTGCTGATTGGCGTTGCCACCTCCAGTCTGGCCCTGC
ACGCGCCGTCGCAAATTGTCGCGGCGATTAAATCTCGCGCCGATCAACTGGGTGCCAGCGTGGTGGTGTCGAT
GGTAGAACGAAGCGGCGTCGAAGCCTGTAAAGCGGCGGTGCACAATCTTCTCGCGCAACGCGTCAGTGGGCTG
ATCATTAACTATCCGCTGGATGACCAGGATGCCATTGCTGTGGAAGCTGCCTGCACTAATGTTCCGGCGTTATTT
CTTGATGTCTCTGACCAGACACCCATCAACAGTATTATTTTCTCCCATGAAGACGGTACGCGACTGGGCGTGGA
GCATCTGGTCGCATTGGGTCACCAGCAAATCGCGCTGTTAGCGGGCCCATTAAGTTCTGTCTCGGCGCGTCTGC
GTCTGGCTGGCTGGCATAAATATCTCACTCGCAATCAAATTCAGCCGATAGCGGAACGGGAAGGCGACTGGAGT
GCCATGTCCGGTTTTCAACAAACCATGCAAATGCTGAATGAGGGCATCGTTCCCACTGCGATGCTGGTTGCCAA
CGATCAGATGGCGCTGGGCGCAATGCGCGCCATTACCGAGTCCGGGCTGCGCGTTGGTGCGGATATCTCGGTA
GTGGGATACGACGATACCGAAGACAGCTCATGTTATATCCCGCCGTCAACCACCATCAAACAGGATTTTCGCCT
GCTGGGGCAAACCAGCGTGGACCGCTTGCTGCAACTCTCTCAGGGCCAGGCGGTGAAGGGCAATCAGCTGTTG
CCCGTCTCACTGGTGAAAAGAAAAACCACCCTGGCGCCCAATACGCAAACCGCCTCTCCCCGCGCGTTGGCCG
ATTCATTAATGCAGCTGGCACGACAGGTTTCCCGACTGGAAAGCGGGCAGTGA
**SEQ ID NO 16**
   LENGTH: 879
   TYPE: DNA
   ORGANISM: Artificial
   FEATURE: Repressor Protein
   OTHER INFORMATION: AraC
   SEQUENCE: 16 
ATGGCTGAAGCGCAAAATGATCCCCTGCTGCCGGGATACTCGTTTAATGCCCATCTGGTGGCGGGTTTAACGCC
GATTGAGGCCAACGGTTATCTCGATTTTTTTATCGACCGACCGCTGGGAATGAAAGGTTATATTCTCAATCTCAC
CATTCGCGGTCAGGGGGTGGTGAAAAATCAGGGACGAGAATTTGTTTGCCGACCGGGTGATATTTTGCTGTTCC
 
CGCCAGGAGAGATTCATCACTACGGTCGTCATCCGGAGGCTCGCGAATGGTATCACCAGTGGGTTTACTTTCGT
CCGCGCGCCTACTGGCATGAATGGCTTAACTGGCCGTCAATATTTGCCAATACGGGGTTCTTTCGCCCGGATGA
AGCGCACCAGCCGCATTTCAGCGACCTGTTTGGGCAAATCATTAACGCCGGGCAAGGGGAAGGGCGCTATTCG
GAGCTGCTGGCGATAAATCTGCTTGAGCAATTGTTACTGCGGCGCATGGAAGCGATTAACGAGTCGCTCCATCC
ACCGATGGATAATCGGGTACGCGAGGCTTGTCAGTACATCAGCGATCACCTGGCAGACAGCAATTTTGATATCG
CCAGCGTCGCACAGCATGTTTGCTTGTCGCCGTCGCGTCTGTCACATCTTTTCCGCCAGCAGTTAGGGATTAGC
GTCTTAAGCTGGCGCGAGGACCAACGTATCAGCCAGGCGAAGCTGCTTTTGAGCACCACCCGGATGCCTATCG
CCACCGTCGGTCGCAATGTTGGTTTTGACGATCAACTCTATTTCTCGCGGGTATTTAAAAAATGCACCGGGGCCA
GCCCGAGCGAGTTCCGTGCCGGTTGTGAAGAAAAAGTGAATGATGTAGCCGTCAAGTTGTCATAA
**SEQ ID NO 17**
   LENGTH: 714
   TYPE: DNA
   ORGANISM: Artificial
   FEATURE: Repressor Protein
   OTHER INFORMATION: Lambda Repressor
   SEQUENCE: 17 
ATGAGCACAAAAAAGAAACCATTAACACAAGAGCAGCTTGAGGACGCACG
TCGCCTTAAAGCAATTTATGAAAAAAAGAAAAATGAACTTGGCTTATCCCA
GGAATCTGTCGCAGACAAGATGGGGATGGGGCAGTCAGGCGTTGGTGCTTT
ATTTAATGGCATCAATGCATTAAATGCTTATAACGCCGCATTGCTTGCAAAA
ATTCTCAAAGTTAGCGTTGAAGAATTTAGCCCTTCAATCGCCAGAGAAATCT
ACGAGATGTATGAAGCGGTTAGTATGCAGCCGTCACTTAGAAGTGAGTATG
AGTACCCTGTTTTTTCTCATGTTCAGGCAGGGATGTTCTCACCTGAGCTTAG
AACCTTTACCAAAGGTGATGCGGAGAGATGGGTAAGCACAACCAAAAAAG
CCAGTGATTCTGCATTCTGGCTTGAGGTTGAAGGTAATTCCATGACCGCACC
AACAGGCTCCAAGCCAAGCTTTCCTGACGGAATGTTAATTCTCGTTGACCCT
GAGCAGGCTGTTGAGCCAGGTGATTTCTGCATAGCCAGACTTGGGGGTGAT
GAGTTTACCTTCAAGAAACTGATCAGGGATAGCGGTCAGGTGTTTTTACAAC
CACTAAACCCACAGTACCCAATGATCCCATGCAATGAGAGTTGTTCCGTTGT
GGGGAAAGTTATCGCTAGTCAGTGGCCTGAAGAGACGTTTGGCTGA
**SEQ ID NO 18**
   LENGTH: 1.233
   TYPE: DNA
   ORGANISM: Artificial
   FEATURE: "rolling circle" Replication Protein
   OTHER INFORMATION: M13 g2p
   SEQUENCE: 18 
ATGATTGACATGCTAGTTTTACGATTACCGTTCATCGATTCTCTTGTTTGCTC
CAGACTCTCAGGCAATGACCTGATAGCCTTTGTAGATCTCTCAAAAATAGCT
ACCCTCTCCGGCATTAATTTATCAGCTAGAACGGTTGAATATCATATTGATG
GTGATTTGACTGTCTCCGGCCTTTCTCACCCTTTTGAATCTTTACCTACACAT
TACTCAGGCATTGCATTTAAAATATATGAGGGTTCTAAAAATTTTTATCCTT
GCGTTGAAATAAAGGCTTCTCCCGCAAAAGTATTACAGGGTCATAATGTTTT
TGGTACAACCGATTTAGCTTTATGCTCTGAGGCTTTATTGCTTAATTTTGCTA
 
ATTCTTTGCCTTGCCTGTATGATTTATTGGATGTTAATGCTACTACTATTAGT
AGAATTGATGCCACCTTTTCAGCTCGCGCCCCAAATGAAAATATAGCTAAAC
AGGTTATTGACCATTTGCGAAATGTATCTAATGGTCAAACTAAATCTACTCG
TTCGCAGAATTGGGAATCAACTGTTATATGGAATGAAACTTCCAGACACCGT
ACTTTAGTTGCATATTTAAAACATGTTGAGCTACAGCATTATATTCAGCAAT
TAAGCTCTAAGCCATCCGCAAAAATGACCTCTTATCAAAAGGAGCAATTAA
AGGTACTCTCTAATCCTGACCTGTTGGAGTTTGCTTCCGGTCTGGTTCGCTTT
GAAGCTCGAATTAAAACGCGATATTTGAAGTCTTTCGGGCTTCCTCTTAATC
TTTTTGATGCAATCCGCTTTGCTTCTGACTATAATAGTCAGGGTAAAGACCT
GATTTTTGATTTATGGTCATTCTCGTTTTCTGAACTGTTTAAAGCATTTGAGG
GGGATTCAATGAATATTTATGACGATTCCGCAGTATTGGACGCTATCCAGTC
TAAACATTTTACTATTACCCCCTCTGGCAAAACTTCTTTTGCAAAAGCCTCTC
GCTATTTTGGTTTTTATCGTCGTCTGGTAAACGAGGGTTATGATAGTGTTGCT
CTTACTATGCCTCGTAATTCCTTTTGGCGTTATGTATCTGCATTAGTTGAATG
TGGTATTCCTAAATCTCAACTGATGAATCTTTCTACCTGTAATAATGTTGTTC
CGTTAGTTCGTTTTATTAACGTAGATTTTTCTTCCCAACGTCCTGACTGGTAT
AATGAGCCAGTTCTTAAAATCGCATAA
**SEQ ID NO 19**
   LENGTH: 1.896
   TYPE: DNA
   ORGANISM: Artificial
   FEATURE: Recombinase
   OTHER INFORMATION: N15 TelN Recombinase
   SEQUENCE: 19 
ATGAGCAAGGTAAAAATCGGTGAGTTGATCAACACGCTTGTGAATGAGGTA
GAGGCAATTGATGCCTCAGACCGCCCACAAGGCGACAAAACGAAGAGAATT
AAAGCCGCAGCCGCACGGTATAAGAACGCGTTATTTAATGATAAAAGAAAG
TTCCGTGGGAAAGGATTGCAGAAAAGAATAACCGCGAATACTTTTAACGCC
TATATGAGCAGGGCAAGAAAGCGGTTTGATGATAAATTACATCATAGCTTT
GATAAAAATATTAATAAATTATCGGAAAAGTATCCTCTTTACAGCGAAGAA
TTATCTTCATGGCTTTCTATGCCTACGGCTAATATTCGCCAGCACATGTCATC
GTTACAATCTAAATTGAAAGAAATAATGCCGCTTGCCGAAGAGTTATCAAA
TGTAAGAATAGGCTCTAAAGGCAGTGATGCAAAAATAGCAAGACTAATAAA
AAAATATCCAGATTGGAGTTTTGCTCTTAGTGATTTAAACAGTGATGATTGG
AAGGAGCGCCGTGACTATCTTTATAAGTTATTCCAACAAGGCTCTGCGTTGT
TAGAAGAACTACACCAGCTCAAGGTCAACCATGAGGTTCTGTACCATCTGC
AGCTAAGCCCTGCGGAGCGTACATCTATACAGCAACGATGGGCCGATGTTC
TGCGCGAGAAGAAGCGTAATGTTGTGGTTATTGACTACCCAACATACATGC
AGTCTATCTATGATATTTTGAATAATCCTGCGACTTTATTTAGTTTAAACACT
CGTTCTGGAATGGCACCTTTGGCCTTTGCTCTGGCTGCGGTATCAGGGCGAA
GAATGATTGAGATAATGTTTCAGGGTGAATTTGCCGTTTCAGGAAAGTATAC
GGTTAATTTCTCAGGGCAAGCTAAAAAACGCTCTGAAGATAAAAGCGTAAC
CAGAACGATTTATACTTTATGCGAAGCAAAATTATTCGTTGAATTATTAACA
GAATTGCGTTCTTGCTCTGCTGCATCTGATTTCGATGAGGTTGTTAAAGGAT
ATGGAAAGGATGATACAAGGTCTGAGAACGGCAGGATAAATGCTATTTTAG
 
CAAAAGCATTTAACCCTTGGGTTAAATCATTTTTCGGCGATGACCGTCGTGT
TTATAAAGATAGCCGCGCTATTTACGCTCGCATCGCTTATGAGATGTTCTTC
CGCGTCGATCCACGGTGGAAAAACGTCGACGAGGATGTGTTCTTCATGGAG
ATTCTCGGACACGACGATGAGAACACCCAGCTGCACTATAAGCAGTTCAAG
CTGGCCAACTTCTCCAGAACCTGGCGACCTGAAGTTGGGGATGAAAACACC
AGGCTGGTGGCTCTGCAGAAACTGGACGATGAAATGCCAGGCTTTGCCAGA
GGTGACGCTGGCGTCCGTCTCCATGAAACCGTTAAGCAGCTGGTGGAGCAG
GACCCATCAGCAAAAATAACCAACAGCACTCTCCGGGCCTTTAAATTTAGC
CCGACGATGATTAGCCGGTACCTGGAGTTTGCCGCTGATGCATTGGGGCAGT
TCGTTGGCGAGAACGGGCAGTGGCAGCTGAAGATAGAGACACCTGCAATCG
TCCTGCCTGATGAAGAATCCGTTGAGACCATCGACGAACCGGATGATGAGT
CCCAAGACGACGAGCTGGATGAAGATGAAATTGAGCTCGACGAGGGTGGC
GGCGATGAACCAACCGAAGAGGAAGGGCCAGAAGAACATCAGCCAACTGC
TCTAAAACCCGTCTTCAAGCCTGCAAAAAATAACGGGGACGGAACGTACAA
GATAGAGTTTGAATACGATGGAAAGCATTATGCCTGGTCCGGCCCCGCCGA
TAGCCCTATGGCCGCAATGCGATCCGCATGGGAAACGTACTACAGCTAA
**SEQ ID NO 20**
   LENGTH: 1.818
   TYPE: DNA
   ORGANISM: Artificial
   FEATURE: Recombinase
   OTHER INFORMATION: PhiC31 Recombinase
   SEQUENCE: 20 
ATGGACACGTACGCGGGTGCTTACGACCGTCAGTCGCGCGAGCGCGAGAAT
TCGAGCGCAGCAAGCCCAGCGACACAGCGTAGCGCCAACGAAGACAAGGC
GGCCGACCTTCAGCGCGAAGTCGAGCGCGACGGGGGCCGGTTCAGGTTCGT
CGGGCATTTCAGCGAAGCGCCGGGCACGTCGGCGTTCGGGACGGCGGAGCG
CCCGGAGTTCGAACGCATCCTGAACGAATGCCGCGCCGGGCGGCTCAACAT
GATCATTGTCTATGACGTGTCGCGCTTCTCGCGCCTGAAGGTCATGGACGCG
ATTCCGATTGTCTCGGAATTGCTCGCCCTGGGCGTGACGATTGTTTCCACTC
AGGAAGGCGTCTTCCGGCAGGGAAACGTCATGGACCTGATTCACCTGATTA
TGCGGCTCGACGCGTCGCACAAAGAATCTTCGCTGAAGTCGGCGAAGATTC
TCGACACGAAGAACCTTCAGCGCGAATTGGGCGGGTACGTCGGCGGGAAGG
CGCCTTACGGCTTCGAGCTTGTTTCGGAGACGAAGGAGATCACGCGCAACG
GCCGAATGGTCAATGTCGTCATCAACAAGCTTGCGCACTCGACCACTCCCCT
TACCGGACCCTTCGAGTTCGAGCCCGACGTAATCCGGTGGTGGTGGCGTGA
GATCAAGACGCACAAACACCTTCCCTTCAAGCCGGGCAGTCAAGCCGCCAT
TCACCCGGGCAGCATCACGGGGCTTTGTAAGCGCATGGACGCTGACGCCGT
GCCGACCCGGGGCGAGACGATTGGGAAGAAGACCGCTTCAAGCGCCTGGG
ACCCGGCAACCGTTATGCGAATCCTTCGGGACCCGCGTATTGCGGGCTTCGC
CGCTGAGGTGATCTACAAGAAGAAGCCGGACGGCACGCCGACCACGAAGA
TTGAGGGTTACCGCATTCAGCGCGACCCGATCACGCTCCGGCCGGTCGAGC
TTGATTGCGGACCGATCATCGAGCCCGCTGAGTGGTATGAGCTTCAGGCGTG
GTTGGACGGCAGGGGGCGCGGCAAGGGGCTTTCCCGGGGGCAAGCCATTCT
GTCCGCCATGGACAAGCTGTACTGCGAGTGTGGCGCCGTCATGACTTCGAA
 
GCGCGGGGAAGAATCGATCAAGGACTCTTACCGCTGCCGTCGCCGGAAGGT
GGTCGACCCGTCCGCACCTGGGCAGCACGAAGGCACGTGCAACGTCAGCAT
GGCGGCACTCGACAAGTTCGTTGCGGAACGCATCTTCAACAAGATCAGGCA
CGCCGAAGGCGACGAAGAGACGTTGGCGCTTCTGTGGGAAGCCGCCCGACG
CTTCGGCAAGCTCACTGAGGCGCCTGAGAAGAGCGGCGAACGGGCGAACCT
TGTTGCGGAGCGCGCCGACGCCCTGAACGCCCTTGAAGAGCTGTACGAAGA
CCGCGCGGCAGGCGCGTACGACGGACCCGTTGGCAGGAAGCACTTCCGGAA
GCAACAGGCAGCGCTGACGCTCCGGCAGCAAGGGGCGGAAGAGCGGCTTG
CCGAACTTGAAGCCGCCGAAGCCCCGAAGCTTCCCCTTGACCAATGGTTCCC
CGAAGACGCCGACGCTGACCCGACCGGCCCTAAGTCGTGGTGGGGGCGCGC
GTCAGTAGACGACAAGCGCGTGTTCGTCGGGCTCTTCGTAGACAAGATCGTT
GTCACGAAGTCGACTACGGGCAGGGGGCAGGGAACGCCCATCGAGAAGCG
CGCTTCGATCACGTGGGCGAAGCCGCCGACCGACGACGACGAAGACGACGC
CCAGGACGGCACGGAAGACGTAGCGGCGTAG
**SEQ ID NO 21**
   LENGTH: 708
   TYPE: DNA
   ORGANISM: Artificial
   FEATURE: Endonuclease
   OTHER INFORMATION: I-SceI Endonuclease
   SEQUENCE: 21 
ATGAAGAACATCAAAAAAAATCAAGTAATCAATCTTGGACCGAACTCGAAA
CTGTTAAAGGAGTACAAGAGCCAACTTATCGAATTGAACATTGAACAGTTT
GAAGCGGGCATTGGACTGATCCTTGGAGACGCTTATATTCGTAGTCGTGACG
AAGGCAAGTTGTATTGTATGCAATTTGAATGAAAAAATAAAGCATACATGG
ACCATGTGTGTTTGCTGTATGACCAGTAAGTGCTGAGTCCGCCACATAAAAA
AGAACGTGTCAACCATCTTGGTAACTTGGTAATCACATAAGGTGCACAGAC
ATTCAAACATCAAGCTTTCAACAAATTGGCGAATCTTTTTATTGTGAACAAC
AAAAAATTGATCCCCAACAATTTAGTTGAAAATTATCTTACTCCAATTTCGC
TGGCATATTGATTTATCGATGACGGAGGGAAGTAAGATTACAACAAGAACA
GCTTAAACAAATCTATTGTCCTGAACACGCAGTCTTTCACGTTTGAAGAGGT
TGAGTATTTAGTGAAAGGCCTTCGCAACAAGTTTCAGTTGAACTGTTACGTC
AAAATTAACAAAAACAAGCCAATCATCTATATTGACTCTATCTCTTATTTAA
TCTTTTACAACCTGATTAAACCATACCTTATCCCTCAAATGATTTATAAGCTG
CCTAATACAATTAGTTCTGAAACTTTTTTGAAATAA
**SEQ ID NO 22**
   LENGTH: 357
   TYPE: DNA
   ORGANISM: Artificial
   FEATURE: Origin of Replication
   OTHER INFORMATION: R6K Origin of Replication
   SEQUENCE: 22 
TGTCAGCCGTTAAGTGTTCCTGTGTCACTCAAAATTGCTTTGAGAGGCTCTAAGGGCTTCTCAGTGCGTTACATC
CCTGGCTTGTTGTCCACAACCGTTAAACCTTAAAAGCTTTAAAAGCCTTATATATTCTTTTTTTTCTTATAAAACTTA
 
AAACCTTAGAGGCTATTTAAGTTGCTGATTTATATTAATTTTATTGTTCAAACATGAGAGCTTAGTACGTGAAACAT
GAGAGCTTAGTACGTTAGCCATGAGAGCTTAGTACGTTAGCCATGAGGGTTTAGTTCGTTAAACATGAGAGCTTA
GTACGTTAAACTTGAGAGCTTAGTACGTGAAACATGAGAGCTTAGTACGTAC
**SEQ ID NO 23**
   LENGTH: 1.136
   TYPE: DNA
   ORGANISM: Artificial
   FEATURE: DNA Antibiotic Resistance Cassette
   OTHER INFORMATION: Ampicillin Resistance Cassette
   SEQUENCE: 23 
CGCGGAACCCCTATTTGTTTATTTTTCTAAATACATTCAAATATGTATCCGCT
CATGAGACAATAACCCTGATAAATGCTTCAATAATATTGAAAAAGGAAGAG
TATGAGTATTCAACATTTCCGTGTCGCCCTTATTCCCTTTTTTGCGGCATTTT
GCCTTCCTGTTTTTGCTCACCCAGAAACGCTGGTGAAAGTAAAAGATGCTGA
AGATCAGTTGGGTGCACGAGTGGGTTACATCGAACTGGATCTCAACAGCGG
TAAGATCCTTGAGAGTTTTCGCCCCGAAGAACGTTTTCCAATGATGAGCACT
TTTAAAGTTCTGCTATGTGGCGCGGTATTATCCCGTATTGACGCCGGGCAAG
AGCAACTCGGTCGCCGCATACACTATTCTCAGAATGACTTGGTTGAGTACTC
ACCAGTCACAGAAAAGCATCTTACGGATGGCATGACAGTAAGAGAATTATG
CAGTGCTGCCATAACCATGAGTGATAACACTGCGGCCAACTTACTTCTGACA
ACGATCGGAGGACCGAAGGAGCTAACCGCTTTTTTGCACAACATGGGGGAT
CATGTAACTCGCCTTGATCGTTGGGAACCGGAGCTGAATGAAGCCATACCA
AACGACGAGCGTGACACCACGATGCCTGTAGCAATGGCAACAACGTTGCGC
AAACTATTAACTGGCGAACTACTTACTCTAGCTTCCCGGCAACAATTAATAG
ACTGGATGGAGGCGGATAAAGTTGCAGGACCACTTCTGCGCTCGGCCCTTC
CGGCTGGCTGGTTTATTGCTGATAAATCTGGAGCCGGTGAGCGTGGGTCTCG
CGGTATCATTGCAGCACTGGGGCCAGATGGTAAGCCCTCCCGTATCGTAGTT
ATCTACACGACGGGGAGTCAGGCAACTATGGATGAACGAAATAGACAGATC
GCTGAGATAGGTGCCTCACTGATTAAGCATTGGTAACTGTCAGACCAAGTTT
ACTCATATATACTTTAGATTGATTTAAAACTTCATTTTTAATTTAAAAGGATC
TAGGTGAAGATCCTTTTTGATAATCTCATGACCAAAATCCCTTAACGTGAGT
TTTCGTTCCACTGAGCGTCAGACCCCGTAGAAAAGATCAAAGGATCTTC
**SEQ ID NO 24**
   LENGTH: 806
   TYPE: DNA
   ORGANISM: Artificial
   FEATURE: DNA Antibiotic Resistance Cassette
   OTHER INFORMATION: Chloramphenicol Resistance Cassette
   SEQUENCE: 24 
ATCGAGATTTTCAGGAGCTAAGGAAGCTAAAATGGAGAAAAAAATCACTGG
ATATACCACCGTTGATATATCCCAATGGCATCGTAAAGAACATTTTGAGGCA
TTTCAGTCAGTTGCTCAATGTACCTATAACCAGACCGTTCAGCTGGATATTA
CGGCCTTTTTAAAGACCGTAAAGAAAAATAAGCACAAGTTTTATCCGGCCTT
TATTCACATTCTTGCCCGCCTGATGAATGCTCATCCGGAATTTCGTATGGCA
 
ATGAAAGACGGTGAGCTGGTGATATGGGATAGTGTTCACCCTTGTTACACC
GTTTTCCATGAGCAAACTGAAACGTTTTCATCGCTCTGGAGTGAATACCACG
ACGATTTCCGGCAGTTTCTACACATATATTCGCAAGATGTGGCGTGTTACGG
TGAAAACCTGGCCTATTTCCCTAAAGGGTTTATTGAGAATATGTTTTTCGTCT
CAGCCAATCCCTGGGTGAGTTTCACCAGTTTTGATTTAAACGTGGCCAATAT
GGACAACTTCTTCGCCCCCGTTTTCACCATGGGCAAATATTATACGCAAGGC
GACAAGGTGCTGATGCCGCTGGCGATTCAGGTTCATCATGCCGTTTGTGATG
GCTTCCATGTCGGCAGAATGCTTAATGAATTACAACAGTACTGCGATGAGTG
GCAGGGCGGGGCGTAATTTGATATCGAGCTCGCTTGGACTCCTGTTGATAGA
TCCAGTAATGACCTCAGAACTCCATCTGGATTTGTTCAGAACGCTCGGTTGC
CGCCGGGCGTTTTTTATTGGTGAGAAT
**SEQ ID NO 25**
   LENGTH: 5.951
   TYPE: DNA
   ORGANISM: Artificial
   FEATURE: Parental Plasmid
   OTHER INFORMATION: pDNAv_A
   SEQUENCE: 25 
GGATCCACTAGTATCTCGACGCATTCATTACCCTGTTATCCCTAGATACAAGATACATTACCCTGTTATCCCAGAT
GACATACCCTGTTATCCCTAGATGACATTACCCTGTTATCCCAGATGACATTACCCTGTTATCCCTAGATACATTA
CCCTGTTATCCCAGATGACATACCCTGTTATCCCTAGATGACATTACCCTGTTATCCCAGATGACATTACCCTGTT
ATCCCTAGATACATTACCCTGTTATCCCAGATGACATACCCTGTTATCCCTAGATGACATTACCCTGTTATCCCAG
ATGACATTACCCTGTTATCCCTAGATACATTACCCTGTTATCCCAGATGACATACCCTGTTATCCCTAGATGACAT
TACCCTGTTATCCCAGATGACATTACCCTGTTATCCCTAGATACATTACCCTGTTATCCCAGATGACATACCCTGT
TATCCCTAGATGACATTACCCTGTTATCCCAGATGACATTACCCTGTTATCCCTAGAAGATCCTTTGATGCGGCC
GCATAACTTCGTATAGCATACATTATACGAAGTTATCTCGAGGTCAGTGTCAAGTCAGTGACTACTGTACTGAACT
GTACGATATCGGTACCTAGATGTCAACTTCAGCACACAATTGCCATTATACGCGCGTATAATGGACTATTGTGTG
CTGATACGATCGCAGACCATGGTCCTTCGATTGGTGTGGAAAGTCCCCAGGCTCCCCAGCAGGCAGAAGTATG
CAAAGCATGCATCTCAATTAGTCAGCAACCAGCATGCATGCATTGGTGTGGAAAGTCCCCAGGCTCCCCAGCAG
GCAGAAGTATGCAAAGCATGCATCTCAATTAGTCAGCAACCAGCATGCATGCATCTATCCATAGCTGATTGGTCT
AAAATGAGATACATCAACGCTCCTCCATGTTTTTTGTTTTCTTTTTAAATGAAAAACTTTATTTTTTAAGAGGAGTTT
CAGGTTCATAGCAAAATTGAGAGGAAGGTACATTCAAGCTGAGGAAGTTTTCCTCTATTCCTAGTTTACTGAGAG
ATTGCATCATGAATGGGTGTTAAATTTTGTCAAATGCTTTTTCTGTGTCTATCAATATGACCATGTGATTTTCTTCT
TTAACCTGTTGATGGGACAAATTACGTTAATTGATTTTCAAACGTTGAACCACCCTTACATATCTGGAATAAATTCT
ACTTGGTTGTGGTGTATATTTTTTGATACATTCTTGGATTCTTTTTGCTAATATTTTGTTGAAAATGTTTGTATCTTT
GTTCATGAGAGATATTGGTCTGTTGTTTTCTTTTCTTGTAATGTCATTTTCTAGTTCCGGTATTAAGGTAATGCTGG
CCTAGTTGAATGATTTAGGAAGTATTCCCTCTGCTTCTGTCTTCTGAAAGAGATTGTAGAAAGTTGATACAATTTTT
TTTTCTTTAAATATCTTGATAGGTGGCTATGGCAGGGCTTGCCGCCCCGACGTTGGCTGCGAGCCCTGGGCCTT
CACCCGAACTTGGGGGTTGGGGTGGGGAAAAGGAAGAAACGCGGGCGTATTGGTCCCAATGGGGTCTCGGTG
GGGTATCGACAGAGTGCCAGCCCTGGGACCGAACCCCGCGTTTATGAACAAACGACCCAACACCCGTGCGTTT
TATTCTGTCTTTTTATTGCCGTCATAGCGCGGGTTCCTTCCGGTATTGTCTCCTTCCGTGTTTCAGTTAGCCTCCC
CCATCTCCCGCTGCAGGAATTCCGCGGAACCCCTATTTGTTTATTTTTCTAAATACATTCAAATATGTATCCGCTC
ATGAGACAATAACCCTGATAAATGCTTCAATAATATTGAAAAAGGAAGAGTATGCGTTTGAAGGTGATGATGGAT
GTCAATAAAAAAACCAAGATTCGTCATCGTAATGAGCTTAATCATACCTTAGCTCAGCTGCCTTTACCAGCAAAAC
GCGTGATGTATATGGCCCTTGCCCCCATTGACAGTAAGGAGCCGTTAGAACGCGGTCGTGTATTTAAGATTCGT
 
GCCGAAGACTTAGCGGCGCTTGCAAAAATTACTCCTAGTTTGGCCTACCGTCAACTGAAGGAGGGAGGCAAATT
GCTGGGCGCCTCGAAGATCAGCTTGCGTGGAGACGATATCATCGCCCTTGCTAAGGAGTTGAACTTGCCATTCA
CTGCGAAAAATTCCCCGGAGGAATTAGACCTGAATATCATTGAGTGGATCGCTTACAGCCCTGACGAGGGCTAC
TTGAGTCTGAAGTTCACGCGTACTATTGAGCCTTATATTTCTAGTCTTATCGGGAAAAAGAACAAATTTACCACGC
AATTATTGACTGCATCCTTGCGCCTGTCGTCACAGTATTCTTCATCACTGTATCAACTGATCCGCAAACATTATAG
TAATTTCAAAAAAAAAAACTATTTTATCATTAGCGTGGACGAGCTTAAAGAAGAACTGATTGCCTATACTTTTGATA
AAGACGGGAACATCGAATATAAATACCCGGACTTCCCAATTTTTAAACGCGATGTTTTAAACAAAGCGATTGCAG
AAATCAAAAAGAAAACTGAGATTTCGTTTGTTGGTTTTACAGTACACGAGAAGGAGGGGCGCAAAATTTCCAAAT
TAAAATTTGAATTTGTCGTTGATGAGGACGAGTTTAGTGGAGATAAAGACGACGAGGCTTTCTTTATGAACTTGTC
GGAGGCCGATGCGGCATTTTTAAAAGTGTTCGATGAAACCGTTCCGCCCAAAAAAGCCAAGGGCTAAAAGCCCG
AAAGGAAGCTGAGTTGGCTGCTGCCACCGCTGAGCAATAACTAGCATAACCCCTTGGGGCCTCTAAACGGGTCT
TGAGGGGTTTTTTGGCTAGCTCTAGAGTCGACGATCATAATCAGCCATACCACATTTGTAGAGGTTTTACTTGCTT
TAAAAAACCTCCCACACCTCCCCCTGAACCTGAAACATAAAATGAATGCAATTGTTGTTGTTAACTTGTTTATTGC
AGCTTATAATGGTTACAAATAAAGCAATAGCATCACAAATTTCACAAATAAAGCATTTTTTTCACTGCATTCTAGTT
GTGGTTTGTCCAAACTCATCAATGTATCTTATCATGTCTGGATCCTATCCATAGCTGATTGGTCTAAAATGAGATA
CATCAACGCTCCTCCATGTTTTTTGTTTTCTTTTTAAATGAAAAACTTTATTTTTTAAGAGGAGTTTCAGGTTCATAG
CAAAATTGAGAGGAAGGTACATTCAAGCTGAGGAAGTTTTCCTCTATTCCTAGTTTACTGAGAGATTGCATCATGA
ATGGGTGTTAAATTTTGTCAAATGCTTTTTCTGTGTCTATCAATATGACCATGTGATTTTCTTCTTTAACCTGTTGA
TGGGACAAATTACGTTAATTGATTTTCAAACGTTGAACCACCCTTACATATCTGGAATAAATTCTACTTGGTTGTG
GTGTATATTTTTTGATACATTCTTGGATTCTTTTTGCTAATATTTTGTTGAAAATGTTTGTATCTTTGTTCATGAGAG
ATATTGGTCTGTTGTTTTCTTTTCTTGTAATGTCATTTTCTAGTTCCGGTATTAAGGTAATGCTGGCCTAGTTGAAT
GATTTAGGAAGTATTCCCTCTGCTTCTGTCTTCTGAAAGAGATTGTAGAAAGTTGATACAATTTTTTTTTCTTTAAA
TATCTTGATAGGCACGCATGCATTGGTGTGGAAAGTCCCCAGGCTCCCCAGCAGGCAGAAGTATGCAAAGCATG
CATCTCAATTAGTCAGCAACCAGCATGCATGCATTGGTGTGGAAAGTCCCCAGGCTCCCCAGCAGGCAGAAGTA
TGCAAAGCATGCATCTCAATTAGTCAGCAACCAGCATGCATGCATGCGAAGTACGAGCTCCGATCTCCAATTTAT
CAGCACACAATAGTCCATTATACGCGCGTATAATGGCAATTGTGTGCTGACTACGTACGTACACTACTACGAACT
CAGAGTCAGTAGATCTGATATCATCGTCTACACTTGCCAGCGCCCTAGCGCCCGCTCCTTTCGCTTTCTTCCCTT
CCTTTCTCGCCACGTTCGCCGGCTTTCCCCGTCAAGCTCTAAATCGGGGGCTCCCTTTAGGGTTCCGATTTAGT
GCTTTACGGCACCTCGACCCCAAAAAACTTGATTTGGGTGATGGTTCACGTAGTGGGCCATCGCCCTGATAGAC
GGTTTTTCGCCCTTTGACGTTGGAGTCCACGTTCTTTAATAGTGGACTCTTGTTCCAAACTGGAACAACACTCAA
CCCTATCTCGGGCTATTCTTTTGATTTATAAGGGATTTTGCCGATTTCGGGAAGATCCTTTGATGGTGCCAGGGC
GTGCCCTTGGGCTCCCCGGGCGCGCTTTTCTACGGGGTTGACGCTCAGTCCTCGCGGAACCCCTATTTGTTTAT
TTTTCTAAATACATTCAAATATGTATCCGCTCATGAGACAATAACCCTGATAAATGCTTCAATAATATTGAAAAAGG
AAGAGTATGAGTATTCAACATTTCCGTGTCGCCCTTATTCCCTTTTTTGCGGCATTTTGCCTTCCTGTTTTTGCTC
ACCCAGAAACGCTGGTGAAAGTAAAAGATGCTGAAGATCAGTTGGGTGCACGAGTGGGTTACATCGAACTGGAT
CTCAACAGCGGTAAGATCCTTGAGAGTTTTCGCCCCGAAGAACGTTTTCCAATGATGAGCACTTTTAAAGTTCTG
CTATGTGGCGCGGTATTATCCCGTATTGACGCCGGGCAAGAGCAACTCGGTCGCCGCATACACTATTCTCAGAA
TGACTTGGTTGAGTACTCACCAGTCACAGAAAAGCATCTTACGGATGGCATGACAGTAAGAGAATTATGCAGTGC
TGCCATAACCATGAGTGATAACACTGCGGCCAACTTACTTCTGACAACGATCGGAGGACCGAAGGAGCTAACCG
CTTTTTTGCACAACATGGGGGATCATGTAACTCGCCTTGATCGTTGGGAACCGGAGCTGAATGAAGCCATACCA
AACGACGAGCGTGACACCACGATGCCTGTAGCAATGGCAACAACGTTGCGCAAACTATTAACTGGCGAACTACT
TACTCTAGCTTCCCGGCAACAATTAATAGACTGGATGGAGGCGGATAAAGTTGCAGGACCACTTCTGCGCTCGG
CCCTTCCGGCTGGCTGGTTTATTGCTGATAAATCTGGAGCCGGTGAGCGTGGGTCTCGCGGTATCATTGCAGCA
CTGGGGCCAGATGGTAAGCCCTCCCGTATCGTAGTTATCTACACGACGGGGAGTCAGGCAACTATGGATGAAC
GAAATAGACAGATCGCTGAGATAGGTGCCTCACTGATTAAGCATTGGTAACTGTCAGACCAAGTTTACTCATATA
TACTTTAGATTGATTTAAAACTTCATTTTTAATTTAAAAGGATCTAGGTGAAGATCCTTTTTGATAATCTCATGACCA
 
AAATCCCTTAACGTGAGTTTTCGTTCCACTGAGCGTCAGACCCCGTAGAAAAGATCAAAGGATCTTCCGGACCGA
AAGAGTTTGTAGAAACGCATGTCAGCCGTTAAGTGTTCCTGTGTCACTCAAAATTGCTTTGAGAGGCTCTAAGGG
CTTCTCAGTGCGTTACATCCCTGGCTTGTTGTCCACAACCGTTAAACCTTAAAAGCTTTAAAAGCCTTATATATTC
TTTTTTTTCTTATAAAACTTAAAACCTTAGAGGCTATTTAAGTTGCTGATTTATATTAATTTTATTGTTCAAACATGA
GAGCTTAGTACGTGAAACATGAGAGCTTAGTACGTTAGCCATGAGAGCTTAGTACGTTAGCCATGAGGGTTTAGT
TCGTTAAACATGAGAGCTTAGTACGTTAAACTTGAGAGCTTAGTACGTGAAACATGAGAGCTTAGTACGTAC
**SEQ ID NO 26**
   LENGTH: 5.603
   TYPE: DNA
   ORGANISM: Artificial
   FEATURE: Parental Plasmid
   OTHER INFORMATION: Parental Plasmid / pDNAv_B
   SEQUENCE: 26

CAAATTCAAATTTTAATTTGGAAATTTTGCGCCCCTCCTTCTCGTGTACTGTAAAACCAACAAACGAAATCTCAGTT
TTCTTTTTGATTTCTGCAATCGCTTTGTTTAAAACATCGCGTTTAAAAATTGGGAAGTCCGGGTATTTATATTCGAT
GTTCCCGTCTTTATCAAAAGTATAGGCAATCAGTTCTTCTTTAAGCTCGTCCACGCTAATGATAAAATAGTTTTTTT
TTTTGAAATTACTATAATGTTTGCGGATCAGTTGATACAGTGATGAAGAATACTGTGACGACAGGCGCAAGGATG
CAGTCAATAATTGCGTGGTAAATTTGTTCTTTTTCCCGATAAGACTAGAAATATAAGGCTCAATAGTACGCGTGAA
CTTCAGACTCAAGTAGCCCTCGTCAGGGCTGTAAGCGATCCACTCAATGATATTCAGGTCTAATTCCTCCGGGG
AATTTTTCGCAGTGAATGGCAAGTTCAACTCCTTAGCAAGGGCGATGATATCGTCTCCACGCAAGCTGATCTTCG
AGGCGCCCAGCAATTTGCCTCCCTCCTTCAGTTGACGGTAGGCCAAACTAGGAGTAATTTTTGCAAGCGCCGCT
AAGTCTTCGGCACGAATCTTAAATACACGACCGCGTTCTAACGGCTCCTTACTGTCAATGGGGGCAAGGGCCAT
ATACATCACGCGTTTTGCTGGTAAAGGCAGCTGAGCTAAGGTATGATTAAGCTCATTACGATGACGAATCTTGGT
TTTTTTATTGACATCCATCATCACCTTCAAACGCATACTCTTCCTTTTTCAATATTATTGAAGCATTTATCAGGGTTA
TTGTCTCATGAGCGGATACATATTTGAATGTATTTAGAAAAATAAACAAATAGGGGTTCCGCGTGCGTTTCTACAA
ACTCTTTCGGTCCGGAAGATCCTTTGATCTTTTCTACGGGGTCTGACGCTCAGTGGAACGAAAACTCACGTTAAG
GGATTTTGGTCATGAACAATAAAACTGTCTGCTTACATAAACAGTAATACAAGGGGTGTTATGAGCCATATTCAAC
GGGAAACGTCTTGCTCTAGGCCGCGATTAAATTCCAACATGGATGCTGATTTATATGGGTATAAATGGGCTCGCG
ATAATGTCGGGCAATCAGGTGCGACAATCTATCGATTGTATGGGAAGCCCGATGCGCCAGAGTTGTTTCTGAAA
CATGGCAAAGGTAGCGTTGCCAATGATGTTACAGATGAGATGGTCAGACTAAACTGGCTGACGGAATTTATGCC
TCTTCCGACCATCAAGCATTTTATCCGTACTCCTGATGATGCATGGTTACTCACCACTGCGATCCCCGGGAAAAC
AGCATTCCAGGTATTAGAAGAATATCCTGATTCAGGTGAAAATATTGTTGATGCGCTGGCAGTGTTCCTGCGCCG
GTTGCATTCGATTCCTGTTTGTAATTGTCCTTTTAACAGCGATCGCGTATTTCGTCTCGCTCAGGCGCAATCACG
AATGAATAACGGTTTGGTTGATGCGAGTGATTTTGATGACGAGCGTAATGGCTGGCCTGTTGAACAAGTCTGGAA
AGAAATGCATAAACTTTTGCCATTCTCACCGGATTCAGTCGTCACTCATGGTGATTTCTCACTTGATAACCTTATT
TTTGACGAGGGGAAATTAATAGGTTGTATTGATGTTGGACGAGTCGGAATCGCAGACCGATACCAGGATCTTGC
CATCCTATGGAACTGCCTCGGTGAGTTTTCTCCTTCATTACAGAAACGGCTTTTTCAAAAATATGGTATTGATAAT
CCTGATATGAATAAATTGCAGTTTCATTTGATGCTCGATGAGTTTTTCTAAGAATTAATTCATGAGCGGATACATAT
TTGAATGTATTTAGAAAAATAAACAAATAGGGGTTCCGCGCACATTTCCCCGAAAAGTGCCACAGGACTGAGCGT
CAACCCCGTAGAAAAGCCCCAACTGGGGTAACCTTTGAGTTCTCTCAGTTGGGGGATCAAAGGATCTTCGTAGA
CGATAGATCTACTGACTCTGAGTTCGTAGTAGTGTACGTACGGATATCAGATCTACTGACTCTGAGTTCGTAGTA
GTGTACGTACGTAGTCAGCACACAATTGCCATTATACGCGCGTATAATGGACTATTGTGTGCTGATAAATTGGAG
ATCGGAGCTCGTACTTCGCATGCATGCATGCTGGTTGCTGACTAATTGAGATGCATGCTTTGCATACTTCTGCCT
GCTGGGGAGCCTGGGGACTTTCCACACCAATGCATGCATGCTGGTTGCTGACTAATTGAGATGCATGCTTTGCA
TACTTCTGCCTGCTGGGGAGCCTGGGGACTTTCCACACCAATGCATGCGTGCCTATCAAGATATTTAAAGAAAAA

AAAATTGTATCAACTTTCTACAATCTCTTTCAGAAGACAGAAGCAGAGGGAATACTTCCTAAATCATTCAACTAGG
CCAGCATTACCTTAATACCGGAACTAGAAAATGACATTACAAGAAAAGAAAACAACAGACCAATATCTCTCATGAA
CAAAGATACAAACATTTTCAACAAAATATTAGCAAAAAGAATCCAAGAATGTATCAAAAAATATACACCACAACCAA
GTAGAATTTATTCCAGATATGTAAGGGTGGTTCAACGTTTGAAAATCAATTAACGTAATTTGTCCCATCAACAGGT
TAAAGAAGAAAATCACATGGTCATATTGATAGACACAGAAAAAGCATTTGACAAAATTTAACACCCATTCATGATG
CAATCTCTCAGTAAACTAGGAATAGAGGAAAACTTCCTCAGCTTGAATGTACCTTCCTCTCAATTTTGCTATGAAC
CTGAAACTCCTCTTAAAAAATAAAGTTTTTCATTTAAAAAGAAAACAAAAAACATGGAGGAGCGTTGATGTATCTC
ATTTTAGACCAATCAGCTATGGATAGGATCCAGACATGATAAGATACATTGATGAGTTTGGACAAACCACAACTA
GAATGCAGTGAAAAAAATGCTTTATTTGTGAAATTTGTGATGCTATTGCTTTATTTGTAACCATTATAAGCTGCAAT
AAACAAGTTAACAACAACAATTGCATTCATTTTATGTTTCAGGTTCAGGGGGAGGTGTGGGAGGTTTTTTAAAGCA
AGTAAAACCTCTACAAATGTGGTATGGCTGATTATGATCNNNNNNGAGACCGTCGACTCTAGAGCTAGCGTACG
TACTAAGCTCTCATGTTTCACGTACTAAGCTCTCAAGTTTAACGTACTAAGCTCTCATGTTTAACGAACTAAACCC
TCATGGCTAACGTACTAAGCTCTCATGGCTAACGTACTAAGCTCTCATGTTTCACGTACTAAGCTCTCATGTTTGA
ACAATAAAATTAATATAAATCAGCAACTTAAATAGCCTCTAAGGTTTTAAGTTTTATAAGAAAAAAAAGAATATATAA
GGCTTTTAAAGCTTTTAAGGTTTAACGGTTGTGGACAACAAGCCAGGGATGTAACGCACTGAGAAGCCCTTAGA
GCCTCTCAAAGCAATTTTGAGTGACACAGGAACACTTAACGGCTGACANNNNNNGAGACCGAATTCCCTGCAGG
CGGGAGATGGGGGAGGCTAACTGAAACACGGAAGGAGACAATACCGGAAGGAACCCGCGCTATGACGGCAAT
AAAAAGACAGAATAAAACGCACGGGTGTTGGGTCGTTTGTTCATAAACGCGGGGTTCGGTCCCAGGGCTGGCA
CTCTGTCGATACCCCACCGAGACCCCATTGGGACCAATACGCCCGCGTTTCTTCCTTTTCCCCACCCCAACCCC
CAAGTTCGGGTGAAGGCCCAGGGCTCGCAGCCAACGTCGGGGCGGCAAGCCCTGCCATAGCCACCTATCAAG
ATATTTAAAGAAAAAAAAATTGTATCAACTTTCTACAATCTCTTTCAGAAGACAGAAGCAGAGGGAATACTTCCTAA
ATCATTCAACTAGGCCAGCATTACCTTAATACCGGAACTAGAAAATGACATTACAAGAAAAGAAAACAACAGACC
AATATCTCTCATGAACAAAGATACAAACATTTTCAACAAAATATTAGCAAAAAGAATCCAAGAATGTATCAAAAAAT
ATACACCACAACCAAGTAGAATTTATTCCAGATATGTAAGGGTGGTTCAACGTTTGAAAATCAATTAACGTAATTT
GTCCCATCAACAGGTTAAAGAAGAAAATCACATGGTCATATTGATAGACACAGAAAAAGCATTTGACAAAATTTAA
CACCCATTCATGATGCAATCTCTCAGTAAACTAGGAATAGAGGAAAACTTCCTCAGCTTGAATGTACCTTCCTCTC
AATTTTGCTATGAACCTGAAACTCCTCTTAAAAAATAAAGTTTTTCATTTAAAAAGAAAACAAAAAACATGGAGGAG
CGTTGATGTATCTCATTTTAGACCAATCAGCTATGGATAGATGCATGCATGCTGGTTGCTGACTAATTGAGATGC
ATGCTTTGCATACTTCTGCCTGCTGGGGAGCCTGGGGACTTTCCACACCAATGCATGCATGCTGGTTGCTGACT
AATTGAGATGCATGCTTTGCATACTTCTGCCTGCTGGGGAGCCTGGGGACTTTCCACACCAATCGAAGGACCAT
GGTCTGCGATCGTATCAGCACACAATAGTCCATTATACGCGCGTATAATGGCAATTGTGTGCTGAAGTTGACATC
TAGGTACCGATATCGTTGACATCTAGTACAGTTCAGTACAGTAGTCACTGACTTGACACTGACCTCGAGATAACT
TCGTATAATGTATGCTATACGAAGTTATGCGGCCGCATCAAAGGATCTTCTAGGGATAACAGGGTAATGTCATCT
GGGATAACAGGGTAATGTCATCTAGGGATAACAGGGTATGTCATCTGGGATAACAGGGTAATGTATCTAGGGAT
AACAGGGTAATGTCATCTGGGATAACAGGGTAATGTCATCTAGGGATAACAGGGTATGTCATCTGGGATAACAG
GGTAATGTATCTAGGGATAACAGGGTAATGTCATCTGGGATAACAGGGTAATGTCATCTAGGGATAACAGGGTAT
GTCATCTGGGATAACAGGGTAATGTATCTAGGGATAACAGGGTAATGTCATCTGGGATAACAGGGTAATGTCATC
TAGGGATAACAGGGTATGTCATCTGGGATAACAGGGTAATGTATCTAGGGATAACAGGGTAATGTCATCTGGGA
TAACAGGGTAATGTCATCTAGGGATAACAGGGTATGTCATCTGGGATAACAGGGTAATGTATCTTGTATCTAGGG
ATAACAGGGTAATGAATGCGTCGAGATACTAGTGGATCCCAAAAAACCCCTCAAGACCCGTTTAGAGGCCCCAA
GGGGTTATGCTAGTTATTGCTCAGCGGTGGCAGCAGCCAACTCAGCTTCCTTTCGGGCTTTTAGCCCTTGGCTT
TTTTGGGCGGAACGGTTTCATCGAACACTTTTAAAAATGCCGCATCGGCCTCCGACAAGTTCATAAAGAAAGCCT
CGTCGTCTTTATCTCCACTAAACTCGTCCTCATCAACGA

## Claims

1. An engineered parental circular covalently closed synthetic plasmid DNA comprising:
a synthetic DNA backbone comprising at least a first, second, and third recombinase recognition sequence, at least one endonuclease recognition sequence, at least one DNA replication enzyme recognition sequence, at least one bacterial selection system, and at least one bacterial origin of replication;
a linear DNA module scaffold comprising at least one DNA nuclear targeting sequence and at least one nuclear matrix-association sequence; and
one or more synthetic transcriptional units comprising at least two multiple cloning sites including endonuclease recognition sequences, at least one promoter sequence, at least one coding sequence of interest, and at least one polyadenylation signal sequence.

2. The engineered parental circular covalently closed synthetic plasmid DNA of claim 1, wherein the recombinase recognition sequence is selected from the group consisting of PhiC31 attP recombinase recognition sequence, phiC31 attP recombinase recognition site, CRE recombinase recognition sequence (LoxP), Flp recombinase recognition sequence, Lambda recombinase recognition sequence, ResT recombinase recognition sequence, ϕpK02 telRL site, the FRT site, TelN recombinase recognition sequence, PY54 Tel recombinase recognition sequence, VP882 TelK recombinase recognition sequence, ΦHAP-1 Tel recombinase recognition sequence, B. burgdorferi Tel recombinase recognition sequence, B. hermsii Tel recombinase recognition sequence, B. parkeri Tel recombinase recognition sequence, B. recurrentis Tel recombinase recognition sequence, B. turicatae Tel recombinase recognition sequence, B. anserine Tel recombinase recognition sequence, A. tumefaciens C58 ResT recombinase recognition sequence, and combinations thereof; the endonuclease recognition sequence is selected from the group consisting of I- SceI endonuclease, F-Scel endonuclease, F-Scell endonuclease, I-Anil endonuclease, I-CeuI endonuclease, I-Chul endonuclease, I-CpaI endonuclease, I-Cpall endonuclease, I-Crel endonuclease, I-Csml endonuclease, I-Dmol endonuclease, I-PorI endonuclease, I-Scal endonuclease, I-SceII endonuclease, I-SceIII endonuclease, I-SceIV endonuclease, PI-Scel endonuclease, Pi-Pspi endonuclease, PI-Tlil endonuclease, F-Suvl, F-Tevl endonuclease, F-Tevll endonuclease, I-DirI, I-HmuI endonuclease, I-HmuII endonuclease, I-PpoI endonuclease, I- TevIII, and combinations thereof; the DNA replication enzyme recognition sequence is selected from the group consisting of Phi29 DNA replication enzyme recognition sequence and P2-A replication enzyme recognition sequence; the bacterial selection system is selected from the group consisting of antibiotics resistance selection, dapD complementation selection, infA complementation selection, amino acid auxotrophy complementation, RNA-OUT selection systems, and combinations thereof; the bacterial origin of replication is selected from the group consisting of pUC19-derived replication origin, pBR322-derived replication origin, p15A-derived replication origin, pSC101-derived replication origin, Pir-R6K replication origin, trfA-oriV replication origin, M13 Phage replication origin, f1 Phage replication origin, PhiX174/ProteinA Ori replication origin, and pT181 (RepC) replication origin; the DNA nuclear targeting sequence is selected from the group consisting of SV40 enhancer nuclear targeting DNA sequence, NF-kB nuclear targeting DNA sequence, GRE nuclear targeting DNA sequence, smooth muscle-specific nuclear targeting DNA sequence, smooth muscle γ-actin (SMGA) promoter nuclear targeting DNA sequence, Sox2 regulatory region 2 (SRR2) nuclear targeting DNA sequence, Karyopherin (importin) β1, isoform 1 (Kpnβ1) nuclear targeting DNA sequence, RanBP7/importin 7 (Ipo7) nuclear targeting DNA sequence, RAN nuclear targeting DNA sequence, Ran binding protein 1 (RanBP1) nuclear targeting DNA sequence, Ran binding protein 3 (RanBP3) nuclear targeting DNA sequence, SYTE-DNTS1 nuclear targeting DNA sequence, and combinations thereof; the nuclear matrix-association sequence is selected from the group consisting of human b-interferon gene-derived S/MAR, human immunoglobulin heavy chain-derived S/MAR, human apolipoprotein B-derived S/MAR, chicken lysozyme-derived S/MAR, other vertebrate-derived S/MAR, SV40T mutated sequence, β-Globin Replicator sequence, Epstein-Barr virus (EBV) elements oriP, and EBNA1; the promoter sequence is selected from the group consisting of RNA polymerase II constitutive mammalian promoters, inducible promoters, and RNA polymerase III promoters; the coding sequence of interest is selected from the group consisting of peptides, polypeptides, vaccine antigens, monoclonal antibodies, mono-specific antibodies, bi-specific antibodies, tri-specific antibodies, tetra-specific antibodies, chimeric antigen receptors, allergens, blood components, gene therapies, organ or cellular products, cytokines, immunomodulators, growth factors, enzymes, antibody fragments, poly epitopes, multispecific antibodies, poly-epitope-specific antibodies, diabodies, single chain molecules, VHH antibodies, antibodies fragments, chimeric antibodies, fusion proteins, plasma membrane anchored proteins, cytoplasmic proteins, cytoskeletal proteins, nuclear proteins, gene editing proteins, zinc fingers, nucleases, Cas9, TALENs, dimers, trimers, tetramers, single-chain polypeptides, multi-chain polypeptides, and combinations thereof; the polyadenylation signal sequence is selected from the group consisting of human α-globin polyadenylation signal sequence, human β-globin polyadenylation signal sequence, rabbit α-globin polyadenylation signal sequence, rabbit β-globin polyadenylation signal sequence, mouse k-light chain polyadenylation signal sequence, and chicken ovalbumin polyadenylation signal sequence.

3. The engineered parental circular covalently closed synthetic plasmid DNA of claim 1, wherein the linear DNA module scaffold further comprises one or more eukaryotic origins of replication.

4. The engineered parental circular covalently closed synthetic plasmid DNA of claim 1, wherein the synthetic transcriptional unit further comprises one or more of: an enhancer sequence, a post-transcriptional regulatory element, a synthetic or wild-type IRES sequence, a MIRES sequence, a sequence for non-coding RNAs, and a plurality of permuted Intron-Exon sequences.

5. The engineered parental circular covalently closed synthetic plasmid DNA of claim 4, wherein the enhancer sequence is selected from the group consisting of constitutive mammalian enhancers; the post-transcriptional regulatory element is selected from the group consisting of Hepatitis B virus (HPRE) regulatory element, Woodchuck Hepatitis virus (WPRE) regulatory element; the synthetic or wild-type IRES sequence is selected from the group consisting of encephalomyocarditis virus (ECMV) IRES, Coxackievirus B3 (CVB3) IRES, Taura Syndrome Virus (TSV) IRES, Triatoma Virus (TV), Human mastadenovirus C (HAdV-C), Human adenovirus 5 (HAdV5), Human adenovirus 7 (HAdV7), Human mastadenovirus B (HAdV-B), Hepatitis GB virus B (HGBV-B), HPV31, Human Poliovirus 3 (HPV-3), Human papillomavirus type 11 (HPV11), Human immunodeficiency virus 1 (HIV-1), Human immunodeficiency virus 2 (HIV-2), Simian T-lymphotropic virus 1 (STLVs-1), Human Parvovirus B19 (HPB19), Human betaherpesvirus 6B (HHV-6B), Human alphaherpesvirus 3 (HHV-3), Human papillomavirus type 41 (HPV41), Human papillomavirus type 6b (HPV6b), Alphapapillomavirus 7, Friend murine leukemia virus, Heilovirus (ThV), Rous sarcoma virus (RSV), Human mastadenovirus F (HAdv-F), Human papillomavirus type 4 (HPV4), Human papillomavirus type 63 (PHV63), Human mastadenovirus A, Feline immunodeficiency virus (FIV), Human T-lymphotropic virus 2 (HTLV-2), Jaagsiekte sheep retrovirus (JSRV), Spleen focus-forming virus (SFFV), Mouse mammary tumor virus (MMTV), Murine osteosarcoma virus (MOV), Ovine lentivirus (OLV/OvLV), Squirrel monkey retrovirus (SMRV), Human papillomavirus type 16 (HPV16), Human papillomavirus type 5 (HPV5), Human polyomavirus 1, Rabies lyssavirus, Simian immunodeficiency virus (SIV), Human papillomavirus type 10 (HPV10), Human papillomavirus type 26 (HPV26), Human papillomavirus type 32 (HPV32), Human papillomavirus type 34 (HPV34), Marburg marburgvirus, Enterovirus A, Rhinovirus A, Human betaherpesvirus 6A (HHV-6A), Macaca mulatta polyomavirus 1, Snakehead retrovirus (SnRV), Bovine foamy virus (BFV), Drosophila C virus (DCV), Hendra henipavirus (HeV), Aichi virus 1 (AiV-1), Influenza B virus (IBV), Zaire ebolavirus (ZEBOV), Human coronavirus 229E (HCoV-229E), Nipah henipavirus (NiV), Human respirovirus 1 (HPIV-1), Modoc virus (MODV), Sudan ebolavirus, Human parvovirus 4 G1, Rotavirus C, Human gammaherpesvirus 4 (Epstein-Barr virus), eukaryotic IRES derived from Homo sapiens, Aplysia californica, Canis lupus familiaris, Drosophila melanogaster, Gallus gallus, Mus pahari, Mus musculus, Saccharomyces cerevisiae, Zea mays, Rattus norvegicus, Ovis aries, SYTE-IRES1, SYTE-IRES2, and combinations thereof; the MIRES sequence comprises a RRACH consensus motif, wherein R is selected from Guanine and Adenine, and H is selected from Adenine, Cytosine, and Thymine; the sequence for non-coding RNAs is selected from the group consisting of non-coding RNA, exRNA, hnRNA, IncRNA, microRNA, rRNA, shRNA, snoRNA, snRNA, siRNA, scaRNA, Y RNA, piRNA, miRNA, tRNA and rRNA, iRNA, guideRNA (gRNA), single-guideRNA(sgRNA), crRNA, tracrRNA, spongeRNA, and combinations thereof; the permuted Intron-Exon sequence is selected from the group consisting of self-splicing group I intron fragment, group II intron fragment introns, and combinations thereof.

6. A helper plasmid comprising a first and a second synthetic genetic construction, wherein the first synthetic genetic construction comprises at least a first repressor protein under at least a first constitutive promoter, at least a second repressor coding sequence under at least a second constitutive promoter, at least a third repressor coding sequence under at least a third constitutive promoter, at least one endonuclease recognition sequence, and wherein the second synthetic genetic construction comprises at least one DNA replicating protein under the control of a first inducible promoter, at least one recombinase protein coding sequence under the control of a second inducible promoter, and at least one endonuclease coding sequence under the control of a third inducible promoter.

7. The helper plasmid of claim 6, wherein the constitutive promoters are selected from the group consisting of P(Bla) constitutive promoter, P(Cat) constitutive promoter, P(Kat) constitutive promoter, Reverse lambda cl-regulated promoter, pBAD reverse constitutive promoter, lacQ constitutive promoter, GlnRS constitutive promoter, and combinations thereof; the inducible promoters are selected from the group consisting of L-Arabinose inducible promoter, IPTG inducible promoter, Lactose inducible promoter, Glucose inducible promoter, Tetracycline inducible promoter, Temperature inducible promoter, pH inducible promoter, HSL inducible promoter, Maltose inducible promoter, Xylose inducible promoter, Phosphate inducible promoter, Cold-Shock inducible promoter, glnG transcription enhancer inducible promoter, and combinations thereof;

8. The helper plasmid of claim 6, further comprising at least one of: a recognition sequence for an endonuclease selected from the group consisting of I- SceI endonuclease, F-Scel endonuclease, F-Scell endonuclease, I-Anil endonuclease, I-CeuI endonuclease, I-Chul endonuclease, I-CpaI endonuclease, I-Cpall endonuclease, I-Crel endonuclease, I-Csml endonuclease, I-Dmol endonuclease, I-PorI endonuclease, I-Scal endonuclease, I-SceII endonuclease, I-SceIII endonuclease, I-SceIV endonuclease, PI-Scel endonuclease, Pi-Pspi endonuclease, PI-Tlil endonuclease, F-Suvl, F-Tevl endonuclease, F-Tevll endonuclease, I-DirI, I-HmuI endonuclease, I-HmuII endonuclease, I-PpoI endonuclease, I- TevIII, and combinations thereof; a bacterial genome integration cassette.

9. A recombinant cell comprising the helper plasmid of any of the precedent claims.

10. The recombinant cell of claim 9, wherein the helper plasmid is maintained episomally.

11. The recombinant cell of claim 9, wherein the helper plasmid is integrated into a bacterial chromosome.

12. A hairpin loop ended self-complementary double-stranded linear covalently closed DNA vector production system comprising:
the recombinant cell of any of claims 9 to 11; and
at least two engineered parental circular covalently closed synthetic plasmids DNA of any of claims 1 to 5, housed in the recombinant cell, wherein the synthetic transcriptional units of the at least two engineered parental circular covalently closed synthetic plasmids DNA are cloned in opposite directions.

13. A hairpin loop ended self-complementary double-stranded linear covalently closed DNA vector production process using the system of claim 12, comprising the stages of:
a. cloning the desired synthetic transcriptional unit between two of the multiple cloning sites in two of the engineered parental circular covalently closed synthetic plasmids DNA in order to generate plasmids pDNAv_Ac and pDNAv_Bc;
b. in order to generate the cointegrate plasmid pDNAv_ABc, alternatively subjecting both pDNAv_Ac and pDNAv_Bc to a first *in vitro* recombination using the first recombinase recognition sequence, or subjecting both pDNAv_Ac and pDNAv_Bc to a first *in vitro* recombination using restriction enzyme digestion in the first recombinase recognition sequence and subjecting digested pDNAv_Ac and pDNAv_Bc to ligation, or subjecting both pDNAv_Ac and pDNAv_Bc to a recombination including PCR amplification of each plasmid and the use of *in vitro* recombination systems;
c. transforming the cointegrate plasmid pDNAv_ABc with rolling circle and theta replication capacity into the recombinant cell and selecting recombinant clones;
d. alternatively subjecting the recombinant cell to incubation in growth conditions to allow theta replication of plasmid pDNAv_ABc, inducing the expression of the second recombinase in the recombinant cell to allow the generation of the Linear DNA molecules and inducing the expression of the endonuclease to allow the degradation and elimination of a resulting parental recombination product and the helper plasmid , or subjecting the cointegrate plasmid pDNAv_ABc to a second *in vivo* recombination using the second recombinase recognition sequence in order to generate pDNA_ABc1 and pDNA_ABc2, inducing the expression of the DNA replicating protein and endonuclease in order to allow "rolling circle" replication of pDNA_ABc1 and self-annealing of the single-stranded DNA products of the rolling circle replicated-DNA into hairpin loop ended double-stranded DNA molecules and allow the degradation of pDNAv_ABc2 by the endonuclease activity and inducing the expression of the second recombinase in the recombinant cell to allow the cleavage and elimination of the loop; and
e. isolating the resulting hairpin loop ended self-complementary double-stranded linear covalently closed DNA molecules from the recombinant cell.

14. A hairpin loop ended self-complementary double-stranded linear covalently closed DNA vector obtainable by the process of claim 13.

15. The hairpin loop ended self-complementary double-stranded linear covalently closed DNA vector of claim 14, wherein the genetic elements of the DNA vector are operably linked in the following order: a single-stranded DNA loop, optionally at least one SV40E sequence, optionally at least one S/MAR sequence, optionally at least one eukaryotic origin of replication, at least one promoter, optionally at least one 5' untranslated region sequence, at least one coding sequence or sequence of interest, optionally one or more IRES, MIRES and/or 2A-peptide sequences, optionally at least one 3' untranslated region sequence, at least one transcriptional terminator sequence, optionally at least one S/MAR sequence, optionally at least one eukaryotic origin of replication, optionally at least one SV40E sequence, a single-stranded DNA loop, with suitable DNA spacer sequences optionally placed between at least two of the aforementioned elements, and said vector is applicable to prevent or treat infectious disorders, genetic and non-genetic conditions.
